# EUROPEAN PATENT APPLICATION

(11) **EP 4 397 353 A1**
(43) Date of publication of application: **10.07.2024**
(21) Application number: 22864276.5
(22) Date of filing: 18.08.2022
(51) Int. Cl.: A61M 25/092, A61B 1/01

(54) **MEDICAL DEVICE**

(30) Priority: 31.08.2021 JP 2021141848
(71) Applicant: CANON KABUSHIKI KAISHA, Tokyo 146-8501 (JP)
(72) Inventor: IWASAWA, Ryo, Tokyo 146-8501 (JP); NIIKAWA, Yusuke, Tokyo 146-8501 (JP); NOGUCHI, Tomio, Tokyo 146-8501 (JP)
(74) Representative: TBK
(86) International application number: PCT/JP2022/031167
(87) International publication number: WO 2023/032691

(57) **Abstract**

A medical apparatus (1) includes a drive source (M), a bending portion (12) capable of bending, a linear body (Wb) configured to bend the bending portion (12) by a driving force of the drive source (M), and a connecting portion (Wc) in which a first member (Wc1) connected to the drive source (M) and a second member (Wc2) connected to the linear body (Wb) are connected. The connecting portion (Wc) is configured such that in a case where a tensile force equal to or smaller than a first threshold value or a compressive force equal to or smaller than a second threshold value acts between the first member (Wc1) and the second member (Wc2), the state in which a protrusion portion (c13) is fitted in the recess portion (c23) is maintained to transmit the driving force from the drive source (M) to the linear body (Wb), and in a case where a tensile force exceeding the first threshold value or a compressive force exceeding the second threshold value acts between the first member (Wc1) and the second member (Wc2), the protrusion portion (c13) is disengaged from the recess portion (c23) to cut off transmission of the driving force from the drive source (M) to the linear body (Wb).

## Description

### TECHNICAL FIELD

The present invention relates to a bendable medical apparatus.

### BACKGROUND ART

A medical apparatus such as an endoscope or a catheter includes a bending portion formed from a linear body connected to a drive source so as to be capable of deforming by bending, in an insertion portion to be inserted into a human body. Patent Literature 1 describes a configuration in which a driving wire connected to a drive source and a control wire connected to the bending portion of the insertion portion are interconnected via a breaker portion having a lower breaking strength than the control wire. According to this document, when a tensile force equal to or greater than a threshold value acts on the control wire, the breaker portion breaks, and thus the distal end of the insertion portion strongly pressing an object is avoided.

### CITATION LIST

### PATENT LITERATURE

Patent Literature 1: Japanese Patent Application Laid-Open No. 2013-248116

### SUMMARY OF INVENTION

### TECHNICAL PROBLEM

However, in the configuration of the document described above, the connection via the breaker portion is not cut off in the case where a strong compressive force acts on the breaker portion.

An object of the present invention is to provide a medical apparatus capable of cutting off connection between a drive source and a linear body in response to both an overload in a direction to press the linear body and an overload in a direction to pull the linear body.

### SOLUTION TO PROBLEM

A first aspect of the present invention is a medical apparatus including a drive source, a bending portion capable of bending, a linear body configured to bend the bending portion by a driving force of the drive source, and a connecting portion which includes a first member connected to the drive source, a second member connected to the linear body, a protrusion portion provided in one of the first member and the second member and protruding in a direction intersecting with an extending direction of the linear body, and a recess portion provided in another of the first member and the second member, and in which the first member and the second member are connected in a state in which the protrusion portion is fitted in the recess portion, wherein the connecting portion is configured such that in a case where a tensile force equal to or smaller than a first threshold value or a compressive force equal to or smaller than a second threshold value acts between the first member and the second member, the state in which the protrusion portion is fitted in the recess portion is maintained to transmit the driving force from the drive source to the linear body, and in a case where a tensile force exceeding the first threshold value or a compressive force exceeding the second threshold value acts between the first member and the second member, the protrusion portion is disengaged from the recess portion to cut off transmission of the driving force from the drive source to the linear body.

A second aspect of the present invention is a medical apparatus including a drive source, a bending portion capable of bending, a linear body configured to bend the bending portion by a driving force of the drive source, and a connecting portion which includes a first member connected to the drive source, a second member connected to the linear body, a first protrusion portion and a second protrusion portion provided in the first member and protruding in a direction intersecting with an extending direction of the linear body, and a third protrusion portion and a fourth protrusion portion provided in the second member, and in which the first member and the second member are connected in a state in which the first protrusion portion is engaged with the third protrusion portion and the second protrusion portion is engaged with the fourth protrusion portion, wherein the connecting portion is configured such that in a case where a tensile force equal to or smaller than a first threshold value acts between the first member and the second member, the state in which the first protrusion portion is engaged with the third protrusion portion is maintained to transmit the driving force from the drive source to the linear body, in a case where a tensile force exceeding the first threshold value acts between the first member and the second member, the first protrusion portion is disengaged from the third protrusion portion to cut off transmission of the driving force from the drive source to the linear body, in a case where a compressive force equal to or smaller than a second threshold value acts between the first member and the second member, the state in which the second protrusion portion is engaged with the fourth protrusion portion is maintained to transmit the driving force from the drive source to the linear body, and in a case where a compressive force exceeding the second threshold value acts between the first member and the second member, the second protrusion portion is disengaged from the fourth protrusion portion to cut off transmission of the driving force from the drive source to the linear body.

### ADVANTAGEOUS EFFECTS OF INVENTION

According to the present invention, connection between a drive source and a linear body can be cut off in response to both an overload in a direction of pressing the linear body and an overload in a direction of pulling the linear body.

Other features and merits of the present invention will be revealed through the following descriptions with reference to attached drawings. To be noted, in the attached drawings, the same or substantially the same elements are denoted by the same reference numerals.

### BRIEF DESCRIPTION OF DRAWINGS

FIG. 1 is an overall view of a medical system.
FIG. 2 is a perspective view of a medical apparatus and a supporting base.
FIG. 3A an explanatory diagram of a catheter.
FIG. 3B is an explanatory diagram of the catheter.
FIG. 4Ais an explanatory diagram of a catheter unit.
FIG. 4B is an explanatory diagram of the catheter unit.
FIG. 5A is an explanatory diagram of a base unit and a wire driving portion.
FIG. 5B is an explanatory diagram of the base unit and the wire driving portion.
FIG. 5C is an explanatory diagram of the base unit and the wire driving portion.
FIG. 6A is an explanatory diagram of the wire driving portion, a coupling device, and a bending driving portion.
FIG. 6B is an explanatory diagram of the wire driving portion, the coupling device, and the bending driving portion.
FIG. 6C is an explanatory diagram of the wire driving portion, the coupling device, and the bending driving portion.
FIG. 7A is an explanatory diagram of attachment of the catheter unit.
FIG. 7B is an explanatory diagram of attachment of the catheter unit.
FIG. 8A is a diagram for describing coupling between the catheter unit and the base unit.
FIG. 8B is a diagram for describing coupling between the catheter unit and the base unit.
FIG. 9 is an exploded view for describing coupling between the catheter unit and the base unit.
FIG. 10 is a diagram for describing fixation of a driving wire via a coupling portion.
FIG. 11 is a diagram for describing fixation of the driving wire via the coupling portion.
FIG. 12 is a diagram for describing fixation of the driving wire via the coupling portion.
FIG. 13 is a diagram for describing fixation of the driving wire via the coupling portion.
FIG. 14 is a diagram for describing fixation of the driving wire via the coupling portion.
FIG. 15A is an explanatory diagram of the catheter unit and the base unit.
FIG. 15B is an explanatory diagram of the catheter unit and the base unit.
FIG. 15C is an explanatory diagram of the catheter unit and the base unit.
FIG. 16A is a diagram for describing an operation of an operation portion.
FIG. 16B is a diagram for describing the operation of the operation portion.
FIG. 16C is a diagram for describing the operation of the operation portion.
FIG. 17A is a section view for describing the operation of the operation portion.
FIG. 17B is a section view for describing the operation of the operation portion.
FIG. 17C is a section view for describing the operation of the operation portion.
FIG. 18A is a diagram for describing a connecting portion according to a first embodiment.
FIG. 18B is a diagram for describing the connecting portion according to the first embodiment.
FIG. 18C is a diagram for describing the connecting portion according to the first embodiment.
FIG. 19A is a diagram for describing an operation of the connecting portion according to the first embodiment.
FIG. 19B is a diagram for describing the operation of the connecting portion according to the first embodiment.
FIG. 19C is a diagram for describing the operation of the connecting portion according to the first embodiment.
FIG. 20A is a diagram for describing the operation of the connecting portion according to the first embodiment.
FIG. 20B is a diagram for describing the operation of the connecting portion according to the first embodiment.
FIG. 20C is a diagram for describing the operation of the connecting portion according to the first embodiment.
FIG. 21A is a diagram for describing a modification example of a connecting portion according to a second embodiment.
FIG. 21B is a diagram for describing a modification example of the connecting portion according to the second embodiment.
FIG. 21C is a diagram for describing a modification example of the connecting portion according to the second embodiment.
FIG. 21D is a diagram for describing a modification example of the connecting portion according to the second embodiment.
FIG. 21E is a diagram for describing a modification example of the connecting portion according to the second embodiment.
FIG. 21F is a diagram for describing a modification example of the connecting portion according to the second embodiment.
FIG. 21G is a diagram for describing a modification example of the connecting portion according to the second embodiment.
FIG. 22Ais a diagram for describing the connecting portion according to the second embodiment.
FIG. 22B is a diagram for describing the connecting portion according to the second embodiment.
FIG. 22C is a diagram for describing the connecting portion according to the second embodiment.
FIG. 23A is a diagram for describing an operation of the connecting portion according to the second embodiment.
FIG. 23B is a diagram for describing the operation of the connecting portion according to the second embodiment.
FIG. 23C is a diagram for describing the operation of the connecting portion according to the second embodiment.
FIG. 24A is a diagram for describing a connecting portion according to a third embodiment.
FIG. 24B is a diagram for describing the connecting portion according to the third embodiment.
FIG. 24C is a diagram for describing the connecting portion according to the third embodiment.
FIG. 25A is a diagram for describing an operation of the connecting portion according to the third embodiment.
FIG. 25B is a diagram for describing the operation of the connecting portion according to the third embodiment.
FIG. 25C is a diagram for describing the operation of the connecting portion according to the third embodiment.
FIG. 26A is a diagram for describing a connecting portion according to a fourth embodiment.
FIG. 26B is a diagram for describing the connecting portion according to the fourth embodiment.
FIG. 26C is a diagram for describing the connecting portion according to the fourth embodiment.
FIG. 27A is a diagram for describing an operation of the connecting portion according to the fourth embodiment.
FIG. 27B is a diagram for describing the operation of the connecting portion according to the fourth embodiment.
FIG. 27C is a diagram for describing the operation of the connecting portion according to the fourth embodiment.
FIG. 28A is a diagram for describing a connecting portion according to a modification example.
FIG. 28B is a diagram for describing the connecting portion according to the modification example.
FIG. 28C is a diagram for describing the connecting portion according to the modification example.
FIG. 29 is a diagram for describing the connecting portion according to the modification example.
FIG. 30A is a diagram for describing an operation of the connecting portion according to the modification example.
FIG. 30B is a diagram for describing the operation of the connecting portion according to the modification example.
FIG. 30C is a diagram for describing the operation of the connecting portion according to the modification example.

### DESCRIPTION OF EMBODIMENTS

Embodiments of the present disclosure will be described below with reference to drawings.

### [First Embodiment]

### <Medical System and Medical Apparatus>

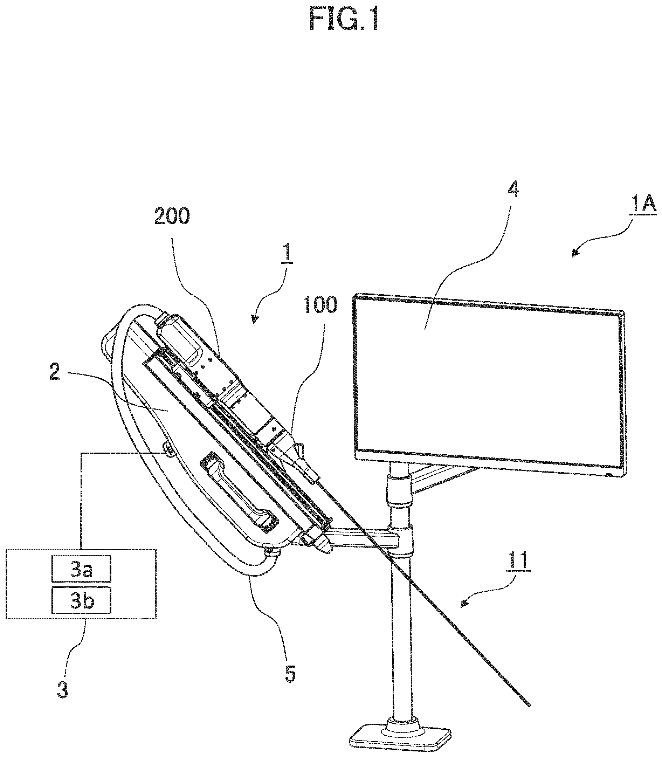

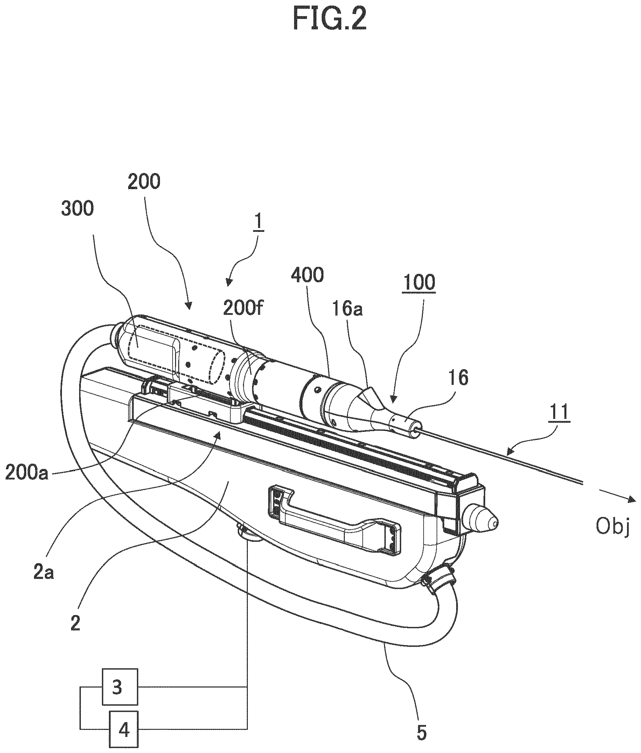

A medical system 1A and a medical apparatus 1 according to a first embodiment will be described with reference to FIGS. 1 and 2. FIG. 1 is an overall view of the medical system 1A. FIG. 2 is a perspective view of the medical apparatus 1 and a supporting base 2.

The medical system 1A includes the medical apparatus 1, the supporting base 2 to which the medical apparatus 1 is attached, and a control portion (control apparatus) 3 that controls the medical apparatus 1. In the present embodiment, the medical system 1A includes a monitor 4 serving as a display apparatus.

The medical apparatus 1 includes a catheter unit (bendable unit) 100 including a catheter 11 serving as a bendable body, and a base unit (driving unit, attached unit) 200. The catheter unit 100 is configured to be attachable to and detachable from the base unit 200.

In the present embodiment, the user of the medical system 1A and the medical apparatus 1 is capable of performing a work such as observing the inside of an object, collecting various specimens from the inside of the object, and treating the inside of the object, by inserting the catheter 11 in the object (Obj). As one embodiment, the user can insert the catheter 11 in a patient serving as the object. Specifically, by insertion in a bronchial tube through the mouth or nose of the patient, a work such as observing, collecting, or cutting a lung tissue can be performed.

The catheter 11 can be used as a guide (sheath) for guiding a medical tool for performing the work described above. As examples of the medical tool (tool), endoscopes, forceps, and abrasion devices can be mentioned. In addition, the bendable body itself may have a function as the medical tools described above, and in this case, the bendable body is not limited to a tubular shape and may have, for example, a columnar shape.

In the present embodiment, the control portion 3 includes a calculation apparatus 3a, and an input apparatus 3b. The input apparatus 3b receives commands and input for operating the catheter 11. The calculation apparatus 3a includes a storage that stores programs and various data for controlling the catheter, a random access memory, and a central processing apparatus for executing the program. In addition, the control portion 3 may include an output portion for outputting a signal for displaying an image on the monitor 4.

As illustrated in FIG. 2, in the present embodiment, the medical apparatus 1 is electrically connected to the control portion 3 via the supporting base 2 and a cable 5 interconnecting a base unit 200 of the medical apparatus 1 and the supporting base 2. To be noted, the medical apparatus 1 and the control portion 3 may be directly connected to each other. The medical apparatus 1 and the control portion 3 may be wirelessly connected to each other.

The medical apparatus 1 is detachably attached to the supporting base 2 via the base unit 200. More specifically, regarding the medical apparatus 1, an attachment portion (connecting portion) 200a of the base unit 200 is detachably attached to a moving stage (receiving portion) 2a of the supporting base 2. Even in a state in which the attachment portion 200a of the medical apparatus 1 is detached from the moving stage 2a, the connection between the medical apparatus 1 and the control portion 3 is maintained such that the medical apparatus 1 can be controlled by the control portion 3. In the present embodiment, the medical apparatus 1 and the supporting base 2 are interconnected via the cable 5 even in a state in which the attachment portion 200a of the medical apparatus 1 is detached from the moving stage 2a.

The user can insert the catheter 11 in the object by manually moving the medical apparatus 1 in a state in which the medical apparatus 1 is detached from the supporting base 2 (state in which the medical apparatus 1 is detached from the moving stage 2a).

The user can use the medical apparatus 1 in a state in which the catheter 11 is inserted in the object and the medical apparatus 1 is attached to the supporting base 2. Specifically, the medical apparatus 1 moves as a result of the moving stage 2a moving in a state in which the medical apparatus 1 is attached to the moving stage 2a. Then, the operation of moving the catheter 11 in a direction to insert the catheter 11 in the object and the operation of moving the catheter 11 in a direction to pull out the catheter 11 from the object are performed. The movement of the moving stage 2a is controlled by the control portion 3.

The attachment portion 200a of the base unit 200 includes a cancellation switch and a detachment switch that are not illustrated. In a state in which the attachment portion 200a is attached to the moving stage 2a, the user can manually move the medical apparatus 1 in a guide direction of the moving stage 2a while continuously pressing the cancellation switch. That is, the moving stage 2a includes a guide element that guides the movement of the medical apparatus 1. When the user stops pressing the cancellation switch, the medical apparatus 1 is fixed to the moving stage 2a. In contrast, if the detachment switch is pressed in a state in which the attachment portion 200a is attached to the moving stage 2a, the user can detach the medical apparatus 1 from the moving stage 2a.

To be noted, one switch may have a function as a cancellation switch and a function as a detachment switch. In addition, if the cancellation switch is provided with a mechanism that switches between a pressed state and a non-pressed state, the user does not need to keep on pressing the cancellation switch when manually sliding the medical apparatus 1.

In a state in which the attachment portion 200a is attached to the moving stage 2a and the cancellation switch and the detachment switch are not pressed, the medical apparatus 1 is fixed to the moving stage 2a, and is moved by the moving stage 2a driven by an unillustrated motor.

The medical apparatus 1 includes a wire driving portion (linear body driving portion, line driving portion, body driving portion) 300 for driving the catheter 11. In the present embodiment, the medical apparatus 1 is a robot catheter apparatus that drives the catheter 11 by the wire driving portion 300 controlled by the control portion 3.

The control portion 3 is capable of performing an operation of bending the catheter 11 by controlling the wire driving portion 300. In the present embodiment, the wire driving portion 300 is included in the base unit 200. More specifically, the base unit 200 includes a base casing 200f accommodating the wire driving portion 300. That is, the base unit 200 includes the wire driving portion 300. The wire driving portion 300 and the base unit 200 can be collectively referred to as a catheter driving apparatus (base apparatus, main body).

In an extending direction of the catheter 11, an end portion where the distal end of the catheter 11 inserted in the obj ect will be referred to as a far end. The opposite side of the far end in the extending direction of the catheter 11 will be referred to as a near end.

The catheter unit 100 includes a near end cover 16 that covers the near end side of the catheter 11. The near end cover 16 has a tool hole 16a. A medical tool can be inserted in the catheter 11 through the tool hole 16a.

As described above, in the present embodiment, the catheter 11 has a function as a guide apparatus for guiding the medical tool medical tool to a desired position in the object.

For example, the catheter 11 is inserted to a target position in the object in a state in which an endoscope is inserted in the catheter 11. At this time, at least one of a manual operation by the user, movement of the moving stage 2a, and driving of the catheter 11 by the wire driving portion 300 is used. After the catheter 11 has reached the target position, the endoscope is pulled out of the catheter 11 through the tool hole 16a. Then, the medical tool is inserted through the tool hole 16a, and a work such as collection of various specimens from the inside of the object or treatment of the inside of the target is performed.

As will be described later, the catheter unit 100 is detachably attached to the catheter driving apparatus (base apparatus, main body), more specifically to the base unit 200. After the medical apparatus 1 is used, the user detaches the catheter unit 100 from the base unit 200, attaches a new catheter unit 100 to the base unit 200, and thus it becomes possible to use the medical apparatus 1 again. That is, the catheter unit 100 can be used as a disposable unit. Here, disposable means that the catheter unit 100 used in one time of treatment is disposed after being used. As a result of this, reuse of the catheter unit 100 is prevented, and thus the medical apparatus 1 can be always maintained in a clean state.

As illustrated in FIG. 2, the medical apparatus 1 includes an operation portion 400. In the present embodiment, the operation portion 400 is provided in the catheter unit 100. The operation portion 400 is operated by the user when fixing the catheter unit 100 to the base unit 200 or detaching the catheter unit 100 from the base unit 200.

By connecting the endoscope inserted in the catheter 11 to the monitor 4, an image captured by the endoscope can be displayed on the monitor 4. In addition, by connecting the monitor 4 to the control portion 3, the state of the medical apparatus 1 and information related to the control of the medical apparatus 1 can be displayed on the monitor 4. For example, the position of the catheter 11 in the object and information related to navigation of the catheter 11 in the object can be displayed on the monitor 4. The monitor 4, the control portion 3, and the endoscope may be connected to each other in a wired manner or in a wireless manner. In addition, the monitor 4 and the control portion 3 may be connected to each other via the supporting base 2.

### <Catheter>

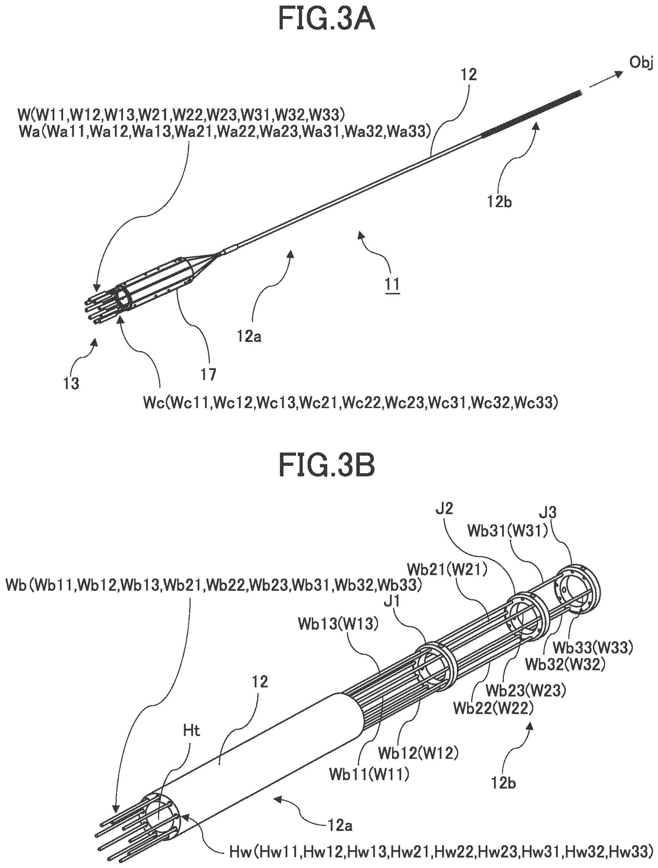

The catheter 11 serving as a bendable body will be described with reference to FIGS. 3A and 3B. FIGS. 3A and 3B are explanatory diagrams of the catheter 11. FIG. 3A is a diagram for describing the entirety of the catheter 11. FIG. 3B is an enlarged view of the catheter 11.

The catheter 11 includes a bending portion (bending body, catheter body) 12, and a bending driving portion (catheter driving portion) 13 configured to bend the bending portion 12. The bending driving portion 13 is configured to bend the bending portion 12 by receiving a driving force of the wire driving portion 300 via a coupling device 21 that will be described later.

The catheter 11 extends in the insertion direction of the catheter 11 with respect to the object (Obj). The extending direction (longitudinal direction) of the catheter 11 is the same as the extending direction (longitudinal direction) of the bending portion 12 and the extending direction (longitudinal direction) of first to ninth driving wires (W11 to W33) that will be described later.

The bending driving portion 13 includes a plurality of driving wires (driving lines, linear members, linear actuators) connected to the bending portion 12. Specifically, the bending driving portion 13 includes a first driving wire W11, a second driving wire W12, a third driving wire W13, a fourth driving wire W21, a fifth driving wire W22, a sixth driving wire W23, a seventh driving wire W31, an eighth driving wire W32, and a ninth driving wire W33.

The first to ninth driving wires (W11 to W33) each includes a held portion (held shaft, rod) Wa. Specifically, the first driving wire W11 includes a first held portion Wa11. The second driving wire W12 includes a second held portion Wa12. The third driving wire W13 includes a third held portion Wa13. The fourth driving wire W21 includes a fourth held portion Wa21. The fifth driving wire W22 includes a fifth held portion Wa22. The sixth driving wire W23 includes a sixth held portion Wa23. The seventh driving wire W31 includes a seventh held portion Wa31. The eighth driving wire W32 includes an eighth held portion Wa32. The ninth driving wire W33 includes a ninth held portion Wa33.

In the present embodiment, the first to ninth held portions (Wa11 to Wa33) each have the same shape.

The first to ninth driving wires (W11 to W33) each include a flexible wire body (wire member, line body, linear body) Wb. Here, the wire body Wb is a member through which a connected object can be pushed and pulled, and has stiffness of a certain degree. Meanwhile, the wire body Wb is a member capable of deforming from a linear shape so as to be able to bend the bending portion 12. The first driving wire W11 includes a first wire body Wb11. The second driving wire W12 includes a second wire body Wb12. The third driving wire W13 includes a third wire body Wb13. The fourth driving wire W21 includes a fourth wire body Wb21. The fifth driving wire W22 includes a fifth wire body Wb22. The sixth driving wire W23 includes a sixth wire body Wb23. The seventh driving wire W31 includes a seventh wire body Wb31. The eighth driving wire W32 includes an eighth wire body Wb32. The ninth driving wire W33 includes a ninth wire body Wb33.

Further, in the present embodiment, a connecting portion Wc that, for each pair of a drive source M and a driving wire W, transmits the driving force from the drive source to the wire body and cuts off the transmission of the driving force from the drive source to the linear body in the case where a load equal to or greater than a threshold value is applied. Specifically, the first wire body Wb11 is connected to a first drive source M11 via a first connecting portion Wc11. The second wire body Wb12 is connected to a second drive source M12 via a second connecting portion Wc12. The third wire body Wb13 is connected to a third drive source M13 via a third connecting portion Wc13. The fourth wire body Wb21 is connected to a fourth drive source M21 via a fourth connecting portion Wc21. The fifth wire body Wb22 is connected to a fifth drive source M22 via a fifth connecting portion Wc22. The sixth wire body Wb23 is connected to a sixth drive source M23 via a sixth connecting portion Wc23. The seventh wire body Wb31 is connected to a seventh drive source M31 via a seventh connecting portion Wc31. The eighth wire body Wb32 is connected to an eighth drive source M32 via an eighth connecting portion Wc32. The ninth wire body Wb33 is connected to a ninth drive source M33 via a ninth connecting portion Wc33.

Here, in each embodiment below, the threshold value is not limited to a fixed value (constant value) that is set in advance. Depending on the apparatus specifications and manufacture condition, there is a case where the threshold value hardly changes and there is a case where the threshold value dynamically changes. That is, although there is a target value set when designing a product, the threshold value for actually cutting off the connection between the drive source M and the wire body Wb can change more or less for each apparatus due to the influence of tolerance variations or the like. In addition, since these threshold values can change more or less due to the influence of the surrounding environment (temperature, humidity, etc.), the threshold values can change even for the same apparatus depending on the timing of operation. Therefore, the medical apparatus 1 described in each embodiment described above may be designed or manufactured in consideration of these influences such that the threshold value is within a predetermined range, and examples of the threshold value include dynamically changing ones. To be noted, the threshold value may be rephrased as a limit value.

The connecting portion Wc functions as a breakaway mechanism or a separation mechanism that cuts off the connection (coupling, drive transmission) between the drive source and the wire body in the case where a load exceeding a predetermined threshold value acts on the driving wire due to abnormal operation of the drive source or an external force on the catheter 11. The detailed configuration and action of the connecting portion Wc will be described later.

In the present embodiment, the first to third wire bodies (Wb11 to Wb13) each have the same shape. The fourth to sixth wire bodies (Wb21 to Wb23) each have the same shape. The seventh to ninth wire bodies (Wb31 to Wb33) each have the same shape. In the present embodiment, the first to ninth wire bodies (Wb11 to Wb33) each have the same shape except for the length thereof.

The first to ninth held portions (Wa11 to Wa33) are attached to the near ends of the first to ninth wire bodies (Wb11 to Wb33) via the first to ninth connecting portions (Wc11 to Wc33). The first to ninth driving wires (W11 to W33) are inserted in the bending portion 12 via the wire guide 17 and are fixed.

In the present embodiment, the material of each of the first to ninth wire bodies (Wb11 to Wb33) is metal. To be noted, the material of each of the first to ninth wire bodies (Wb11 to Wb33) may be resin. The material of each of the first to ninth wire bodies (Wb11 to Wb33) may include metal and resin.

Arbitrary one of the first to ninth driving wires (W11 to W33) can be referred to as a driving wire W. In the present embodiment, the first to ninth driving wires (W11 to W33) each have the same shape except for the lengths of the first to ninth wire bodies (Wb11 to Wb33).

In the present embodiment, the bending portion 12 is a tubular member having flexibility and having a pathway Ht for inserting the medical tool.

A plurality of wire holes for respectively allowing the first to ninth driving wires (W11 to W33) to pass through are provided in a wall surface of the bending portion 12. Specifically, a first wire hole Hw11, a second wire ole Hw12, and a third wire hole Hw13 are provided in the wall surface of the bending portion 12. Further, a fourth wire hole Hw21, a fifth wire hole Hw22, and a sixth wire hole Hw23 are provided in the wall surface of the bending portion 12. Further, a seventh wire hole Hw31, an eighth wire hole Hw32, and a ninth wire hole Hw33 are provided in the wall surface of the bending portion 12. The first to ninth wire holes Hw (Hw11 to Hw33) respectively correspond to the first to ninth driving wires (W11 to W33). The suffices of the reference sign Hw indicate the numbers of the corresponding driving wires. For example, the first driving wire W11 is inserted in the first wire hole Hw11.

Arbitrary one of the first to ninth wire holes (Hw11 to Hw33) can be referred to as a wire hole Hw. In the present embodiment, the first to ninth wire holes (Hw11 to Hw33) each have the same shape.

The bending portion 12 has an intermediate region 12a and a bending region 12b. The bending region 12b is disposed at the far end of the bending portion 12, and a first guide ring J1, a second guide ring J2, and a third guide ring J3 are disposed in the bending region 12b. The bending region 12b is a region where the degree and direction of bending of the bending portion 12 can be controlled by moving the first guide ring J1, the second guide ring J2, and the third guide ring J3 by the bending driving portion 13. FIG. 3B illustrates the bending portion 12 by omitting part thereof covering the first to third guide rings (J1 to J3).

In the present embodiment, the bending portion 12 includes a plurality of auxiliary rings (not illustrated). In the bending region 12b, the first guide ring J1, the second guide ring J2, and the third guide ring J3 are fixed to the wall surface of the bending portion 12. In the present embodiment, the plurality of auxiliary rings are disposed between the first guide ring J1 and the second guide ring J2 and between the second guide ring J2 and the third guide ring J3.

The medical tool is guided to the distal end of the catheter 11 by the pathway Ht, the first to third guide rings (J1 to J3), and the plurality of auxiliary rings.

The first to ninth driving wires (W11 to W33) are respectively fixed to the first to third guide rings (J1 to J3) through the intermediate region 12a. Specifically, the first driving wire W11, the second driving wire W12, and the third driving wire W13 are fixed to the first guide ring J1. The fourth driving wire W21, the fifth driving wire W22, and the sixth driving wire W23 penetrate the first guide ring J1 and the plurality of auxiliary rings, and are fixed to the second guide ring J2. The seventh driving wire W31, the eighth driving wire W32, and the ninth driving wire W33 penetrate the first guide ring J1, the second guide ring J2, and the plurality of auxiliary rings, and are fixed to the third guide ring J3.

The medical apparatus 1 can bend the bending portion 12 toward a direction intersecting with the extending direction of the catheter 11 by driving the bending driving portion 13 by the wire driving portion 300. Specifically, by moving each of the first to ninth driving wires (W11 to W33) in the extending direction of the bending portion 12, the bending region 12b of the bending portion 12 can be bent in a direction intersecting with the extending direction via the first to third guide rings (J1 to J3).

The user can insert the catheter 11 to a target portion in the obj ect by using at least one of movement of the medical apparatus 1 moved manually or by the moving stage 2a and bending of the bending portion 12.

To be noted, although the bending portion 12 is bent by moving the first to third guide rings (J1 to J3) by the first to ninth driving wires (W11 to W33) in the present embodiment, the configuration is not limited to this. One or two of the first to third guide rings (J1 to J3) and driving wires fixed thereto may be omitted.

For example, the catheter 11 may have a configuration in which the seventh to ninth driving wires (W31 to W33) and the third guide ring J3 are provided and the first to sixth driving wires (W11 to W23) and the first and second guide rings (J1 to J2) are omitted. In addition, the catheter 11 may have a configuration in which the fourth to ninth driving wires (W21 to W33) and the second and third guide rings J2 and J3 are provided and the first to third driving wires (W11 to W13) and the first guide ring (J1) is omitted.

In addition, the catheter 11 may have a configuration in which one guide ring is driven by two driving wires. Also in this case, the number of guide rings may be equal to or more than one.

### <Catheter Unit>

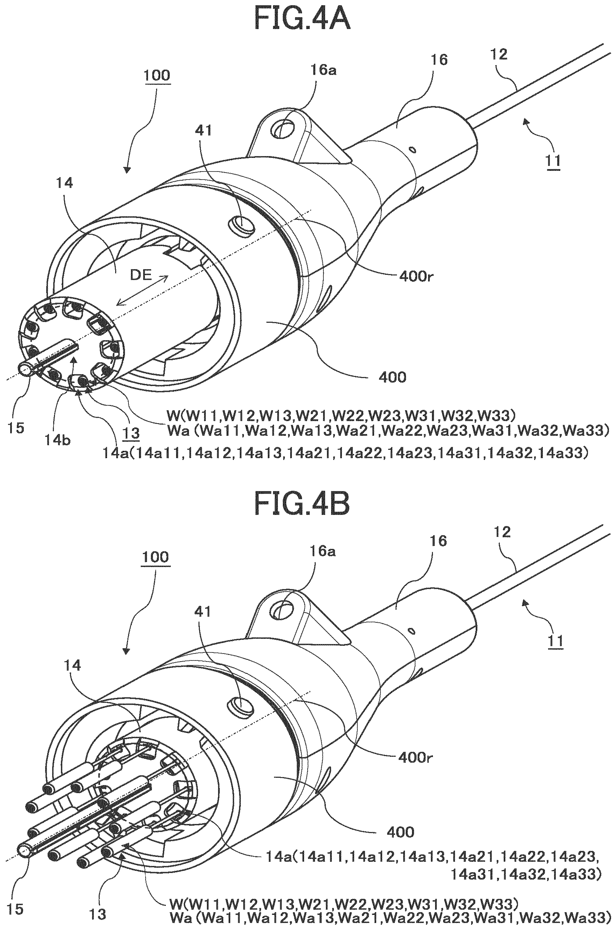

The catheter unit 100 will be described with reference to FIGS. 4A and 4B. FIGS. 4Aand 4B are explanatory diagrams of the catheter unit 100. FIG. 4Ais an explanatory diagram of the catheter unit 100 in a state in which a wire cover 14 that will be described later is at a covering position. FIG. 4B is an explanatory diagram of the catheter unit 100 in a state in which the wire cover 14 that will be described later is at an exposing position.

The catheter unit 100 includes the bending portion 12, the catheter 11 including the bending driving portion 13, and a near end cover 16 supporting the near end of the catheter 11. The catheter unit 100 includes a cover (wire cover) 14 for covering and protecting the first to ninth driving wires (W11 to W33) serving as a plurality of driving wires.

The catheter unit 100 is attachable to and detachable from the base unit 200 in an attachment/detachment direction DE. The attachment direction of the catheter unit 100 to the base unit 200 and the detachment direction of the catheter unit 100 from the base unit 200 are parallel to the attachment/detachment direction DE.

The near end cover (frame body, bending portion casing, catheter casing) 16 is a cover covering a part of the catheter 11. The near end cover 16 includes a tool hole 16a for inserting a medical tool into the pathway Ht of the bending portion 12.

The wire cover 14 is provided with a plurality of exposing holes (wire cover holes, cover holes) for allowing the first to ninth driving wires (W11 to W33) to pass through. The wire cover 14 is provided with a first exposing hole 14a11, a second exposing hole 14a12, a third exposing hole 14a13, a fourth exposing hole 14a21, a fifth exposing hole 14a22, a sixth exposing hole 14a23, a seventh exposing hole 14a31, and an eighth exposing hole 14a32, and a ninth exposing hole 14a33. The first to ninth exposing holes (14a11 to 14a33) respectively correspond to the first to ninth driving wires (W11 to W33). The suffix numerals after the reference sign 14a indicate the numerals of the corresponding driving wires. For example, the first driving wire W11 is inserted in the first exposing hole 14a11.

Arbitrary one of the first to ninth exposing holes (14a11 to 14a33) can be referred to as an exposing hole 14a. In the present embodiment, the first to ninth exposing holes (14a11 to 14a33) each have the same shape.

The wire cover 14 can move to a covering position (see FIG. 4A) covering the first to ninth driving wires (W11 to W33), and a cover retraction position (FIG. 4B) retracted from the covering position. The cover retraction position can be also referred to as an exposing position where the first to ninth driving wires (W11 to W33) are exposed.

Before attaching the catheter unit 100 to the base unit 200, the wire cover 14 is positioned at the covering position. When the catheter unit 100 is attached to the base unit 200, the wire cover 14 moves from the covering position to the exposing position in the attachment/detachment direction DE.

In the present embodiment, the wire cover 14 is maintained at the exposing position after moving from the covering position to the exposing position. Therefore, the wire cover 14 is maintained at the exposing position even if the catheter unit 100 is detached from the base unit 200 after the catheter unit 100 is attached to the base unit 200.

However, the wire cover 14 may be configured to return to the covering position after moving from the covering position to the exposing position. For example, the catheter unit 100 may include an urging member that urges the wire cover 14 from the exposing position to the covering position. In this case, when the catheter unit 100 is detached from the base unit 200 after the catheter unit 100 is attached to the base unit 200, the wire cover 14 is moved from the exposing position to the covering position.

When the wire cover 14 is at the exposing position, the first to ninth held portions (Wa11 to Wa33) of the first to ninth driving wires (W11 to W33) are exposed. As a result of this, the coupling between the bending driving portion 13 and a coupling device 21 that will be described later is allowed. When the wire cover 14 is at the exposing position, the first to ninth held portions (Wa11 to Wa33) of the first to ninth driving wires (W11 to W33) project from the first to ninth exposing holes (14a11 to 14a33). More specifically, the first to ninth held portions (Wa11 to Wa33) project from the first to ninth exposing holes (14a11 to 14a33) in an attachment direction Da that will be described later.

As illustrated in FIG. 4B, the first to ninth driving wires (W11 to W33) are each arranged along a circle (virtual circle) having a predetermined radius.

In the present embodiment, the catheter unit 100 includes a key shaft (key, catheter-side key) 15. In the present embodiment, the key shaft 15 extends in the attachment/detachment direction DE. The wire cover 14 is provided with a shaft hole 14b through which the key shaft 15 passes. The key shaft 15 is capable of engaging with a key receiving portion 22 that will be described later. As a result of the key shaft 15 engaging with the key receiving portion 22, the movement of the catheter unit 100 with respect to the base unit 200 is limited to a predetermined range in a circumferential direction of the circle (virtual circle) on which the first to ninth driving wires (W11 to W33) are arranged.

In the present embodiment, the first to ninth driving wires (W11 to W33) are disposed outside the key shaft 15 so as to surround the key shaft 15 as viewed in the attachment/detachment direction DE. In other words, the key shaft 15 is disposed inside the circle (virtual circle) on which the first to ninth driving wires (W11 to W33) are arranged. Therefore, the key shaft 15 and the first to ninth driving wires (W11 to W33) can be disposed in a small space.

In the present embodiment, the catheter unit 100 includes an operation portion 400. The operation portion 400 is configured to be movable (rotatable) with respect to the near end cover 16 and the bending driving portion 13. The operation portion 400 is, the operation portion 400 is rotatable about a rotation shaft 400r. The rotation shaft 400r of the operation portion 400 extends in the attachment/detachment direction DE.

The operation portion 400 is configured to movable (rotatable) with respect to the base unit 200 in a state in which the catheter unit 100 is attached to the base unit 200. More specifically, the operation portion 400 is configured to be movable (rotatable) with respect to the base casing 200f, the wire driving portion 300, and the coupling device 21 that will be described later.

### <Base Unit>

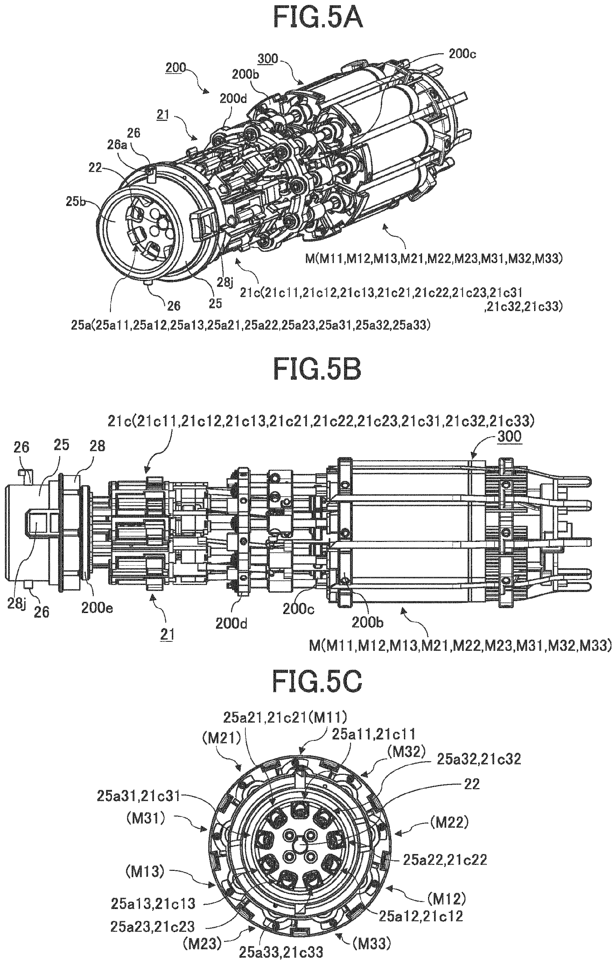

The base unit 200 and the wire driving portion 300 will be described with reference to FIGS. 5A to 5C. FIGS. 5A to 5C are explanatory diagrams of the base unit 200 and the wire driving portion 300. FIG. 5Ais a perspective view illustrating an inner structure of the base unit 200. FIG. 5B is a side view illustrating the inner structure of the base unit 200. FIG. 5C is a diagram illustrating the base unit 200 as viewed in the attachment/detachment direction DE.

As described above, the medical apparatus 1 includes the base unit 200 and the wire driving portion 300. In the present embodiment, the wire driving portion 300 is accommodated in the base casing 200f, and is provided inside the base unit 200. In other words, the base unit 200 includes the wire driving portion 300.

The wire driving portion 300 includes a plurality of drive sources (motors, actuators). In the present embodiment, the wire driving portion 300 includes a first drive source M11, a second drive source M12, a third drive source M13, a fourth drive source M21, a fifth drive source M22, a sixth drive source M23, a seventh drive source M31, an eighth drive source M32, and a ninth drive source M33.

Arbitrary one of the first to ninth drive sources (M11 to M33) can be referred to as a drive source M. In the present embodiment, the first to ninth drive sources (M11 to M33) each have the same configuration.

The base unit 200 includes the coupling device 21. The coupling device 21 is accommodated in the base casing 200f. The coupling device 21 is connected to the wire driving portion 300. The coupling device 21 includes a plurality of coupling portions. In the present embodiment, the coupling device 21 includes a first coupling portion 21c11, a second coupling portion 21c12, a third coupling portion 21c13, a fourth coupling portion 21c21, a fifth coupling portion 21c22, a sixth coupling portion 21c23, a seventh coupling portion 21c31, an eighth coupling portion 21c32, and a ninth coupling portion 21c33.

Arbitrary one of the first to ninth coupling portions (21c11 to 21c33) can be referred to as a coupling portion 21c. In the present embodiment, the first to ninth coupling portions (21c11 to 21c33) each have the same configuration.

The plurality of coupling portions are respectively connected to the plurality of drive sources, and are respectively driven by the plurality of drive sources. Specifically, the first coupling portion 21c11 is connected to the first drive source M11, and is driven by the first drive source M11. The second coupling portion 21c12 is connected to the second drive source M12, and is driven by the second drive source M12. The third coupling portion 21c13 is connected to the third drive source M13, and is driven by the third drive source M13. The fourth coupling portion 21c21 is connected to the fourth drive source M21, and is driven by the fourth drive source M21. The fifth coupling portion 21c22 is connected to the fifth drive source M22, and is driven by the fifth drive source M22. The sixth coupling portion 21c23 is connected to the sixth drive source M23, and is driven by the sixth drive source M23. The seventh coupling portion 21c31 is connected to the seventh drive source M31, and is driven by the seventh drive source M31. The eighth coupling portion 21c32 is connected to the eighth drive source M32, and is driven by the eighth drive source M32. The ninth coupling portion 21c33 is connected to the ninth drive source M33, and is driven by the ninth drive source M33.

As will be described later, the coupling device 21 is coupled to the bending driving portion 13 including the first to ninth driving wires (W11 to W33). The bending driving portion 13 receives the driving force of the wire driving portion 300 via the coupling device 21 and bends the bending portion 12.

The driving wire W is coupled to the coupling portion 21c via the held portion Wa. The plurality of driving wires are respectively coupled to the plurality of coupling portions. Specifically, the first held portion Wa11 of the first driving wire W11 is coupled to the first coupling portion 21c11. The second held portion Wa12 of the second driving wire W12 is coupled to the second coupling portion 21c12. The third held portion Wa13 of the third driving wire W13 is coupled to the third coupling portion 21c13. The fourth held portion Wa21 of the fourth driving wire W21 is coupled to the fourth coupling portion 21c21. The fifth held portion Wa22 of the fifth driving wire W22 is coupled to the fifth coupling portion 21c22. The sixth held portion Wa23 of the sixth driving wire W23 is coupled to the sixth coupling portion 21c23. The seventh held portion Wa31 of the seventh driving wire W31 is coupled to the seventh coupling portion 21c31. The eighth held portion Wa32 of the eighth driving wire W32 is coupled to the eighth coupling portion 21c32. The ninth held portion Wa33 of the ninth driving wire W33 is coupled to the ninth coupling portion 21c33.

The base unit 200 includes a base frame 25. The base frame 25 is provided with a plurality of insertion holes for respectively allowing the first to ninth driving wires (W11 to W33) to pass through. The base frame 25 has a first insertion hole 25a11, a second insertion hole 25a12, a third insertion hole 25a13, a fourth insertion hole 25a21, a fifth insertion hole 25a22, a sixth insertion hole 25a23, a seventh insertion hole 25a31, an eighth insertion hole 25a32, and a ninth insertion hole 25a33. The first to ninth insertion holes (25a11 to 25a33) respectively correspond to the first to ninth driving wires (W11 to W33). The suffices of the reference sign 25a indicate the numerals of the corresponding driving wires. For example, the first driving wire W11 is inserted in the first insertion hole 25a11.

Arbitrary one of the first to ninth insertion holes (25a11 to 25a33) can be referred to as an insertion hole 25a. In the present embodiment, the first to ninth insertion holes (25a11 to 25a33) each have the same shape.

The base frame 25 is provided with an attachment opening 25b in which the wire cover 14 is inserted. The first to ninth insertion holes (25a11 to 25a33) are provided at a bottom portion of the attachment opening 25b.

Further, the base unit 200 includes a motor frame 200b, a first bearing frame 200c, a second bearing frame 200d, and a third bearing frame 200e. The motor frame 200b, the first bearing frame 200c, the second bearing frame 200d, and the third bearing frame 200e are coupled.

The base frame 25 includes a key receiving portion (key hole, base-side key, body-side key) 22 that receives the key shaft 15. As a result of the key shaft 15 and the key receiving portion 22 engaging with each other, the catheter unit 100 is prevented from being attached to the base unit 200 in a wrong phase.

As a result of the key shaft 15 and the key receiving portion 22 engaging with each other, the movement of the catheter unit 100 with respect to the base unit 200 is limited to a predetermined range in the circumferential direction of the circle (virtual circle) on which the first to ninth driving wires (W11 to W33) are arranged.

As a result, the first to ninth driving wires (W11 to W33) respectively engage with the corresponding ones of the first to ninth insertion holes (25a11 to 25a33) and the corresponding ones of the first to ninth coupling portions (21c11 to 21c33). In other words, the driving wire W is prevented from engaging with an insertion hole 25a different from the corresponding insertion hole 25a or 21c different from the corresponding coupling portion 21c.

The user can correctly couple the first to ninth driving wires (W11 to W33) respectively to the first to ninth coupling portions (21c11 to 21c33) by engaging the key shaft 15 and the key receiving portion 22 with each other. Therefore, the user can easily attach the catheter unit 100 to the base unit 200.

In the present embodiment, the key shaft 15 includes a protrusion portion protruding in a direction intersecting with the attachment/detachment direction DE, and the key receiving portion 22 has a recess portion in which the protrusion portion is inserted. In the circumferential direction, a position where the protrusion portion and the recess portion engage with each other is a position where the driving wire W engages with the corresponding insertion hole 25a and the corresponding coupling portion 21c.

To be noted, the key shaft 15 can be provided at one of the base unit 200 and the catheter unit 100, and the key receiving portion 22 can be provided at the other. For example, the key shaft 15 may be provided on the base unit 200 side, and the key receiving portion 22 may be provided on the catheter unit 100 side.

The base unit 200 includes a j oint 28 including a joint engagement portion 28j. The base frame 25 includes a locking shaft 26 including a locking protrusion 26a. Functions of these will be described later.

### <Coupling between Motors and Driving Wires>

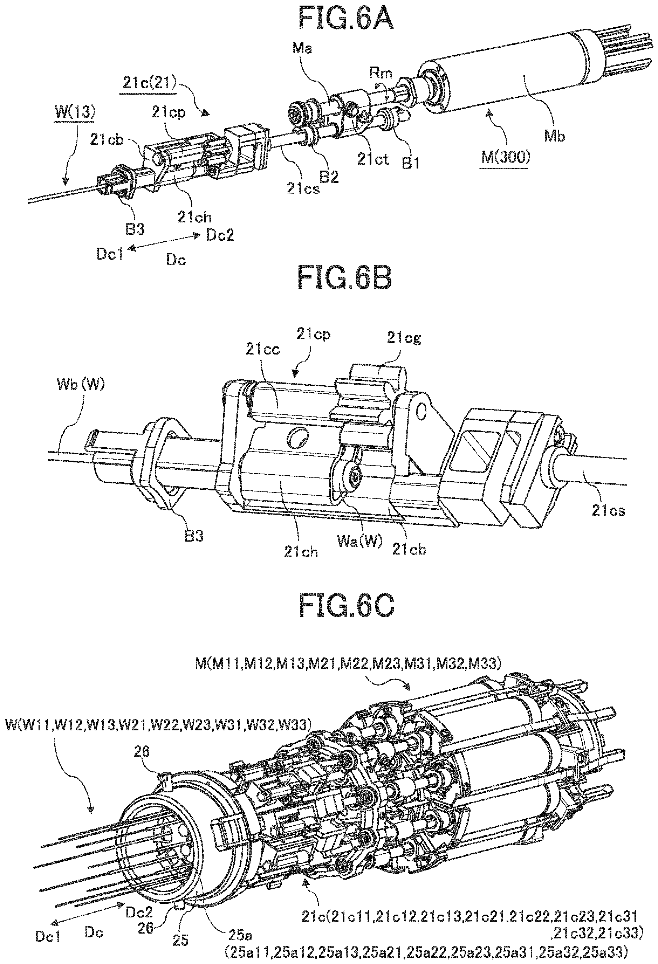

Coupling between the wire driving portion 300, the coupling device 21, and the bending driving portion 13 will be described with reference to FIGS. 6A to 6C. FIGS. 6A to 6C are explanatory diagrams of the wire driving portion 300, the coupling device 21, and the bending driving portion 13. FIG. 6A is a perspective view of the drive source M, the coupling portion 21c, and the driving wire W. FIG. 6B is an enlarged view of the coupling portion 21c and the driving wire W. FIG. 6C is a perspective view illustrating the coupling between the wire driving portion 300, the coupling device 21, and the bending driving portion 13.

In the present embodiment, the configurations of coupling respectively between the first to ninth driving wires (W11 to W33) and the first to ninth coupling portions (21c11 to 21c33) are the same. In addition, the configurations of respective connection between the first to ninth coupling portions (21c11 to 21c33) and the first to ninth drive sources (M11 to M33) are the same. Therefore, in the description below, the configurations of connection between these will be described by using one driving wire W, one coupling portion 21c, and one drive source M.

As illustrated in FIG. 6A, the drive source M includes an output shaft Ma and a motor body Mb that rotates the output shaft Ma in the rotation direction Rm. A spiral groove is provided on the surface of the output shaft Ma. The output shaft Ma has a so-called screw shape. The motor body Mb is fixed to the motor frame 200b.

The coupling portion 21c includes a tractor 21ct connected to the output shaft Ma and a tractor support shaft 21cs that supports the tractor 21ct. The tractor support shaft 21cs is connected to a coupling base 21cb.

The coupling portion 21c includes a plate spring 21ch serving as a holding portion for holding the held portion Wa of the driving wire W. The driving wire W is engaged with the coupling portion 21c through the insertion hole 25a. More specifically, the held portion Wa engages with the plate spring 21ch. As will be described later, the plate spring 21ch can take a state (fixing state) of sandwiching and fixing the held portion Wa and a state (releasing state) of releasing the held portion Wa.

The coupling portion 21c includes a pressing member 21cp. The pressing member 21cp includes a gear portion 21cg that engages with an internal gear 29 that will be described later, and a cam 21cc serving as a pressing portion for pressing the plate spring 21ch.

As will be described later, the cam 21cc is capable of moving with respect to the plate spring 21ch. As a result of the cam 21cc moving, the fixing state and the releasing state of the plate spring 21ch can be switched.

The coupling portion 21c is supported by a first bearing B1, a second bearing B2, and a third bearing B3. The first bearing B1 is supported by a first bearing frame 200c of the base unit 200. The second bearing B2 is supported by a second bearing frame 200d of the base unit 200. The third bearing B3 is supported by a third bearing frame 200e of the base unit 200. Therefore, when the output shaft Ma rotates in a rotation direction Rm, rotation of the coupling portion 21c about the output shaft Ma is restricted. To be noted, the first bearing B1, the second bearing B2, and the third bearing B3 are provided for each of the first to ninth coupling portions (21c11 to 21c33).

Since the rotation of the coupling portion 21c about the output shaft Ma is restricted, a force in the rotation axis direction of the output shaft Ma acts on the tractor 21ct due to the spiral groove of the output shaft Ma when the output shaft Ma rotates. As a result, the coupling portion 21c moves in the rotation axis direction (Dc direction) of the output shaft Ma. As a result of the coupling portion 21c moving, the driving wires W moves, and the bending portion 12 bends. At this time, by switching the rotation direction of the drive source M, the coupling portion 21c can drive the driving wire W in both a direction to press the driving wire W (Dc1 direction) and a direction to pull the driving wire W (Dc2 direction).

That is, the output shaft Ma and the tractor 21ct constitute a so-called feed screw that converts the rotational motion transmitted from the drive source M into a linear motion by the screw. In the present embodiment, the output shaft Ma and the tractor 21ct are a slide screw, but may be a ball screw.

As illustrated in FIG. 6C, by attaching the catheter unit 100 to the base unit 200, the first to ninth driving wires (W11 to W33) are respectively coupled to the first to ninth coupling portions (21c11 to 21c33).

The control portion 3 can control the first to ninth drive sources (M11 to M33) independently from each other. That is, an arbitrary drive source among the first to ninth drive sources (M11 to M33) can be independently operated or stopped regardless of whether or not the other drive sources are stopped. In other words, the control portion 3 can control the first to ninth driving wires (W11 to W33) independently from each other. As a result, the first to third guide rings (J1 to J3) are controlled independently from each other, and the bending region 12b of the bending portion 12 can be bent in an arbitrary direction.

### <Attachment of Catheter Unit>

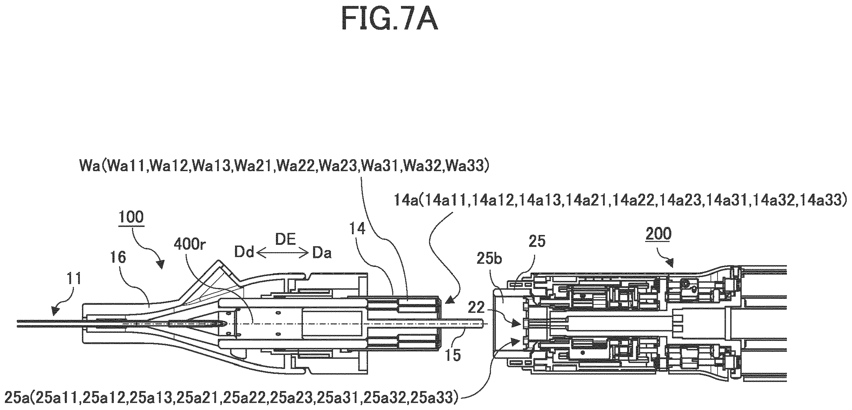

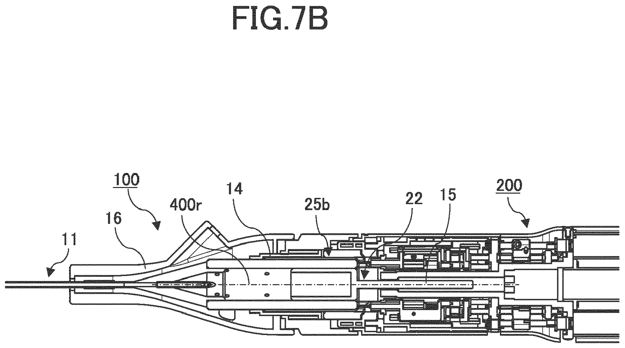

An operation of attaching the catheter unit 100 to the base unit 200 will be described with reference to FIGS. 7A and 7B. FIGS. 7A and 7B are explanatory diagrams of attachment of the catheter unit 100. FIG. 7A is a diagram before the catheter unit 100 is attached to the base unit 200. FIG. 7B is a diagram after the catheter unit 100 is attached to the base unit 200.

In the present embodiment, the attachment/detachment direction DE of the catheter unit 100 is the same as the direction of the rotation shaft 400r of the operation portion 400. In the attachment/detachment direction DE, a direction in which the catheter unit 100 is attached to the base unit 200 will be referred to as an attachment direction Da. In the attachment/detachment direction DE, a direction in which the catheter unit 100 is detached from the base unit 200 (direction opposite to the attachment direction Da) will be referred to as a detachment direction Dd.

As illustrated in FIG. 7A, in a state before the catheter unit 100 is attached to the base unit 200, the wire cover 14 is positioned at the covering position. At this time, the wire cover 14 covers the first to ninth driving wires (W11 to W33) such that the first to ninth held portions (Wa11 to Wa33) do not project from the first to ninth exposing holes (14a11 to 14a33) of the wire cover 14. Therefore, the first to ninth driving wires (W11 to W33) can be protected in a state before the catheter unit 100 is attached to the base unit 200.

When the catheter unit 100 is attached to the base unit 200, the key shaft 15 is caused to engage with the key receiving portion 22. The key shaft 15 projects from the wire cover 14. In the present embodiment, the wire cover 14 does not engage with an attachment opening 25b in a state in which the key shaft 15 has reached an entrance of the key receiving portion 22. That is, when the phase of the catheter unit 100 with respect to the base unit 200 is a phase in which the key shaft 15 and the key receiving portion 22 cannot engage with each other, the wire cover 14 does not engage with the attachment opening 25b, and the state of being positioned at the covering position is maintained. Therefore, the first to ninth driving wires (W11 to W33) are protected even in the case where the catheter unit 100 is moved such that the key shaft 15 and the key receiving portion 22 engage with each other.

When the key shaft 15 and the key receiving portion 22 engage with each other and the catheter unit 100 moves in the attachment direction Da with respect to the base unit 200, the catheter unit 100 is attached to the base unit 200. By attaching the catheter unit 100 to the base unit 200, the wire cover 14 moves to the exposing position. In the present embodiment, the wire cover 14 moves from the covering position to the exposing position by abutting the base frame 25 (see FIG. 7B).

More specifically, when attaching the catheter unit 100, the wire cover 14 stops by abutting the base frame 25. In this state, by moving the catheter unit 100 in the attachment direction Da, the wire cover 14 relatively moves with respect to parts other than the wire cover 14 in the catheter unit 100. As a result, the wire cover 14 moves from the covering position to the exposing position.

While the wire cover 14 moves from the covering position to the exposing position, the held portion Wa of the driving wire W projects from the exposing hole 14a of the wire cover 14, and is inserted in the insertion hole 25a. Then, the held portion Wa engages with the plate spring 21ch of the coupling portion 21c of the held portion Wa (see FIG. 6B).

In a state in which the catheter unit 100 is merely attached to the base unit 200, the catheter unit 100 can be detached from the catheter unit 100 by moving the catheter unit 100 in the detachment direction Dd with respect to the base unit 200. In addition, as will be described later, in a state in which the catheter unit 100 is merely attached to the base unit 200, the fixation between the driving wire W and the coupling portion 21c is cancelled.

Detachment of the catheter unit 100 from the base unit 200 can be prevented by operating the operation portion 400 in a state in which the catheter unit 100 is attached to the base unit 200. Further, by operating the operation portion 400 in a state in which the catheter unit 100 is attached to the base unit 200, the bending driving portion 13 is fixed to the coupling device 21, and the bending driving portion 13 is coupled to the wire driving portion 300 via the coupling device 21.

### <Fixation of Bending Driving Portion and Cancellation of Fixation>

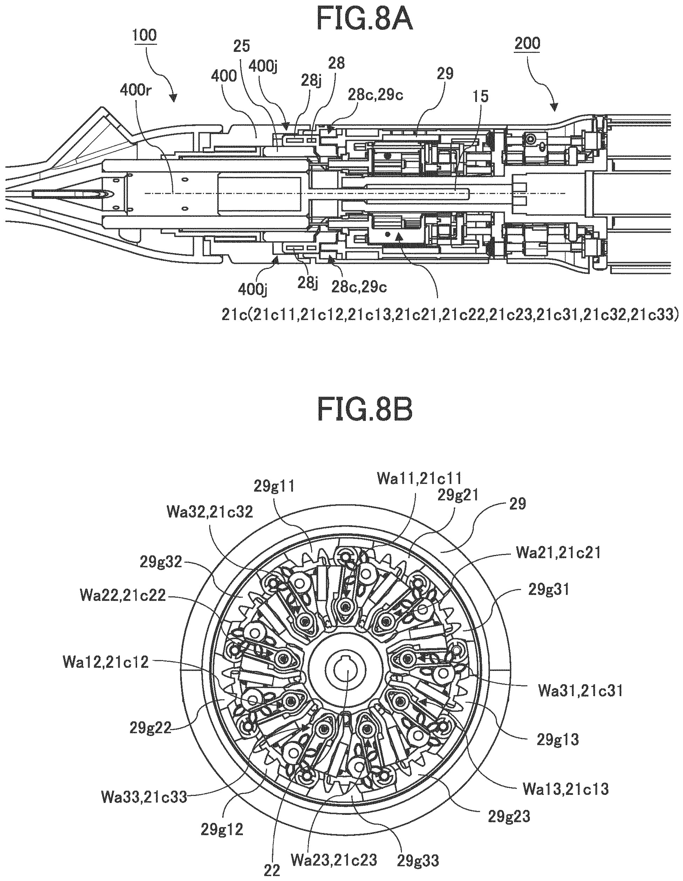

Elements for fixing the bending driving portion 13 to the coupling device 21 and elements for cancelling the fixation of the bending driving portion 13 by the coupling device 21 will be described with reference to FIGS. 8A, 8B, 9, 10, 11, 12, 13, and 14.

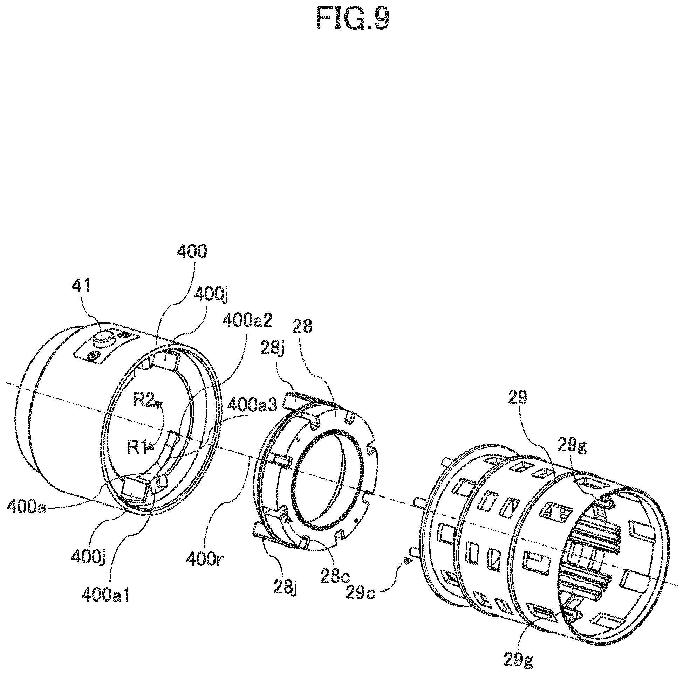

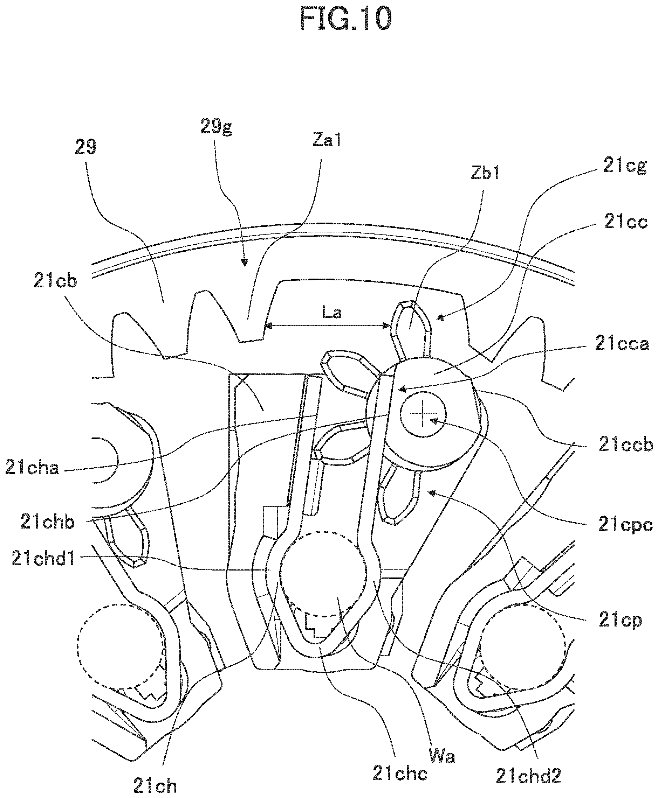

FIGS. 8A and 8B are diagrams for describing the coupling between the catheter unit 100 and the base unit 200. FIG. 8A is a section view of the catheter unit 100 and the base unit 200. FIG. 8A is a section view of the catheter unit 100 and the base unit 200 taken along the rotation shaft 400r. FIG. 8B is a section view of the base unit 200. It's a section view of the base unit 200 taken at a part of the coupling portion 21c in a direction orthogonal to the rotation shaft 400r. FIG. 9 is an exploded view for describing the coupling between the catheter unit 100 and the base unit 200. FIGS. 10, 11, 12, 13, and 14 are diagrams for describing the fixation of the driving wire W by the coupling portion 21c.

As illustrated in FIGS. 8A and 9, the base unit 200 includes the joint 28 (intermediate member, second transmission member) 28, and the internal gear 29 serving as a moving gear (interlocking gear, transmission member, first transmission member) that moves in an interlocked manner with the operation portion 400 via the joint 28.

The joint 28 includes a plurality of transmission portions 28c, and the internal gear 29 includes a plurality of transmitted portions 29c. The plurality of transmission portions 28c are engaged with the plurality of transmitted portions 29c, and in the case where the joint 28 rotates, the rotation of the joint 28 is transmitted to the internal gear 29.

When the catheter unit 100 is attached to the base unit 200, an engagement portion 400j provided on the operation portion 400 engages with a joint engagement portion 28j of the joint 28. In the case where the operation portion 400 rotates, the rotation of the operation portion 400 is transmitted to the joint 28. The operation portion 400, the joint 28, and the internal gear 29 rotate in the same direction.

The internal gear 29 includes a plurality of tooth portions for switching the first to ninth coupling portions (21c11 to 21c33) between a state of respectively fixing the first to ninth driving wires (W11 to W33) and a state of respectively releasing the first to ninth driving wires (W11 to W33). The plurality of tooth portions (acting portions, switching gear portions) of the internal gear 29 respectively engage with the gear portions 21cg of the pressing members 21cp of the first to ninth coupling portions (21c11 to 21c33).

Specifically, in the present embodiment, the internal gear 29 includes a first tooth portion 29g11, a second tooth portion 29g12, a third tooth portion 29g13, a fourth tooth portion 29g21, a fifth tooth portion 29g22, a sixth tooth portion 29g23, a seventh tooth portion 29g31, an eighth tooth portion 29g32, and a ninth tooth portion 29g33. The first to ninth tooth portions (29g11 to 29g33) are formed with gaps therebetween.

The first tooth portion 29g11 engages with the gear portion 21cg of the first coupling portion 21c11. The second tooth portion 29g12 engages with the gear portion 21cg of the second coupling portion 21c12. The third tooth portion 29g13 engages with the gear portion 21cg of the third coupling portion 21c13. The fourth tooth portion 29g21 engages with the gear portion 21cg of the fourth coupling portion 21c21. The fifth tooth portion 29g22 engages with the gear portion 21cg of the fifth coupling portion 21c22. The sixth tooth portion 29g23 engages with the gear portion 21cg of the sixth coupling portion 21c23. The seventh tooth portion 29g31 engages with the gear portion 21cg of the seventh coupling portion 21c31. The eighth tooth portion 29g32 engages with the gear portion 21cg of the eighth coupling portion 21c32. The ninth tooth portion 29g33 engages with the gear portion 21cg of the ninth coupling portion 21c33.

Arbitrary one of the first to ninth tooth portions (29g11 to 29g33) can be referred to as a tooth portion 29g. In the present embodiment, the first to ninth tooth portions (29g11 to 29g33) each have the same configuration.

In the present embodiment, configurations in which the first to ninth driving wires (W11 to W33) are respectively coupled to the first to ninth coupling portions (21c11 to 21c33) are the same. In addition, configurations in which the first to ninth coupling portions (21c11 to 21c33) are respectively connected to the first to ninth tooth portions (29g11 to 29g33) are the same. Therefore, in the description below, a configuration in which one driving wire W, one coupling portion 21c, and one tooth portion 29g are connected will be described below.

In each of the first to ninth coupling portions (21c11 to 21c33), as a result of the gear portion 21cg being moved by the internal gear 29, the pressing member 21cp rotates, and the cam 21cc moves to a pressing position and a retracted position retracted from the pressing position.

By rotating the operation portion 400, the internal gear 29 rotates. As a result of the internal gear 29 rotating, the first to ninth coupling portions (21c11 to 21c33) each operate. That is, by an operation of rotating one operation portion 400, the first to ninth coupling portions (21c11 to 21c33) can be operated.

The operation portion 400 can move to a fixation position (locking position) and a detachment position in a state in which the catheter unit 100 is attached to the base unit 200. In addition, as will be described later, the operation portion 400 can move to a release position in a state in which the catheter unit 100 is attached to the base unit 200. The release position is positioned between the fixation position and the detachment position in the circumferential direction of the operation portion 400. In a state in which the operation portion 400 is at the detachment position, the catheter unit 100 is attached to the base unit 200.

In a state in which the catheter unit 100 is attached to the base unit 200, the fixation (lock) of the driving wire W to the coupling portion 21c is cancelled. This state will be referred to as a released state of the coupling portion 21c. To be noted, a state in which the driving wire W is fixed (locked) to the coupling portion 21c will be referred to as a locked state of the coupling portion 21c.

The operation when fixing the driving wire W to the coupling portion 21c will be described with reference to FIGS. 10, 11, 12, 13, and 14.

In a state after the catheter unit 100 is attached to the base unit 200 and before the operation portion 400 is operated, the catheter unit 100 can be detached from the base unit 200. A state in which the catheter unit 100 can be detached from the base unit 200 will be referred to as a detachable state.

FIG. 10 is a diagram illustrating a state of the internal gear 29 and the coupling portion 21c in the detachable state. FIG. 10 is a diagram illustrating the internal gear 29 and the coupling portion 21c in the state in which the operation portion 400 is positioned at the fixation position.

The plate spring 21ch of the coupling portion 21c includes a fixed portion 21 cha fixed to the coupling base 21cb and a pressed portion 21chb that abuts the cam 21cc of the pressing member 21cp. The plate spring 21ch includes a first portion 21chd1 and a second portion 21chd2. When the catheter unit 100 is attached to the base unit 200, the held portion Wa is inserted between the first portion 21chd1 and the second portion chd2.

The cam 21cc has a holding surface 21cca and a pressing surface 21ccb. In the rotational radius direction of the pressing member 21cp, the holding surface 21cca is disposed at a position closer to a rotational center 21cpc of the pressing member 21cp than the pressing surface 21ccb.

As illustrated in FIG. 10, in the detachable state (state in which the operation portion 400 is at the detachment position), the plate spring 21ch is held at a position where the pressed portion 21chb is abutting the holding surface 21cca. In addition, a tooth Za1 of the internal gear 29 and a tooth Zb1 of the gear portion 21cg are stopped with a clearance La therebetween.

In the rotation direction of the operation portion 400, a direction in which the operation portion 400 moves from the detachment position to the release position and the fixation position will be referred to as a locking direction (fixing direction), and a direction in which the operation portion 400 moves from the fixation position to the release position and the detachment position will be referred to as a releasing direction. The operation portion 400 rotates in the releasing direction from the release position and thus moves to the detachment position. The operation portion 400 rotates in the locking direction from the release position and thus moves to the fixation position.

In a state in which the catheter unit 100 is attached to the base unit 200 and the operation portion 400 is at the detachment position, the coupling portion 21c is in a released state, and the fixation of the driving wire W by the coupling portion 21c is cancelled.

When the coupling portion 21c is in the released state, the cam 21cc is positioned at a retracted position retracted from a pressing portion that will be described later. At this time, the fixation of the held portion Wa by the plate spring 21ch is cancelled. The force of the first portion 21chd1 and the second portion 21chd2 clamping the held portion Wa when the coupling portion 21c is in the released state is smaller than the force of the first portion 21chd1 and the second portion 21chd2 clamping the held portion Wa when the coupling portion 21c is in the locked state.

When the coupling portion 21c is in the released state, in the case where the catheter unit is moved in the detachment direction Dd with respect to the base unit 200, the held portion Wa can be pulled out from between the first portion 21chd1 and the second portion 21chd2.

When the coupling portion 21c is in the released state, a state in which the force of the first portion 21chd1 and the second portion 21chd2 clamping the held portion Wa is not generated (state in which the magnitude thereof is zero) is preferable. When the coupling portion 21c is in the released state, it is preferable that there is a gap between the held portion Wa and at least one of the first portion 21chd1 and the second portion 21chd2.

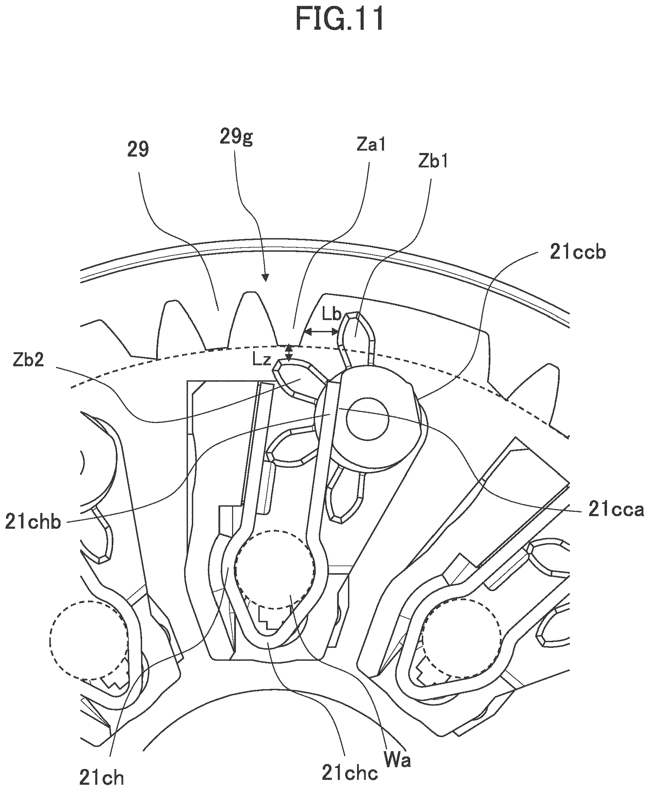

FIG. 11 is a diagram illustrating a state of the internal gear 29 and the coupling portion 21c when the operation portion 400 is rotated in the locking direction from the detachment position. FIG. 11 is a diagram illustrating a state of the internal gear 29 and the coupling portion 21c in a state in which the operation portion 400 is at the release position.

When the operation portion 400 is rotated in the locking direction in a state in which the operation portion 400 is at the detachment position (FIG. 10), the internal gear 29 rotates in the clockwise direction. Further, the operation portion 400 is positioned at the release position.

To be noted, even in the case where the operation portion 400 is rotated, since the key shaft 15 is engaged with the key receiving portion 22, rotation of the entirety (excluding the operation portion 400) of the catheter unit 100 with respect to the base unit 200 is restricted. That is, in a state in which the entirety (excluding the operation portion 400) of the catheter unit 100 and the base unit 200 are stopped, the operation portion 400 is rotatable with respect to these.

As a result of the internal gear 29 rotating in the clockwise direction, the clearance between the tooth Za1 of the internal gear 29 and the tooth Zb1 of the gear portion 21eg decreases from the clearance La to a clearance Lb.

A tooth Zb2 of the gear portion 21cg is disposed at a position with a clearance Lz from a tooth tip circle (dot line) of the tooth portion 29g of the internal gear 29. Therefore, the internal gear 29 can rotate without interfering with the tooth Zb2. Meanwhile, the coupling portion 21c is maintained at the same state (released state) as the state illustrated in FIG. 10.

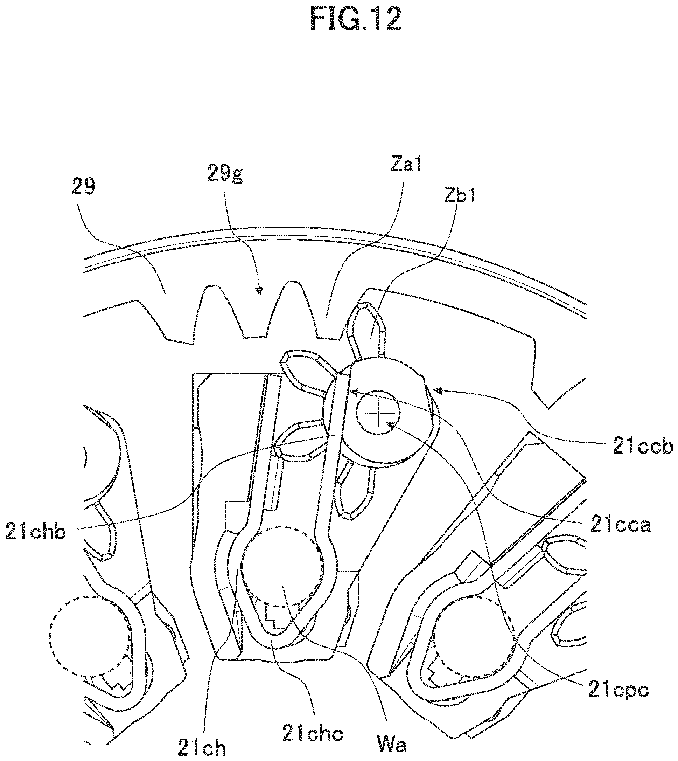

When the operation portion 400 is rotated in the locking direction from the state illustrated in FIG. 11, the internal gear 29 further rotates in the clockwise direction. FIG. 12 illustrates the state of the internal gear 29 and the coupling portion 21c.

FIG. 12 is a diagram illustrating the state of the internal gear 29 and the coupling portion 21c when the operation portion 400 is rotated in the locking direction from the release position. As illustrated in FIG. 12, when the operation portion 400 is rotated in the locking direction from the release position, the tooth Za1 of the internal gear 29 and the tooth Zb1 of the gear portion 21cg come into contact with each other. Meanwhile, the coupling portion 21c is in the same state as the state illustrated in FIGS. 10 and 11, and is maintained in the released state.

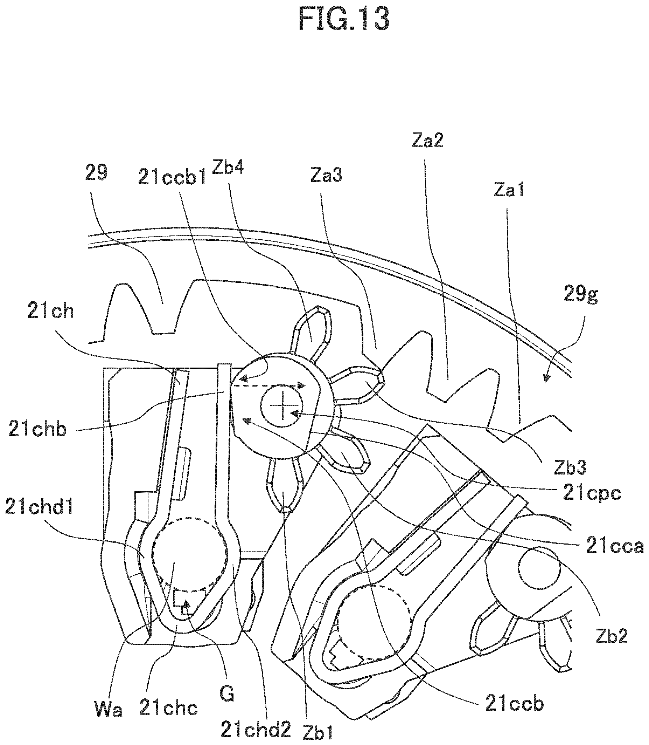

FIG. 13 is a diagram illustrating a state in which the pressing member 21cp is rotated as a result of the operation portion 400 rotating in the locking direction. As illustrated in FIG. 13, when the operation portion 400 is rotated in the locking direction from the state of FIG. 12, the internal gear 29 further rotates in the clockwise direction.

As a result of the internal gear 29 moving from the state of FIG. 12 to the state of FIG. 13, the internal gear 29 rotates the gear portion 21cg in the clockwise direction. When the gear portion 21cg rotates, the holding surface 21cca moves away from the pressed portion 21chb, and the pressing surface 21ccb moves closer to the pressed portion 21chb. Then, the clamping of the held portion Wa by the first portion 21chd1 and the second portion 21chd2 is started.

Then, a tooth Za3 of the internal gear 29 moves to a position away from a tooth Zb3 of the gear portion 21cg while the pressed portion 21chb is pressed by a corner portion 21ccb1 provided at an end portion of the pressing surface 21ccb. At this time, the held portion Wa is clamped between the first portion 21chd1 and the second portion 21chd2.

When the tooth Za3 of the internal gear 29 is separated from the tooth Zb3 of the gear portion 21cg, the transmission of the driving force from the internal gear 29 to the gear portion 21cg is finished. At this time, the cam 21cc is in a state in which the corner portion 21ccb1 receives a reaction force from the plate spring 21ch.

In the rotational radius direction of the pressing member 21cp, the reaction force of the plate spring 21ch acting on the corner portion 21ccb1 acts at a position away from the rotational center 21cpc of the pressing member 21cp, and the pressing member 21cp rotates in the clockwise direction. At this time, the pressing member 21cp rotates in the same direction as a direction in which the pressing member 21cp is rotated by the internal gear 29 that rotates in the clockwise direction.

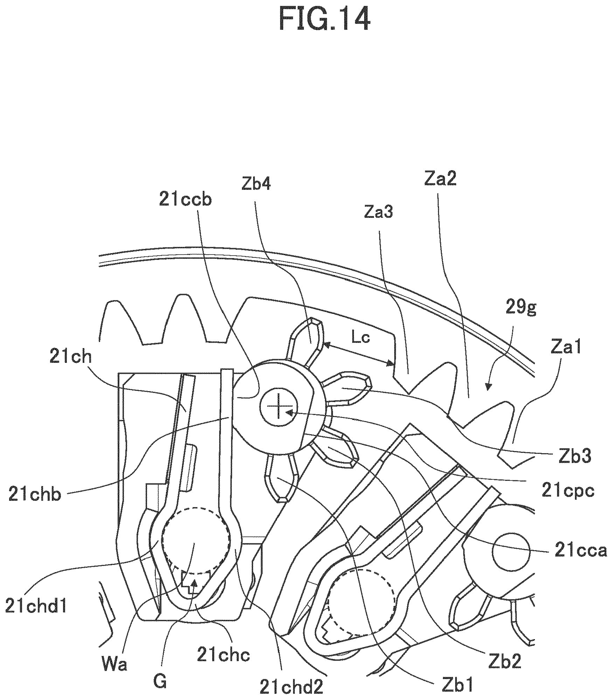

FIG. 14 is a diagram illustrating a state of the internal gear 29 and the coupling portion 21c in a state in which the operation portion 400 is at the fixation position. As illustrated in FIG. 14, the pressing member 21cp further rotates from the state illustrated in FIG. 13 by receiving the reaction force of the plate spring 21ch.

As illustrated in FIG. 14, the pressing member 21cp stops in a state in which the pressing surface 21ccb of the cam 21cc and the pressed portion 21chb of the plate spring 21ch are in surface contact. That is, the pressing surface 21ccb and the surface of the pressed portion 21chb are on the same plane. At this time, the coupling portion 21c is in the locked state. When the coupling portion 21c is in the locked state, the cam 21cc of the pressing member 21cp is positioned at the pressing position, and the pressing surface 21ccb presses the pressed portion 21chb.

When the coupling portion 21c is in the locked state, the held portion Wa is clamped between the first portion 21chd1 and the second portion 21chd2. That is, the cam 21cc presses the plate spring 21ch, and the held portion Wa is clamped by the plate spring 21ch. As a result, the held portion Wa is fixed by the plate spring 21ch.

In the present embodiment, the first portion 21chd1 and the second portion 21chd2 of the plate spring 21ch press the held portion Wa at positions away from each other. Further, a bent portion 21chc interconnecting the first portion 21chd1 and the second portion 21chd2 is disposed between the first portion 21chd1 and the second portion 21chd2. The bent portion 21chc is provided with a gap G from the held portion Wa. As a result of this, the held portion Wa can be stably fixed by the first portion 21chd1 and the second portion 21chd2.

As the material of the plate spring 21ch, resin or metal can be used, but metal is preferably used.

When the coupling portion 21c is in the locked state, pulling the held portion Wa out from between the first portion 21chd1 and the second portion 21chd2 is restricted.

To be noted, the tooth Za3 of the internal gear 29 and a tooth Zb4 of the gear portion 21cg are stopped at positions where there is a clearance Lc therebetween. In addition, the surface of the tooth tip of the tooth Za3 is inclined so as to be farther from the rotation axis toward the downstream side in the releasing direction with respect to a cylindrical surface centered on the rotation axis of the internal gear 29 and circumscribing the surface of the tooth tips of the other teeth Za1 and Za2. As a result of this, in the case where the internal gear 29 is rotated in the releasing direction from the state of FIG. 14 as will be described later, the tooth Za3 of the internal gear 29 can reach the next tooth Zb4 without colliding with the tooth Zb3 of the gear portion 21cg.

When cancelling the fixation between the driving wire W and the coupling portion 21c, the operation portion 400 positioned at the fixation position is rotated in the releasing direction. At this time, the internal gear 29 rotates in the counterclockwise direction from the state illustrated in FIG. 14. When the internal gear 29 rotates in the counterclockwise direction, the tooth Za3 of the internal gear 29 abuts the tooth Zb4 of the gear portion 21cg, and the pressing member 21cp is rotated in the counterclockwise direction.

By rotating the internal gear 29 in a counterclockwise direction, the fixation of the driving wire W by the coupling portion 21c is cancelled. The operation of the internal gear 29 and the pressing member 21cp at this time is an operation reversed from the operation described above. That is, the fixation of the driving wire W by the coupling portion 21c is cancelled by the operation reversed from the operation of fixing the driving wire W by the coupling portion 21c described above.

The operation described above is performed by each of the first to ninth coupling portions (21c11 to 21c33). That is, in the course of the operation portion 400 moving from the detachment position to the fixation position, the first to ninth coupling portions (21c11 to 21c33) are switched from the released state to the locked state by the movement (rotation) of the operation portion 400. In the course of the operation portion 400 moving from the fixation position to the detachment position, the first to ninth coupling portions (21c11 to 21c33) are switched from the locked state to the released state by the movement (rotation) of the operation portion 400. That is, the user can switch the released state and the locked state of the plurality of coupling portions by operating the one operation portion 400.

That is, there is no need to provide an operation portion for switching between the released state and the locked state for each of the plurality of coupling portions, and the user does not need to operate those. Therefore, the user can easily attach and detach the catheter unit 100 to and from the base unit 200. Further, the medical apparatus 1 can be simplified.

A state in which the first to ninth driving wires (W11 to W33) are respectively fixed by the first to ninth coupling portions (21c11 to 21c33) will be referred to as a first state. A state in which the fixation of the first to ninth driving wires (W11 to W33) respectively by the first to ninth coupling portions (21c11 to 21c33) is cancelled will be referred to as a second state.

The first state and the second state are switched in an interlocked manner with the movement of the operation portion 400. That is, the first state and the second state are switched in an interlocked manner with movement of the operation portion 400 between the detachment position and the fixation position.

The internal gear 29 is configured to move in an interlocked manner with the operation portion 400. In the present embodiment, the joint 28 functions as a transmission member for causing the operation portion 400 and the internal gear 29 to move in an interlocked manner. The internal gear 29 and the joint 28 have a function as an interlocked portion that moves in an interlocked manner with the operation portion 400 such that the first state and the second state are switched in an interlocked manner with the movement of the operation portion 400.

Specifically, the internal gear 29 and the joint 28 move part (pressed portion 21chb) of the plate spring 21ch with respect to the held portion Wa in an interlocked manner with the movement of the operation portion 400 in a state in which the catheter unit 100 is attached to the base unit 200. As a result of the movement of the pressed portion 21chb, the locked state and the released state of the coupling portion 21c are switched.

To be noted, the internal gear 29 may be configured to be directly moved by the operation portion 400. In this case, the internal gear 29 has a function as an interlocked portion.

### <Movement of Operation Portion>

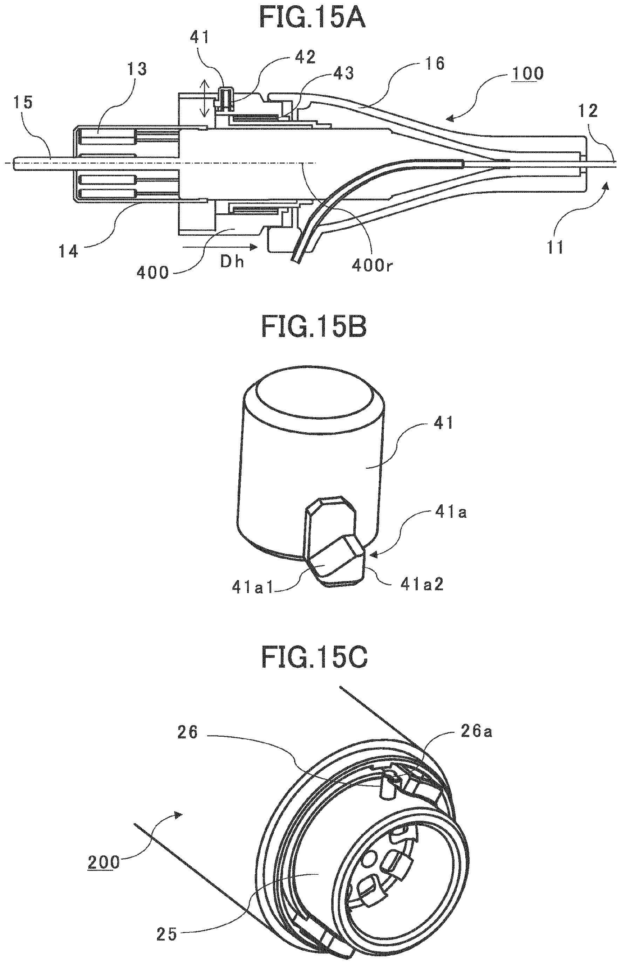

The movement of the operation portion 400 will be described with reference to FIGS. 15Ato 15C, 16Ato 16C, and 17Ato 17C.

In the present embodiment, the operation portion 400 is configured to be movable between the detachment position, the release position, and the fixation position in a state in which the catheter unit 100 is attached to the base unit 200. The release position is located between the detachment position and the fixation position.

In the present embodiment, the first state and the second state are switched in an interlocked manner with the movement of the operation portion 400 of the operation portion 400 between the release position and the fixation position.

In the present embodiment, the operation portion 400 can move between the detachment position and the fixation position by moving in a direction different from the attachment/detachment direction DE. The operation portion 400 moves between the detachment position and the fixation position by moving in a direction intersecting with (preferably orthogonal to) the attachment/detachment direction DE. In the present embodiment, the operation portion 400 moves between the detachment position and the fixation position by rotating about the rotation shaft 400r extending in the attachment/detachment direction DE. Therefore, the operability when the user operates the operation portion 400 is good.

FIGS. 15A to 15C are explanatory diagrams of the catheter unit 100 and the base unit 200. FIG. 15A is a section view of the catheter unit 100. FIG. 15B is a perspective view of a button 41. FIG. 15C is a perspective view of the base unit 200.

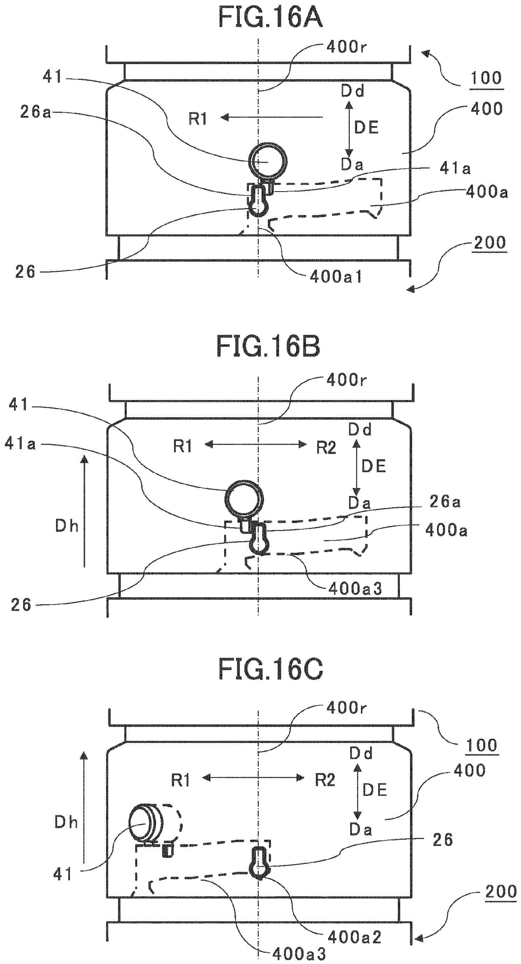

FIGS. 16A to 16C are diagrams for describing the operation of the operation portion 400. FIG. 16A is a diagram illustrating a state in which the operation portion 400 is at the detachment position. FIG. 16B is a diagram illustrating a state in which the operation portion 400 is at the release position. FIG. 16C is a diagram illustrating a state in which the operation portion 400 is at the fixation position.

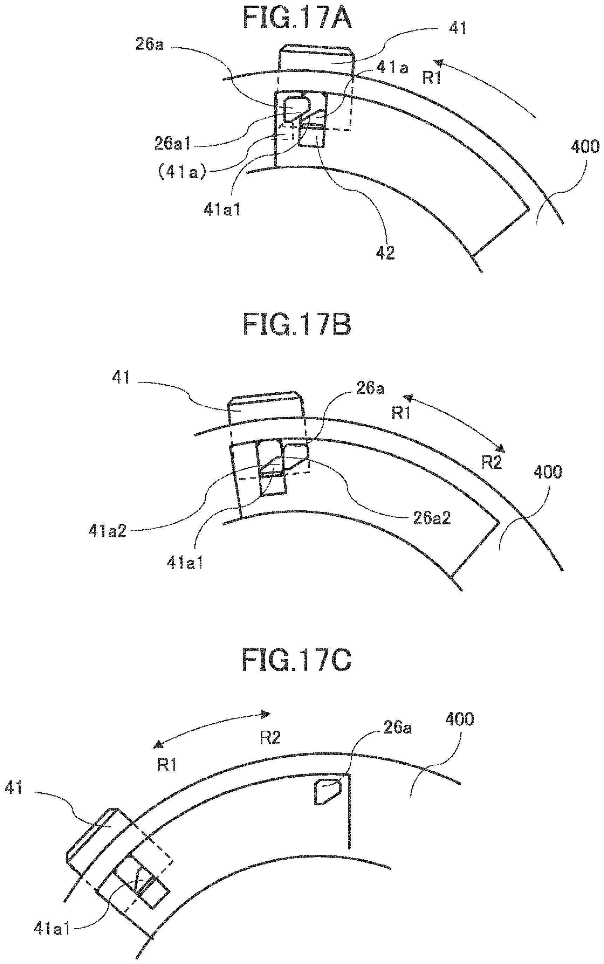

FIGS. 17A to 17C are section views for describing the operation of the operation portion 400. FIG. 17A is a section view illustrating a state in which the operation portion 400 is at the detachment position. FIG. 17B is a section view illustrating a state in which the operation portion 400 is at the release position. FIG. 17C is a section view illustrating a state in which the operation portion 400 is at the fixation position.

When the operation portion 400 is at the fixation position, the coupling portion 21c is in the locked state, and the held portion Wa of the driving wire W is fixed to the corresponding coupling portion 21c (see FIG. 14).

When the operation portion 400 is at the release position, the coupling portion 21c is in the released state, and the lock between the held portion Wa of the driving wire W and the coupling portion 21c is cancelled (see FIG. 11). In this state, the connection between the driving wire W and the wire driving portion 300 is cut off. Therefore, when the catheter 11 receives an external force, the bending portion 12 can be freely bent without receiving a resistance from the wire driving portion 300.

When the operation portion 400 is at the detachment position, detachment of the catheter unit 100 from the base unit 200 is allowed. In addition, the catheter unit 100 can be attached to the base unit 200 in a state in which the operation portion 400 is at the detachment position. When the operation portion 400 is at the detachment position, the coupling portion 21c is in the released state, and the lock between the held portion Wa of the driving wire W and the coupling portion 21c is cancelled (see FIG. 10).

As illustrated in FIG. 15A, the catheter unit 100 includes an operation portion urging spring 43 that urges the operation portion 400, a button 41 serving as a moving member, and a button spring 42 that urges the button 41.

In the present embodiment, the operation portion urging spring 43 is a compression spring. The operation portion 400 is urged by the operation portion urging spring 43 in a direction Dh of approaching the near end cover 16.

In the present embodiment, the button 41 and the button spring 42 are provided in the operation portion 400. When the operation portion 400 moves to the detachment position, the release position, and the fixation position, the button 41 and the button spring 42 move together with the operation portion 400.

The button 41 is configured to be movable with respect to the operation portion 400 in a direction intersecting with the direction of the rotation shaft 400r of the operation portion 400. The button 41 is urged outward (in a direction away from the rotation shaft 400r) with respect to the catheter unit 100 by the button spring 42.

As will be described later, the movement of the operation portion 400 from the release position to the detachment position is restricted by the button 41. In addition, by moving the button 41 with respect to the operation portion 400, movement of the operation portion 400 from the release position to the detachment position is allowed.

The button 41 includes a button protrusion (restricted portion) 41a. The button protrusion 41a includes an inclined surface 41a1 and a restricted surface 41a2.

The base unit 200 includes the base frame 25. The base frame 25 is provided with the locking shaft 26. The locking shaft 26 includes the locking protrusion (restricting portion) 26a.

In the present embodiment, a plurality of (two in the present embodiment) locking shafts 26 are provided. Each locking shaft 26 may include the locking protrusion 26a, or part of the locking shafts 26 may include the locking protrusion 26a.

Meanwhile, as illustrated in FIGS. 9, 16A, 16B, and 16C, locking grooves 400a that engage with the locking shafts 26 are provided on the inner side of the operation portion 400. The locking grooves 400a extend in a direction different from the attachment/detachment direction DE, and extend in the rotation direction of the operation portion 400 in the present embodiment. It can be also said that the locking grooves 400a extend in a direction intersecting with (orthogonal to) the attachment/detachment direction DE.

The locking grooves 400a are respectively provided for the plurality of locking shafts 26 in the case where a plurality of locking shafts 26 are provided.

As illustrated in FIG. 16A, when the catheter unit 100 is attached to the base unit 200, a locking shaft 26 engages with a locking groove 400a via an entrance 400a1 of the locking groove 400a.

At this time, the operation portion 400 is positioned at the detachment position, and the coupling portion 21c is in the released state (see FIG. 10). Therefore, the fixation of the first to ninth driving wires (W11 to W33) respectively by the first to ninth coupling portions (21c11 to 21c33) is cancelled. In addition, as illustrated in FIG. 17A, the button protrusion 41a and the locking protrusion 26a face each other.

When the operation portion 400 is rotated in the locking direction R1 in a state in which the operation portion 400 is at the detachment position, an inclined surface 41a1 of the button protrusion 41a abuts an inclined surface 26a1 of the locking protrusion 26a. The button 41 moves inward (in a direction to approach the rotation shaft 400r) with respect to the operation portion 400 against the urging force of the button spring 42. Then, the button protrusion 41a climbs over the locking protrusion 26a, and the operation portion 400 moves to the release position (see FIG. 17B).

At this time, the coupling portion 21c is in the released state (see FIG. 11). Therefore, the fixation of the first to ninth driving wires (W11 to W33) respectively by the first to ninth coupling portions (21c11 to 21c33) is cancelled.

In the present embodiment, the movement of the operation portion 400 from the detachment position to the release position is allowed without operating the button 41. That is, the user does not need to operate the button 41 when moving the operation portion 400 from the detachment position to the release position.

When the operation portion 400 is rotated in the locking direction R1 in a state in which the operation portion 400 is positioned at the release position, the operation portion 400 moves to the fixation position. In a state in which the operation portion 400 is at the fixation position, a positioning portion 400a2 of the locking groove 400a is positioned at a position corresponding to the locking shaft 26. The operation portion 400 is urged in the direction Dh to approach the near end cover 16 by the operation portion urging spring 43. As a result, the positioning portion 400a2 engages with the locking shaft 26.

In the course of the operation portion 400 moving from the release position to the fixation position, the held portion Wa of the driving wire W is fixed to the coupling portion 21c as described above.

In a state in which the operation portion is positioned at the fixation position, the coupling portion 21c is in the locked state (see FIG. 14). Therefore, the first to ninth driving wires (W11 to W33) are respectively fixed to the first to ninth coupling portions (21c11 to 21c33). In this state, the driving force from the wire driving portion 300 can be transmitted to the bending driving portion 13. That is, the driving force respectively from the first to ninth drive sources (M11 to M33) can be respectively transmitted to the first to ninth driving wires (W11 to W33) via the first to ninth coupling portions (21c11 to 21c33).

When the operation portion 400 is at the release position, a wall 400a3 defining the locking groove 400a is positioned upstream of the locking shaft 26 in the detachment direction Dd of the catheter unit 100. When the operation portion 400 is at the fixation position, the positioning portion 400a2 is positioned upstream of the locking shaft 26 in the detachment direction Dd. As a result, when the operation portion 400 is at the release position or the fixation position, detachment of the catheter unit 100 from the base unit 200 is restricted. In contrast, when the operation portion 400 is at the detachment position, the entrance 400a1 of the locking groove 400a is positioned upstream of the locking shaft 26 in the detachment direction Dd. As a result, detachment of the catheter unit 100 from the base unit 200 is allowed.

When the operation portion 400 is rotated in a releasing direction R2 in a state in which the operation portion 400 is at the fixation position, the operation portion 400 is positioned at the release position. In the course of the operation portion 400 moving from the fixation position to the release position, the held portion Wa of the driving wire W is released from the coupling portion 21c as described above.

In a state in which the operation portion 400 is positioned at the release position, the restricted surface 41a2 of the button protrusion 41a abuts the restricting surface 26a2 of the locking protrusion 26a (see FIG. 17B). In this state, rotation of the operation portion 400 in the releasing direction R2 is restricted. In addition, detachment of the catheter unit 100 from the base unit 200 is restricted.

As a result of the user pushing the button 41 inward with respect to the operation portion 400 in a state in which the operation portion 400 is positioned at the release position, the restricted surface 41a2 is separated from the restricting surface 26a2, and the button protrusion 41a climbs over the locking protrusion 26a. As a result, rotation of the operation portion 400 in the releasing direction R2 is allowed, and thus the operation portion 400 can move from the release position to the detachment position.

When the operation portion 400 is positioned at the detachment position, the coupling portion 21c takes the released state. Therefore, when detaching and attaching the catheter unit 100 from and to the base unit 200, the load (for example, resistance received the coupling portion 21c) acting on the driving wire W can be reduced. Therefore, the user can easily attach and detach the catheter unit 100.

When the operation portion 400 is positioned at the release position, detachment of the catheter unit 100 from the base unit 200 is restricted, and the coupling portion 21c takes the released state. As described above, when the coupling portion 21c is in the released state, the connection between the driving wire W and the wire driving portion 300 is cut off, and the bending portion 12 can be freely bent without receiving the resistance from the wire driving portion 300.

The user can stop the driving of the catheter 11 by the wire driving portion 300 by positioning the operation portion 400 at the release position in a state in which the catheter 11 is inserted in the object. Further, since detachment of the catheter unit 100 from the base unit 200 is restricted, the user can pull out the catheter 11 from the inside of the object by holding the base unit 200.

In addition, in the configuration of the present embodiment, movement of the operation portion 400 from the release position to the detachment position is restricted in the case where the button 41 is not operated. Therefore, when the user moves the operation portion 400 from the fixation position to the release position, erroneously moving the operation portion 400 to the detachment position can be suppressed.

To be noted, in the present embodiment, one locking protrusion 26a and one button 41 are provided. To be noted, the medical apparatus 1 may include a plurality of each of the locking protrusion 26a and the button 41.

### <Connecting Portion>

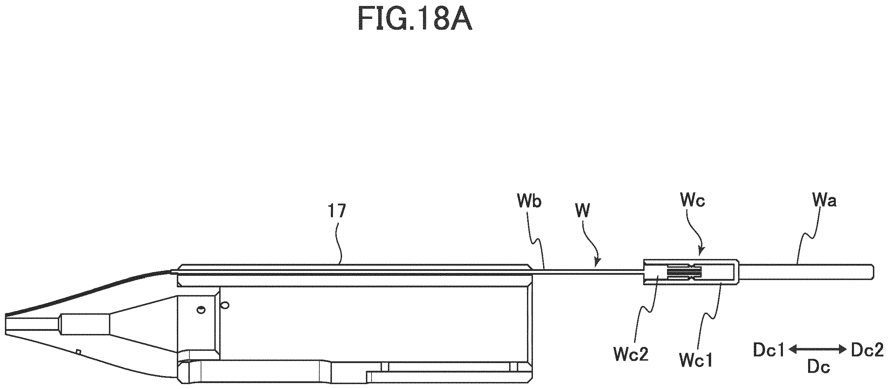

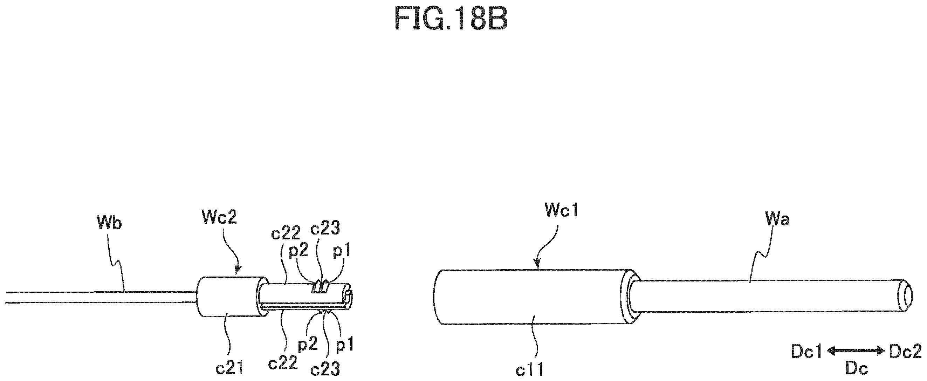

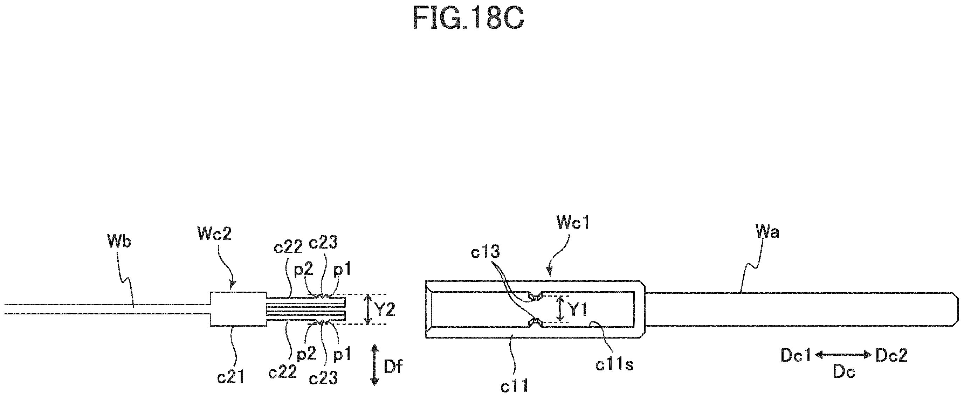

Next, the connecting portion Wc of the present embodiment will be described with reference to FIGS. 18A to 18C, 19A to 19C, and 20A to 20C. FIG. 18Ais a schematic view of the driving wire W according to the present embodiment including the connecting portion Wc. FIG. 18B is a perspective view of a holder Wc1 and a rod Wc2 constituting the connecting portion Wc. FIG. 18C is a section view of the holder Wc1 and the rod Wc2 constituting the connecting portion Wc. FIG. 18A illustrates a state (connected state) in which the rod Wc2 is held by the holder Wc1, and FIGS. 18B and 18C illustrate a state (disconnected state) in which the rod Wc2 is not held by the holder Wc1.

In the description below, the connecting portion Wc provided on the driving wire W that is arbitrary one of the first to ninth driving wires (W 1 1 to W33) included in the catheter unit 100 of the present embodiment will be described. In the configuration example of the present embodiment, the connecting portion Wc having substantially the same configuration as that described below is disposed on each of the nine driving wires (W11 to W33).

As illustrated in FIG. 18A, the connecting portion Wc includes the holder Wc1 serving as a first member (engaging member, holding member) connected to the drive source M, and the rod Wc2 serving as a second member (engaged member, held member) connected to the wire body Wb of the driving wire W. Here, "connected to the drive source M" and "connected to the wire body Wb" indicate being provided on the upstream side (drive source side) or on the downstream side (wire body side) in a drive transmission path from the drive source M to the wire body Wb, and do not require direct physical contact.

In the present embodiment, the holder Wc1 is a member integrally formed with the held portion Wa of the driving wire W. Therefore, the holder Wc1 is connected to the drive source M via the held portion Wa and the coupling portion 21c (FIGS. 6A to 6C. To be noted, the holder Wc1 and the held portion Wa may be separate bodies. Meanwhile, the rod Wc2 is fixed to the near end of the wire body Wb by an arbitrary fixing method such as compression bonding (swaging) or gluing.

In the connected state in which the rod Wc2 is held by the holder Wc1 as illustrated in FIG. 18A, the driving force of the drive source M is transmitted to the wire body Wb via the connecting portion Wc. As described above, the driving wire W is driven in both the Dc1 direction that is a direction to press the near end of the wire body Wb to the far end side and the DC2 direction that is a direction to pull the near end of the wire body Wb to the opposite side to the Dc1 direction by switching the rotation direction of the drive source M. To be noted, the extending direction of the wire body Wb in the connecting portion Wc is a Dc direction substantially the same as the axial direction of the output shaft Ma (FIG. 6A) of the drive source. The Dc1 direction is one side of the Dc direction, and the Dc2 direction is the other side of the Dc direction.

The holder Wc1 includes a cylindrical portion c11 having an approximate bottomed cylinder shape extending in the Dc direction and opening in the Dc1 direction as illustrated in FIGS. 18B and 18C. The cylindrical portion c11 is disposed to be coaxial with and aligned with the held portion Wa in the Dc direction, and the held portion Wa extends in the Dc2 direction from the bottom portion of the cylinder portion c11. The cylindrical portion c11 is an example of a tubular portion extending in the extending direction of the wire body Wb and defining a space in which the second member can be inserted, and may be formed in, for example, a prismatic tube shape. The cylindrical portion c11 has a higher flexural rigidity in a Df direction that is a main deformation direction of a protruding piece c22 than the protruding piece c22 of the rod Wc2 that will be described later.

In addition, the holder Wc1 includes a protrusion portion c13 formed on the inside of the cylindrical portion c11. The protrusion portion c13 is provided to protrude from the inner surface of the cylindrical portion c11 toward the axial center. That is, the protrusion portion c13 is an example of a protrusion portion protruding in a direction intersecting with the extending direction (Dc direction) of the wire body Wb in the connecting portion Wc.

The protrusion portion c13 is disposed at a position corresponding to a recess portion c23 of the rod Wc2 that will be described later, in a state as viewed in the Dc direction. In the present embodiment, the protrusion portion c13 is provided at two positions away from each other by 180° in correspondence with the recess portion c23 provided at two positions away from each other by 180° in the circumferential direction of the cylindrical portion e11. To be noted, it suffices as long as the protrusion portion c13 is provided at a position engageable with the recess portion c23, and for example, an annular protrusion portion c13 may be formed on the entire circumference of the inner surface of the cylindrical portion c11.

The rod Wc2 includes a base portion c21 having an approximate columnar shape extending in the Dc2 direction from the near end of the wire body Wb, and two protruding pieces c22 each protruding in the Dc2 direction from the base portion c21 as illustrated in FIGS. 18B and 18C. The protruding piece c22 has a shape obtained by dividing a cylindrical shape having a smaller outer diameter than the base portion c21 into two by a plane extending in the Dc direction. That is, the two protruding pieces c22 are two arcs positioned at positions opposing each other on the same circumference as viewed from the Dc2 direction.

Therefore, in the case where the protruding pieces c22 receive an external force, the protruding pieces c22 mainly deform in the Df direction in which the two protruding pieces c22 oppose each other through the center of the same circumference. That is, the protruding pieces c22 function as deforming elements capable of deforming so as to allow the protrusion portions c13 to be disengaged from the recess portions c23. The Df direction is a direction orthogonal to the extending direction (Dc direction) of the wire body Wb in the connecting portion Wc. To be noted, the shape of the protruding pieces c22 is not limited to that described above as long as the protruding pieces c22 have appropriate stiffness in the Df direction and is capable of elastic deformation.

In addition, the rod Wc2 has a recess portion c23 on the outside of each protruding piece c22. The recess portion c23 is formed between protrusions p1 and p2 protruding outward from the outer surface of the protruding piece c22. The protrusions p1 and p2 are arranged in the Dc direction.

The rod Wc2 is inserted in the Dc2 direction into a space c11s on the inside of the cylindrical portion c11 of the holder Wc1, and is attached to the holder Wc1 by being pushed in until the protrusion portions c13 of the holder Wc1 are fitted in the recess portions c23 of the rod Wc2. In the present embodiment, the entirety of the rod Wc2, that is, the part from the base portion c21 to the protruding pieces c22 is an insertion portion to be inserted in the cylindrical portion c11. Therefore, the recess portions c23 of the protruding pieces c22 are provided on the outer surface of the insertion portion to be inserted in the cylindrical portion c11.

In the description below, a state in which the rod Wc2 is held by the holder Wc1 such that the driving force in the Dc1 direction or the Dc2 direction transmitted from the drive source M can be transmitted to the wire body Wb will be referred to as a connected state (engaged state, attached state) of the connecting portion Wc. In addition, a state in which the protrusion portions c13 are disengaged from the recess portions c23 and the connection between the drive source M and the wire body Wb is cut off will be referred to as a disconnected state (non-connected state, disengaged state, detached state) of the connecting portion Wc.

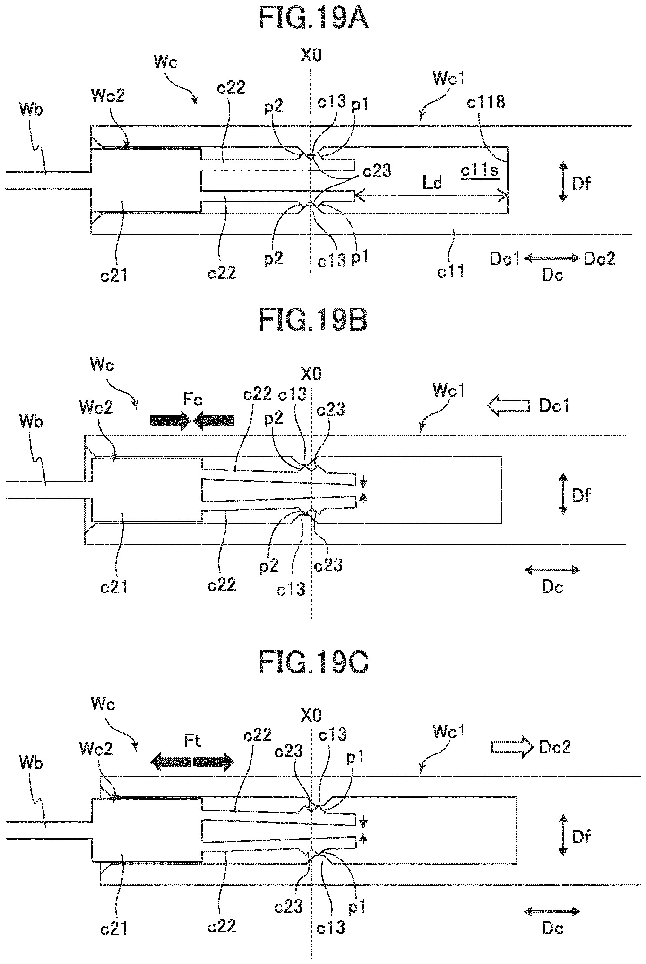

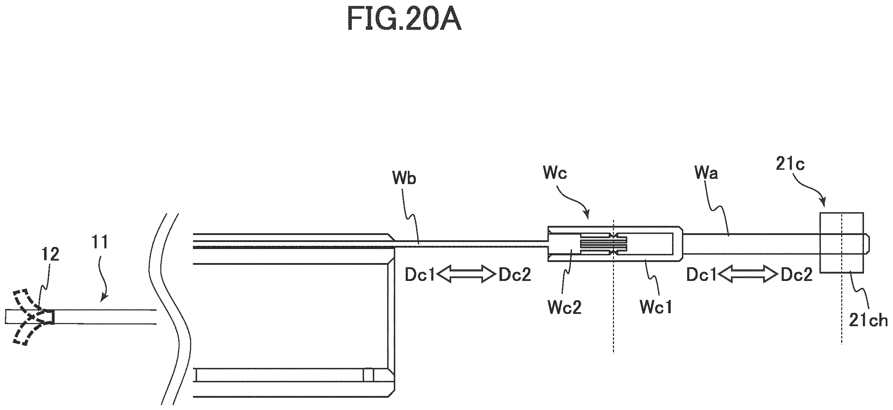

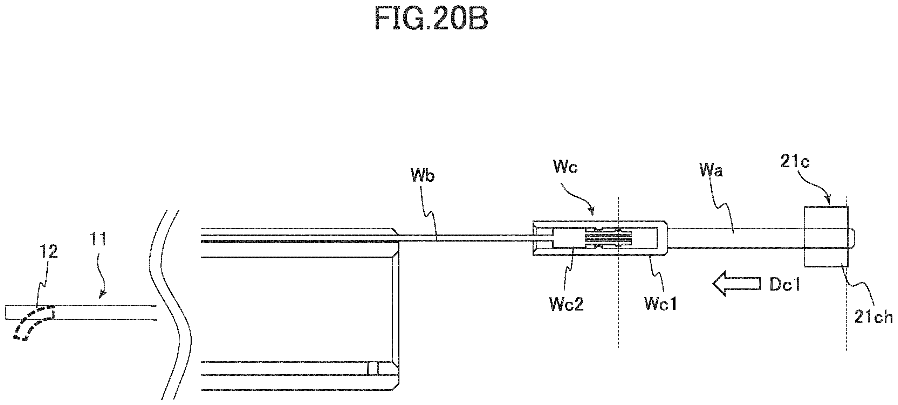

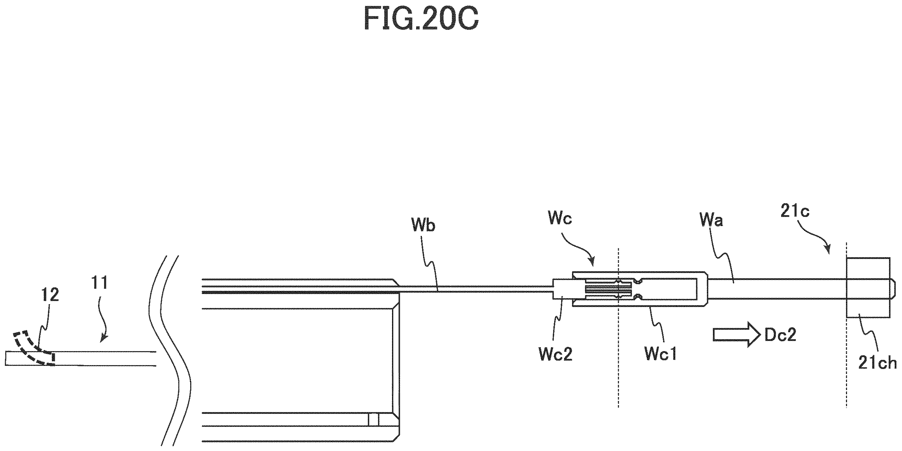

Next, the behavior of the connecting portion Wc in the connected state and at the time of overload will be described with reference to FIGS. 19A to 19C and 20A to 20C. FIG. 19Ais a schematic diagram illustrating a cross-section of the connecting portion Wc in the connected state. FIG. 19B is a schematic diagram illustrating the state of the connecting portion Wc in the case where an overload in the direction (Dc1 direction) of to press the wire body Wb is applied. FIG. 19C is a schematic diagram illustrating the state of the connecting portion Wc in the case where an overload in a direction (Dc2 direction) to pull the wire body Wb is applied. FIG. 20A is a schematic diagram for describing the behavior of the catheter 11 in the case where the connecting portion Wc is in the connected state. FIG. 20B is a schematic diagram for describing the behavior of the catheter 11 in the case where an overload in the direction (Dc1 direction) to press the wire body Wb is applied. FIG. 20C is a schematic diagram for describing the behavior of the catheter 11 in the case where an overload in the direction (Dc2 direction) of to pull the wire body Wb is applied.

To be noted, as described above, in the present embodiment, the catheter 11 is operated by pressing or pulling the wire body Wb. In contrast, a configuration in which the catheter 11 is operated by just pulling the wire body Wb without pressing can be also considered. However, in such a configuration, the number of actuators and driving wires increases, which increases the size and cost of the medical apparatus 1. For the reason described above, a configuration in which the catheter 11 is operated by pressing or pulling the wire body Wb is employed.

### (Connected State)

As illustrated in FIG. 19A, in the connected state of the connecting portion Wc, the protrusion portions c13 of the holder Wc1 are fitted in the recess portions c23 of the rod Wc2. Specifically, the protrusion portions c13 are held between the protrusions p1 and p2 in the Dc direction. Here, as illustrated in FIG. 18C, the distance in the Df direction between apices of the protrusions p1 and the distance in the Df direction between apices of the protrusions p2 in a state in which the rod Wc2 is not attached to the holder Wc1 are substantially the same at a distance Y2. This distance Y2 is larger than a distance Y1 in the Df direction between the protrusion portions c13 of the holder Wc1 in a state in which the rod Wc2 is not attached to the holder Wc1. Therefore, in the connected state illustrated in FIG. 19A, the protrusion portions c13 of the holder Wc1 and part of the rod Wc2 (protrusions p1 and p2) are in such a positional relationship as to interfere with each other in the Dc direction. In other words, at least part of the protrusion portions provided on one of the first member and the second member overlap recess portions provided on the other of the first member and the second member as viewed in the extending direction (Dc direction) of the wire body in the connected state.

According to such a configuration, in the case where the connecting portion Wc is in the connected state, the holder Wc1 and the rod Wc2 integrally move in the Dc1 direction and the Dc2 direction. That is, in the case where the connecting portion Wc is in the connected state, as illustrated in FIG. 20A, as a result of the plate spring 21ch of the coupling portion 21c moving the held portion Wa by the driving force from the drive source M, the driving force is transmitted to the wire body Wb via the connecting portion Wc. At this time, when a driving force in a direction (Dc1 direction) to press the wire body Wb is transmitted, the bending portion 12 of the catheter 11 bends such that the side on which the wire body Wb is inserted is the outer side of the bending. In addition, when a driving force in a direction (Dc2 direction) to pull the wire body Wb is transmitted, the bending portion 12 of the catheter 11 bends such that the side on which the wire body Wb is inserted is the outer side of the bending.

In the connected state of FIG. 19A, the protrusion portions c13 of the holder Wc1 are configured to fit in the recess portions c23 of the rod Wc2 in a state in which the backlash is reduced. In the present embodiment, the protruding pieces c22 on which the recess portions c23 are provided are formed from an elastic member, and the protrusion portions c13 are pressed against the protrusions p1 and p2 on the two sides thereof by the elastic force of the protruding pieces c22 as a result of the protruding pieces c22 being slightly elastically deformed in the Df direction in the connected state.

That is, in the present embodiment, the holder Wc1 serving as a first member and the rod Wc2 serving as a second member are engaged with each other by a mechanism (snap fit mechanism) using a deformation element (elastic element) formed from an elastic material. In other words, the connecting portion of the present embodiment is maintained in a state in which the protrusion portions are fitted in the recess portions by the elastic force of the elastic element in the case where a force equal to or smaller than a threshold value (tensile force equal to or smaller than a first threshold value described below or compressive force equal to or smaller than a second threshold value) acts between the first member and the second member. As a result of employing a snap fit mechanism for the connecting portion Wc, the connecting portion Wc can be assembled by an easy work of inserting the rod Wc2 in the holder Wc1.

Specifically, in a state of FIG. 19A in which the rod Wc2 is attached to the holder Wc1, the protruding pieces c22 are slightly more bent than in a state before attachment (FIG. 18C) as a result of the protrusions p1 and p2 of the rod Wc2 abutting the protrusion portions c13 of the holder Wc1. That is, a distance Y2' between the apices of the protrusions p1 in a state in which the rod Wc2 is attached to the holder Wc1 is slightly smaller than the distance Y2 between the apices of the protrusions p1 in a state in which the rod Wc2 is not attached to the holder Wc1.

As described above, the responsiveness of the bending portion 12 of the catheter 11 to the driving of the drive source M can be improved by the configuration of engaging the holder Wc1 and the rod Wc2 with each other in the state in which the backlash is reduced.

### (When Overload Is Generated in Pressing Direction)

Next, a case where an excessive force in a direction (Dc1 direction) to press the wire body Wb acts on the connecting portion Wc will be described. As illustrated in FIG. 19B, when a driving force in a direction (Dc1 direction) to press the wire body Wb is transmitted from the drive source M to the holder Wc1, a compressive force Fc acts between the holder Wc1 and the rod Wc2.

In the case where this compressive force Fc does not exceed a preset threshold value (second threshold value), the compressive force Fc is received by the rod Wc2 via the contact portion between the protrusion portions c13 of the holder Wc1 and the protrusions p2 of the rod Wc2. That is, the connecting portion Wc of the present embodiment is configured to maintain the state in which the protrusion portions c13 are fitted in the recess portions c23 and transmit the driving force from the drive source M to the wire body Wb in the case where the compressive force Fc equal to or smaller than the second threshold value is applied.

However, in the case where a large compressive force Fc exceeding the preset threshold value (second threshold value) is applied, the protrusions p2 of the rod Wc2 are pressed by the protrusion portions c13 of the holder Wc1, and thus the protruding pieces c22 of the rod Wc2 elastically deform inward. As a result of this, the holder Wc1 and the rod Wc2 relatively move while the protrusion portions c13 climb over the protrusions p2, and the protrusion portions c13 are disengaged from the recess portions c23. In the state (disconnected state) in which the protrusion portions c13 are disengaged from the recess portions c23, the rod Wc2 does not move in the Dc1 direction even when the holder Wc1 moves in the Dc1 direction. That is, the connecting portion Wc of the present embodiment is configured such that in the case where the compressive force Fc exceeding the second threshold value is applied, the protrusion portions c13 are disengaged from the recess portions c23 and the transmission of the driving force from the drive source M to the wire body Wb is cut off (FIG. 20B).

As a result of the connecting portion Wc switching from the connected state to the disconnected state at the time of overload, for example, even in the case where the holder Wc1 is driven in the Dc1 direction by an excessively large driving force due to abnormal operation of the drive source M, the excessively large driving force is prevented from being transmitted to the wire body Wb. As a result of this, a situation in which the bending portion 12 of the catheter 11 is bent by an excessively strong force can be avoided.

In addition, as described above, the connecting portion Wc is switched from the connected state to the disconnected state in accordance with the magnitude of the compressive force Fc acting between the holder Wc1 and the rod Wc2. Therefore, the connection of the connecting portion Wc is cut off also in the case where the catheter 11 has contacted an object and thus an excessively large external force is applied to the wire body Wb. Even in the case where the wire body Wb receives an external force and the near end of the wire body Wb is strongly pulled in the Dc1 direction, possibility of breakage of members such as the coupling portion 21c can be reduced.

To be noted, as illustrated in FIG. 19A, a clearance Ld allowing the relative movement of the holder Wc1 and the rod Wc2 is secured between the distal end position of the rod Wc2 in the Dc2 direction in the connected state and a wall surface c118 of a bottom portion of the space c11s of the holder Wc1. The length in the Dc direction of the clearance Ld is set on the basis of a position X0 of the protrusion portions c13 in the connected state such that the wall surface c118 does not come into contact with the rod Wc2 even when the holder Wc1 has further moved in the Dc1 direction by a predetermined distance after the protrusion portions c13 are completely disengaged from the recess portions c23. As a result of securing such clearance Ld, a situation in which the rod Wc2 is pressed by the wall surface c118 of the holder Wc1 and the driving force in the Dc1 direction is transmitted even though the protrusion portions c13 have been disengaged from the recess portions c23 can be prevented.

In addition, the control portion 3 of the medical apparatus 1 may include a detection means for detecting cut-off of the connection of the connecting portion Wc and may be configured to stop the driving of each drive source M in the case where the cut-off of the connection of the connecting portion Wc is detected.

### (When Overload Is Generated in Pulling Direction)

Next, a case where an excessive force in a direction (Dc2 direction) to pull the wire body Wb acts on the connecting portion Wc will be described. As illustrated in FIG. 19C, when a driving force in a direction (Dc2 direction) to pull the wire body Wb is transmitted from the drive source M to the holder Wc1, a tensile force Ft acts between the holder Wc1 and the rod Wc2.

In the case where this tensile force Ft does not exceed a preset threshold value (first threshold value), the tensile force Ft is received by the rod Wc2 via the contact portion between the protrusion portions c13 of the holder Wc1 and the protrusions p1 of the rod Wc2. That is, the connecting portion Wc of the present embodiment is configured to maintain the state in which the protrusion portions c13 are fitted in the recess portions c23 and transmit the driving force from the drive source M to the wire body Wb in the case where the tensile force Ft equal to or smaller than the first threshold value is applied.

However, in the case where a large tensile force Ft exceeding the preset threshold value (first threshold value) is applied, the protrusions p1 of the rod Wc2 are pressed by the protrusion portions c13 of the holder Wc1, and thus the protruding pieces c22 of the rod Wc2 elastically deform inward. As a result of this, the holder Wc1 and the rod Wc2 relatively move such that the protrusion portions c13 climb over the protrusions p1, and the protrusion portions c13 are disengaged from the recess portions c23. In the state (disconnected state) in which the protrusion portions c13 are disengaged from the recess portions c23, the rod Wc2 does not move in the Dc2 direction even when the holder Wc1 moves in the Dc2 direction. That is, the connecting portion Wc of the present embodiment is configured such that in the case where the tensile force Ft exceeding the second threshold value is applied, the protrusion portions c13 are disengaged from the recess portions c23 and the transmission of the driving force from the drive source M to the wire body Wb is cut off (FIG. 20C).

As a result of the connecting portion Wc switching from the connected state to the disconnected state at the time of overload, for example, even in the case where the holder Wc1 is driven in the Dc2 direction by an excessively large driving force due to abnormal operation of the drive source M, the excessively large driving force is prevented from being transmitted to the wire body Wb. As a result of this, a situation in which the bending portion 12 of the catheter 11 is bent by an excessively strong force can be avoided.

In addition, as described above, the connecting portion Wc is switched from the connected state to the disconnected state in accordance with the magnitude of the tensile force Ft acting between the holder Wc1 and the rod Wc2. Therefore, the connection of the connecting portion Wc is cut off also in the case where the catheter 11 has contacted an object and thus an excessively large external force is applied to the wire body Wb. As a result of this, even in the case where the wire body Wb receives an external force and the near end of the wire body Wb is strongly pushed in the Dc2 direction, possibility of breakage of members such as the coupling portion 21c can be reduced.

To be noted, in the configuration example of the present embodiment, the threshold value (first threshold value) of the tensile force Ft is set to be equal to the threshold value (second threshold value) of the compressive force Fc. In this case, a configuration in which the connection between the drive source M and the wire body Wb is cut off in the case where a load exceeding a predetermined threshold value is applied to the connecting portion Wc regardless of the direction of the load is obtained.

### (Merits of Present Embodiment)

As described above, according to the present embodiment, the connection between the drive source M and the wire body Wb can be cut off in response to both an overload in a direction to press the wire body Wb and an overload in a direction to pull the wire body Wb.

In addition, in the present embodiment, each member constituting the connecting portion Wc is part of the catheter unit 100 that is a unit attachable to and detachable from another unit (base unit 200 or the like) of the medical apparatus 1. Therefore, in the case where a load exceeding a threshold value is applied to the one of the connecting portions Wc of the catheter unit 100 and the disconnected state is taken, the catheter unit 100 whose connection is cut off can be detached to attach a new catheter unit 100.

As a substitute configuration for the present embodiment, providing each of the first member connected to the drive source M and the second member connected to the wire body Wb with a magnet and connecting the first member and the second member together by magnetic force can be considered. In this case, when a load exceeding the attractive force between the magnets is applied between the first member and the second member, the first member and the second member are separated by overcoming the magnetic force, and thus the connection between the drive source M and the wire body Wb is cut off.

However, since the strength of the magnetic force generated between magnets has a nature of being inversely proportional to the square of the distance between the poles of the magnets, in the substitute configuration described above, the magnets need to be separated from each other by a long distance equal to or larger than a certain value to sufficiently cut off the connection between the drive source M and the wire body Wb. That is, there is a possibility that, to secure the distance for separation between the magnets, an installation space for the connecting portion is needed. In contrast, in the present embodiment, the interaction between the first member (holder Wc1) and the second member (rod Wc2) is basically no longer generated after the protrusion portions c13 are disengaged from the recess portion c23, and the connection between the drive source M and the wire body Wb is completely cut off. Therefore, it suffices as long as a space for the first member and the second member to relatively move by a movement amount enough for the protrusion portions c13 to be disengaged from the recess portions c23 at the time of occurrence of an overload is secured, the connection portion Wc can be easily disposed even in a small space.

In addition, in a configuration using magnets, there is a possibility that the magnetic force of a connecting portion connecting a certain pair of the drive source M and the wire body Wb and the magnetic force of a connecting portion connecting another pair of the drive source M and the wire body Wb interfere with each other and the function of the connecting portion becomes unstable. In contrast, in the present embodiment, a plurality of connecting portions do not affect each other even when being spatially close to each other. Therefore, in the case where the catheter 11 includes a plurality of wire bodies Wb, a plurality of connecting portions can be easily disposed in a small space according to the present embodiment.

In addition, to connect the first member on the drive source side and the second member on the wire body side by a sufficient connection strength by using magnets, causing the magnets to face each other in the Dc direction that is the direction of the load and securing a sufficient area of surfaces at which the magnets face each other can be considered. However, in this configuration, the installation area of the connecting portion as viewed in the Dc direction is large. In contrast, in the present embodiment, since the relative movement of the first member and the second member is restricted by the physical interference between the protrusion portions c13 and the recess portions c23, a sufficient connection strength can be secured by a small interference amount. Therefore, the connecting portion Wc can be installed in a small installation area as viewed in the Dc direction.

As another substitute configuration of the present embodiment, cutting off the connection between the drive source M and the wire body Wb by using a detection element (strain gauge or the like) and an electrically controllable clutch (electromagnetic clutch or the like) in combination and cancelling the engagement of the clutch when an overload is detected can be considered. However, in such a configuration, since the detection element and the clutch are provided, the apparatus becomes larger and more complicated. In contrast, in the present embodiment, the function of cutting off the connection between the drive source M and the wire body Wb can be realized with a simple configuration in which the protrusion portions c13 are disengaged from the recess portions c23 when an overload is generated.

### (Modification Example)

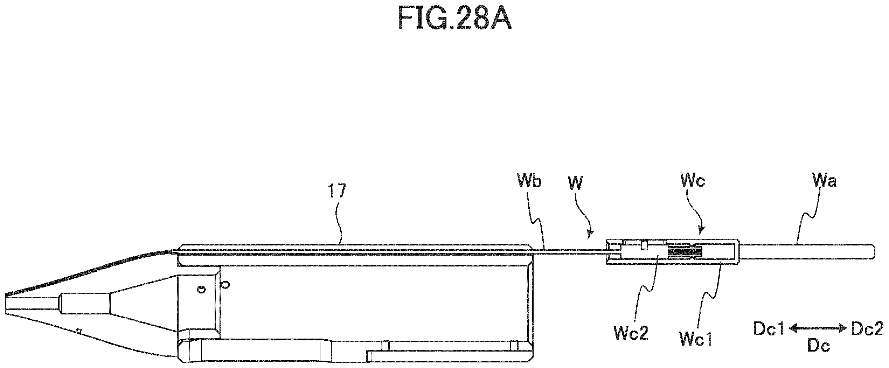

As a modification example of the first embodiment, a configuration in which the separation between the first member and the second member is restricted even in the case where the connection in the connecting portion Wc is cut off will be described with reference to FIGS. 28A to 28C, 29, and 30A to 30C. In the description below, part different from the first embodiment will be mainly described assuming that elements denoted by the same reference signs as in the first embodiment have substantially the same configurations and effects as those described in the first embodiment.

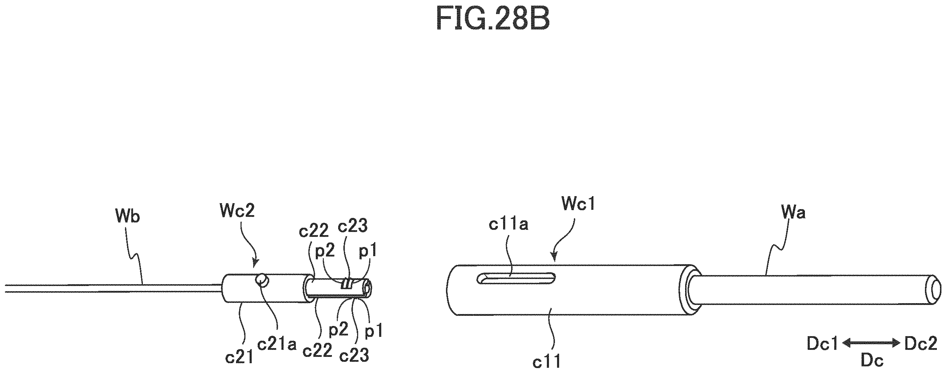

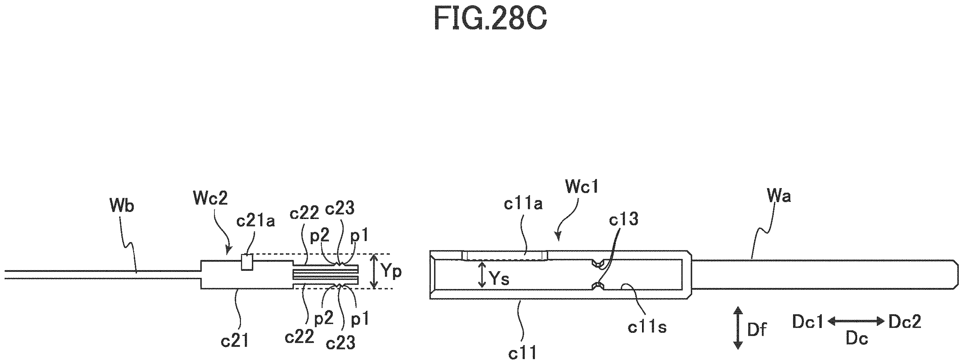

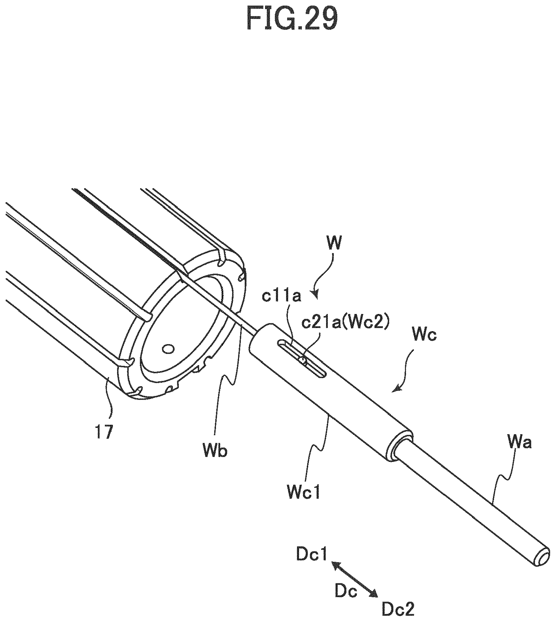

FIG. 28A is a schematic view of the driving wire W according to the present modification example including the connecting portion Wc. FIG. 28B is a perspective view of the holder Wc1 and the rod Wc2 constituting the connecting portion Wc. FIG. 28C is a section view of the holder Wc1 and the rod Wc2 constituting the connecting portion Wc. FIG. 28A illustrates a state (connected state) in which the rod Wc2 is held by the holder Wc1, and FIGS. 28B and 28C illustrate a state (unattached state) in which the rod Wc2 is not attached to the holder Wc1. FIG. 29 is a perspective view of the driving wire W according to the present modification example.

As illustrated in FIGS. 28A, 28B, 28C, and 29, the rod Wc2 serving as a second member according to the present modification example is provided with a pin c21a projecting outward from the outer circumferential surface of the base portion c21. In contrast, a slit c11a for receiving the pin c21a is provided in the cylindrical portion c11 of the holder Wc1 serving as a first member according to the present modification example. The slit c1 1a is an elongated hole extending in the extending direction (Dc direction) of the wire body Wb.

As illustrated in FIG. 29, the rod Wc2 is attached to the holder Wc1 in a state in which the pin c21a is fitted in the slit c11a. To attach the rod Wc2 including the pin c21a to the holder Wc1, for example, a configuration in which an elastic member such as a coil spring is disposed between the bottom portion of the pin 21a and the base portion c21 and the bottom portion of the pin 21a is urged to the distal end side (upper side in FIG. 28C) is employed. In this case, by inserting the rod Wc2 in the cylindrical portion c11 in a state in which the pin c21a is pushed in while compressing the elastic member, the pin c21a can be caused to pass through an opening portion of the cylindrical portion c11 and fit in the slit c11a. This is not limiting, and for example, after the rod Wc2 to which the pin c21a is not attached is inserted in the cylindrical portion c11 of the holder Wc1, the pin c21a may be inserted from the outside of the cylindrical portion c11 via the slit c11a and fixed by using an adhesive or the like.

As illustrated in FIG. 28C, the distal end of the pin c21a projects more outward than the inner circumferential surface of the cylindrical portion c11 of the holder Wc1. In other words, a height Yp of the rod Wc2 in a direction orthogonally intersecting with the extending direction (Dc direction) of the wire body Wb at the position of the pin c21a is larger than the inner diameter Ys of the cylindrical portion c11 of the holder Wc1 in a range where the slit c11a is provided. In addition, the pin c21a is configured not to be disengaged from the slit c11a at at least a threshold value (first threshold value and second threshold value) of a load by which the protrusion portions c13 are disengaged from the recess portions c23.

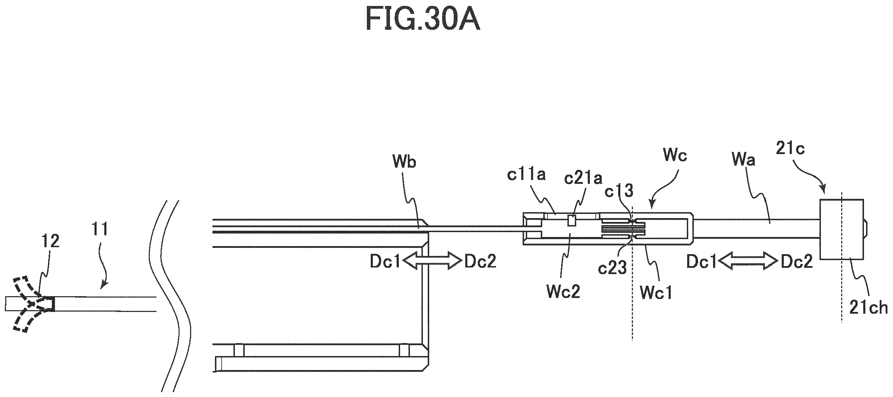

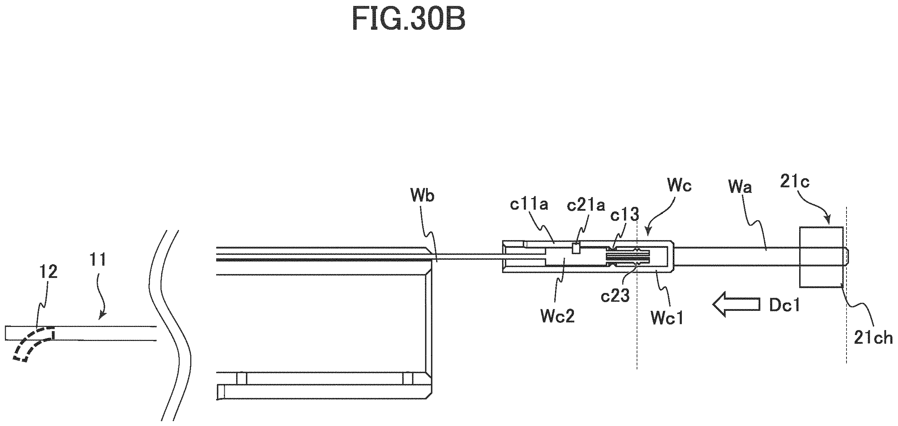

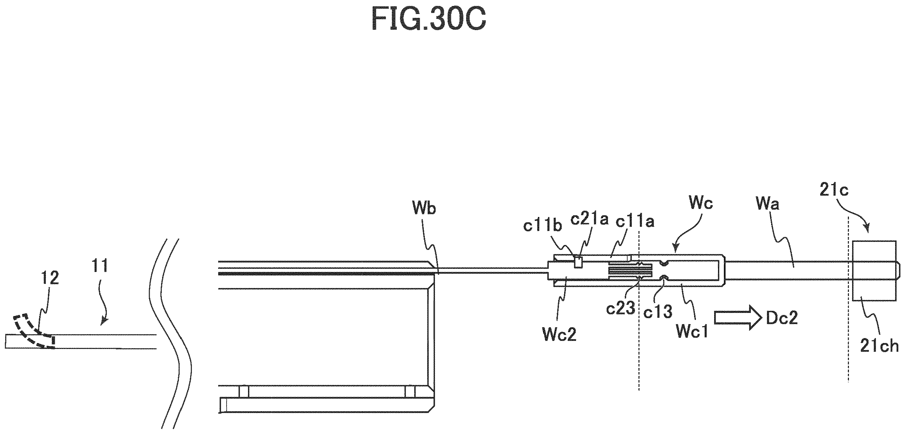

The behavior in the connected state and at the time of overload of the connecting portion Wc in the present modification example will be described with reference to FIGS. 30A to 30C. FIG. 30A is a schematic diagram illustrating a cross-section of the connecting portion Wc in the connected state. FIG. 30B is a schematic diagram illustrating the state of the connecting portion Wc in the case where an overload in a direction (Dc1 direction) to press the wire body Wb is applied. FIG. 30C is a schematic diagram illustrating the state of the connecting portion Wc in the case where an overload in a direction (Dc2 direction) to pull the wire body Wb is applied.

As illustrated in FIG. 30A, similarly to the first embodiment, in the connected state of the connecting portion Wc, the protrusion portions c13 of the holder Wc1 are fitted in the recess portions c23 of the rod Wc2. Therefore, in the connected state, the relative movement of the holder Wc1 and the rod Wc2 is restricted, and the holder Wc1 and the rod Wc2 integrally move in the Dc1 direction and the Dc2 direction. That is, the driving force from the drive source M is transmitted to the wire body Wb via the connecting portion Wc. In the connected state, the pin c21a of the rod Wc2 is positioned between the two end portions of the slit c11a of the holder Wc1 and is in contact with neither of the end portions.

As illustrated in FIGS. 30B and 30C, when an overload in the Dc1 direction or the Dc2 direction is applied to the connecting portion Wc, similarly to the first embodiment, the protrusion portions c13 are disengaged from the recess portions c23 and the transmission of the driving force from the drive source M to the wire body Wb is cut off (disconnected state).

However, even when the connecting portion Wc is in the disconnected state, the pin c21a is still fitted in the slit c11a. Therefore, in the disconnected state of the connecting portion Wc, the holder Wc1 and the rod Wc2 are connected in a state in which the relative movement of the holder Wc1 and the rod Wc2 within a range in which the pin c21a slides on the inside of the slit c11a.

In the case where the connecting portion Wc is in the disconnected state for any one of the driving wires W, the catheter unit 100 can be detached from the base unit 200 by operating the operation portion 400 described above. At this time, in the present modification example, since a state in which the holder Wc1 and the rod Wc2 are connected is maintained even after the connecting portion Wc is switched to the disconnected state, the holder Wc1 and the held portion Wa are detached together with the rod Wc2 when detaching the catheter unit 100. Specifically, when the catheter unit 100 is detached in the Dc1 direction after the connecting portion Wc is switched to the disconnected state, the holder Wc1 and the held portion Wa are pulled out together with the rod Wc2 connected to the wire body Wb in a state in which the pin c21a is engaged with an end portion c11b on the Dc1 direction side of the slit c11a as illustrated in FIG. 30C.

That is, in the case where the connecting portion Wc is configured as part of the catheter unit 100, a situation in which the first member is left on the base unit 200 side when detaching the catheter unit 100 can be prevented. Therefore, the replacement operation of the catheter unit 10 is facilitated, and the usability is improved.

The pin c21a described in the present modification example is an example of a stopper (release stopping portion, separation restricting portion) that restricts the separation between the first member and the second member in the disconnected state of the connecting portion Wc. This is not limiting, and for example, a pin serving as a stopper may be disposed on the holder Wc1, and a slit for receiving the pin may be defined in the rod Wc2. In addition, the slit c11a is not limited to a through hole, and may be a groove shape. In addition, a plurality of pairs of the pin c21a and the slit c11a may be provided to more reliably stop the release. In addition, the stopper is not limited to a pin as long as a shape capable of restricting the separation between the first member and the second member is employed. For example, an annular protrusion portion such as a snap ring may be used.

### (Other Modification Examples)

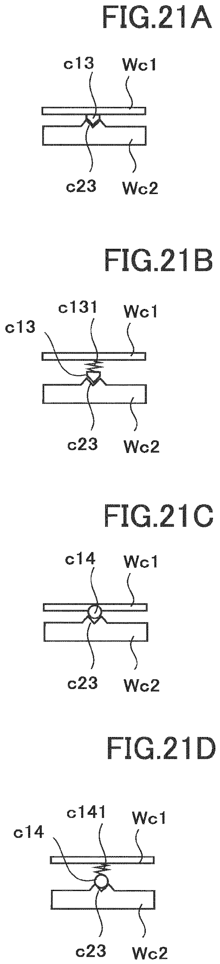

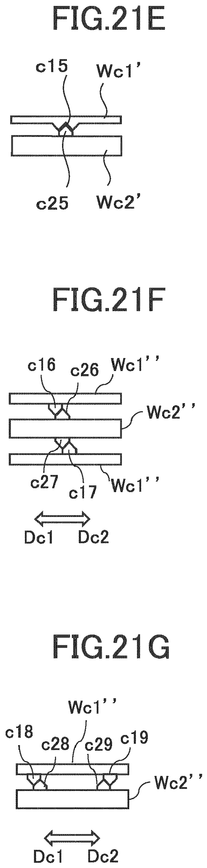

In the first embodiment, a configuration of the connecting portion Wc in which the protrusion portions c13 are disposed inside the holder Wc1 formed in a tubular shape and the recess portions c23 are provided on the two protruding pieces c22 of the rod Wc2 is employed, but a different configuration having a similar function may be employed. Modification examples of the connecting portion Wc are exemplified in FIGS. 21Ato 21G.

FIG. 21A is a schematic diagram schematically illustrating a connection configuration of the holder Wc1 and the rod Wc2 exemplified in the first embodiment. As described above, the holder Wc1 and the rod Wc2 are connected and integrally move in the Dc direction as a result of the protrusion portions c13 provided on the holder Wc1 fitting in the recess portions c23 provided on the rod Wc2.

FIG. 21B illustrates a modification example in which the protrusion portions c13 provided on the holder Wc1 are urged toward the recess portions c23 by elastic members c131 such as springs. In this case, the magnitude of the load by which the protrusion portions c13 are disengaged from the recess portions c23, that is, the threshold value (first threshold value, second threshold value) of the load by which the connection of the connecting portion Wc is cut off can be changed by changing the spring constant or the deformation amount in the connected state of the elastic members c131.

FIG. 21C illustrates a modification example in which protrusion portions c14 provided on the holder Wc1 are rotary members having spherical or cylindrical shapes rotatably supported by support portions of the holder Wc1. In this case, the influence of friction on a contact surface when the protrusion portions c13 are disengaged from the recess portions c23 is reduced. Therefore, the magnitude of the load by which the protrusion portions c13 are disengaged from the recess portions c23, that is, the threshold value (first threshold value, second threshold value) of the load by which the connection of the connecting portion Wc is cut off can be set with a higher precision.

FIG. 21D illustrates a modification example in which the protrusion portions c14 provided on the holder Wc1 are rotary members and the protrusion portions c13 are urged toward the recess portions c23 by the elastic members c131 such as springs. In this case, the threshold value (first threshold value, second threshold value) of the load by which the connection of the connecting portion Wc is cut off can be changed by changing the elastic members c131, and the threshold value of the load (first threshold value, second threshold value) can be set with a higher precision.

FIG. 21E illustrates a modification example in which a recess portion c15 is provided on a first member Wc1' and a protrusion portion c25 is provided on a second member Wc2'. The present modification example can be realized by swapping the holder Wc1 used as a first member in the first embodiment and the rod Wc2 used as a second member. That is, a member having the same shape as the holder Wc1 of the first embodiment may be attached to the near end of the wire body Wb as the first member Wc1' and a member having the same shape as the rod Wc2 of the first embodiment may be integrally formed with the held portion Wa as the second member Wc2' . To be noted, the second member Wc2' may be a separate member from the held portion Wa.

In addition, as another configuration for realizing the modification example of FIG. 21E, instead of the protrusion portions c13, two protrusions aligned in the Dc direction may be provided on the inside of the cylindrical portion c11 of the holder Wc1 of the first embodiment to form the recess portion c15, and a protrusion portion that fits in this recess portion may be provided on a protruding piece c22 of the rod Wc2. That is, in this case, similarly to the configuration of FIGS. 21Ato 21D, the holder Wc1 is integrally formed with the held portion Wa, and the rod Wc2 is attached to the near end of the wire body Wb. As described above, according to a configuration in which a protrusion portion is provided on one of the first member and the second member and a recess portion in which the protrusion is fitted is provided on the other of the first member and the second member, a connecting portion having a similar function to the first embodiment can be formed. To be noted, the positions of the protrusion portions and the recess portions can be swapped for also the second to fourth embodiments described below as long as a protrusion portion is provided on either one of the first member and the second member and a recess portion in which the protrusion portion is fitted is provided on the other of the first member and the second member.

FIG. 21F illustrates a modification example in which a protrusion portion c16 and a protrusion portion c17 are respectively provided at different positions on a first member Wc1" and a protrusion portion c26 and a protrusion portion c27 are respectively provided at different positions on a second member Wc2". In the present modification example, for example, a member in which the protrusion portions c16 and c17 are disposed on the holder Wc1 of the first embodiment instead of the protrusion portions c13 is integrally formed with the held portion Wa as the first member Wc1". Further, it can be realized by attaching a member in which the protrusion portions c26 and c27 are disposed on the rod Wc2 of the first embodiment instead of the protrusions p1 and p2 to the near end of the wire body Wb as the second member Wc2'. To be noted, the first member Wc1' and the held portion Wa may be separate members.

The protrusion portion c16 is an example of a first protrusion portion, the protrusion portion c17 is an example of a second protrusion portion, the protrusion portion c26 is an example of a third protrusion position that engages with the first protrusion portion, and the protrusion portion c27 is an example of a fourth protrusion portion that engages with the second protrusion portion. To be noted, also for second to fourth embodiments described below, a function similar to that of the combination of protrusion portions and recess portions can be obtained by a configuration in which a first protrusion portion and a second protrusion portion are disposed instead of the "protrusion portions" of the first member and a third protrusion portion and a fourth protrusion portion are disposed instead of the "recess portions" of the second embodiment.

In the first embodiment and modification examples described above, the connecting portion Wc is formed by the fitting between the protrusion portions and the recess portions. However, this configuration is not limiting, and the connecting portion Wc may be formed by engagement between protrusion portions at two different positions as exemplified in the present modification example and a modification example of FIG. 21F described below. In other words, the connecting portion Wc is sufficient as long as a shape on the first member side and a shape of the second member side are engaged to restrict relative movement of the first member and the second member in the Dc1 direction and the Dc2 direction regardless of the specific shapes of the first member and the second member. At this time, if there is a portion where part of the second member abuts part of the first member from the Dc1 direction side and a portion where part of the second member abuts part of the first member from the Dc2 direction side, the relative movement of the first member and the second member in the Dc1 direction and the Dc2 direction is restricted. In addition, by employing a configuration in which the former contact portion is disengaged due to an overload in the Dc1 direction and the latter contact portion is disengaged by an overload in the Dc2 direction, the connection between the first member and the second member is cut off in response to the overload.

In the configuration of FIG. 21F, in the case where a driving force is applied in the Dc1 direction, since the first member Wc1" attempts to move in the Dc1 direction, the first member Wc1" and the second member Wc2" move integrally due to the engagement between the protrusion portion c17 and the protrusion portion c27. When an overload occurs at this time, the protrusion portion c17 moves in the Dc1 direction beyond the protrusion portion c27, and the connection between the first member Wc1" and the second member Wc2" is cut off. Meanwhile, in the case where a driving force is applied in the Dc2 direction, since the first member Wc1" attempts to move in the Dc2 direction, the first member Wc1" and the second member Wc2" move integrally due to the engagement between the protrusion portion c16 and the protrusion portion c26. When an overload occurs at this time, the protrusion portion c16 moves in the Dc2 direction beyond the protrusion portion c26, and the connection between the first member Wc1" and the second member Wc2" is cut off.

To be noted, although the protrusion portion c26 and the protrusion portion c27 are provided at positions on opposite sides in the circumferential direction of the second member Wc2" having an approximate columnar shape in the configuration of FIG. 21F, this is not limiting. As illustrated in FIG. 21G, a configuration in which a protrusion portion c28 and a protrusion portion c29 are provided at different positions in the extending direction of the second member Wc2" may be employed. FIG. 21G illustrates a modification example in which a protrusion portion c18 and a protrusion portion c19 are provided at different positions on the first member Wc1" and a protrusion portion c28 and a protrusion portion c29 are provided at different positions on the second member Wc2". The protrusion portion c18 is an example of a first protrusion portion, the protrusion portion c19 is an example of a second protrusion portion, the protrusion portion c28 is an example of third protrusion portion that engages with the first protrusion portion, and the protrusion portion c29 is an example of a fourth protrusion portion that engages with the second protrusion portion.

In the configuration of the FIG. 21G, in the case where a driving force is applied in the Dc1 direction, since the first member Wc1" attempts to move in the Dc1 direction, the first member Wc1" and the second member Wc2" move integrally due to the engagement between the protrusion portion c19 and the protrusion portion c29. When an overload occurs at this time, the protrusion portion c19 moves in the Dc1 direction beyond the protrusion portion c29, and the connection between the first member Wc1" and the second member Wc2" is cut off. Meanwhile, in the case where a driving force is applied in the Dc2 direction, since the first member Wc1" attempts to move in the Dc2 direction, the first member Wc1" and the second member Wc2" move integrally due to the engagement between the protrusion portion c18 and the protrusion portion c28. When an overload occurs at this time, the protrusion portion c18 moves in the Dc2 direction beyond the protrusion portion c28, and the connection between the first member Wc1" and the second member Wc2" is cut off.

In addition, in this configuration, even if an overload occurs in the Dc1 direction and the engagement between the protrusion portions c19 and c29 is cancelled, the protrusion portion c19 starts engaging with the protrusion portion c28 next. Similarly, even if an overload occurs in the Dc2 direction and the engagement between the protrusion portions c18 and c28 is cancelled, the protrusion portion c18 starts engaging with the protrusion portion c29 next. That is, even if the breakaway functions and the transmission of the driving force is temporarily cut off, the integral nature of the first member Wc1" and the second member Wc2" is maintained, and the first member Wc1" and the second member Wc2" are not completely separated. As a result of this, in the case where, for example, the catheter 11 is pulled out from the inside of the body of the patient and the catheter unit 100 is detached from the base unit 200 for replacement by a new one, the following effect is obtained. That is, the possibility of the first member Wc1" connected to the held portion Wa remaining on the base unit 200 side can be reduced. Therefore, the usability is improved.

To be noted, although configurations in which the first member Wc1" and the second member Wc2" are engaged with each other at two positions are illustrated in FIGS. 21F and 21G, these may be engaged at three or more positions as a matter of course, and the shape of the protrusion portion is neither limited. Further, an elastic member such as a spring illustrated in FIGS. 21B and 21D may be attached to the protrusion portion.

Further, as another modification example, a configuration in which, in the case where a compressive force or a tensile force equal to or larger than a threshold value is applied between the first member and the second member, the protrusion portions or the recess portions plastically deform (yield) to disengage the protrusion portions from the recess portions may be employed. In this case, in the modification example based on FIG. 21G, a configuration in which the first protrusion portion c18 or the third protrusion portion c28 plastically deforms by a compressive force equal to or larger than the threshold value to disengage (release engagement of) the protrusion portions c18 and c28 may be employed. In addition, in the modification example based on FIG. 21G, a configuration in which the second protrusion portion c19 or the fourth protrusion portion c29 plastically deforms by a tensile force equal to or larger than the threshold value to disengage (release engagement of) the protrusion portions c19 and c29 may be employed. The function of cutting off the connection between the drive source M and the wire body Wb at the time of occurrence of an overload can be also realized by irreversible deformation as described above.

### [Second Embodiment]

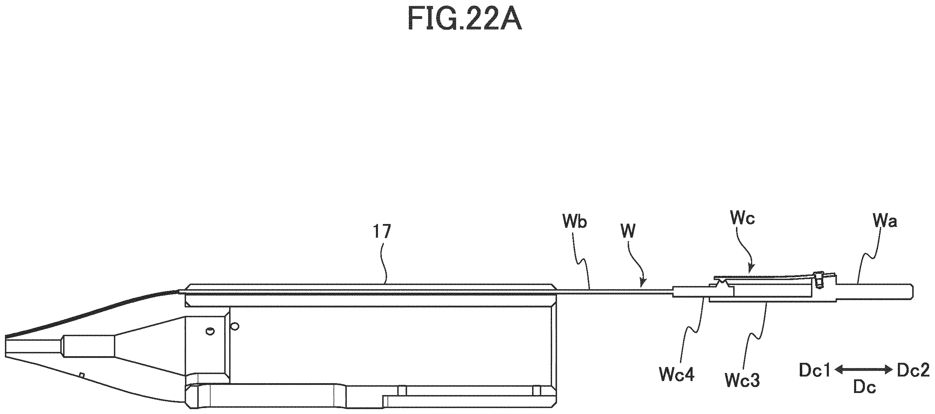

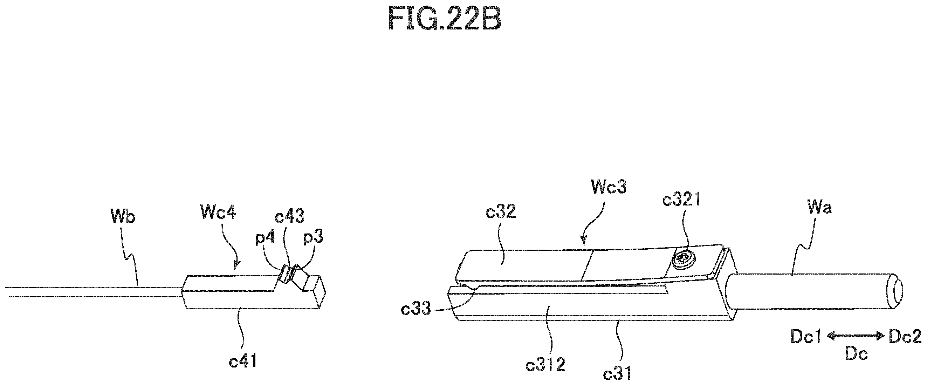

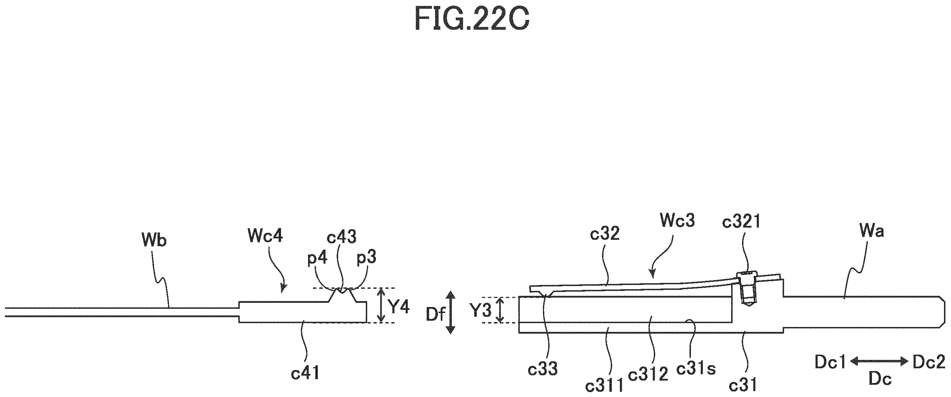

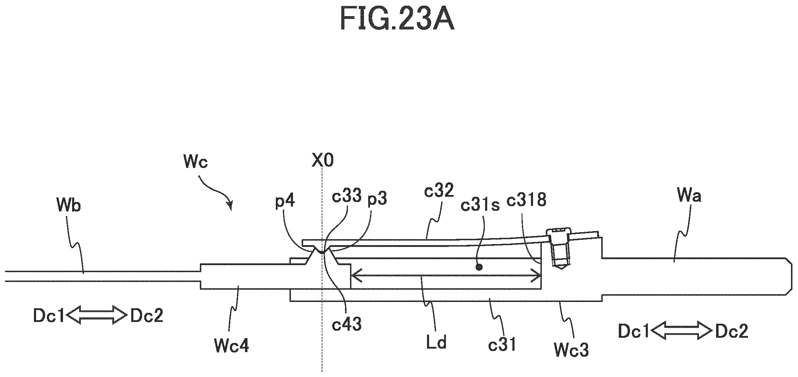

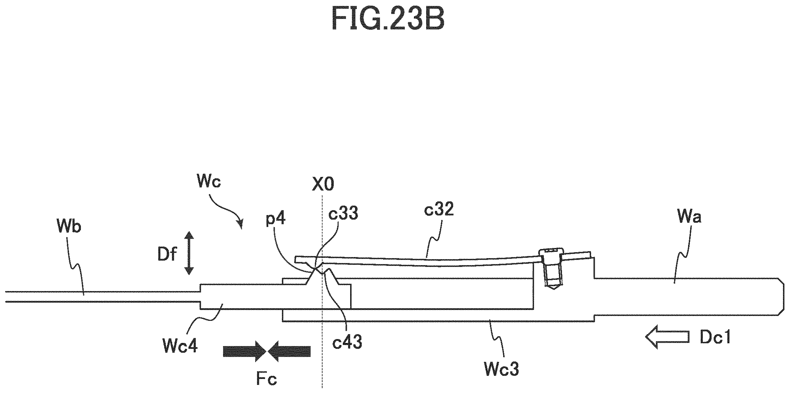

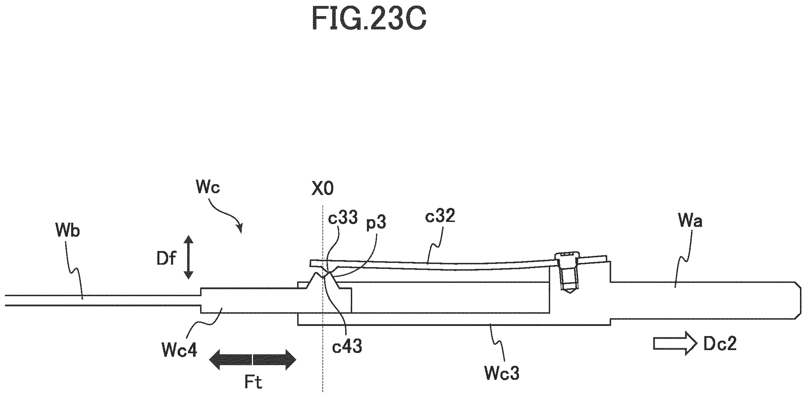

A medical apparatus according to a second embodiment will be described with reference to FIGS. 22A, 22B, 22C, 23A, 23B, and 23C. In the present embodiment, the configuration of the connecting portion Wc interconnecting the drive source M and the wire body Wb is different from the first embodiment. In the description below, part different from the first embodiment will be mainly described assuming that elements denoted by the same reference signs as in the first embodiment have substantially the same configurations and effects as those described in the first embodiment.

FIG. 22A is a schematic view of the driving wire W according to the present embodiment including the connecting portion Wc. FIG. 22B is a perspective view of a holder Wc3 and a rod Wc4 constituting the connecting portion Wc. FIG. 22C is a section view of the holder Wc3 and the rod Wc4 constituting the connecting portion Wc. FIG. 22A illustrates a state (connected state) in which the rod Wc4 is held by the holder Wc3, and FIGS. 22B and 22C illustrate a state (disconnected state) in which the rod Wc4 is not held by the holder Wc3.

In the description below, the connecting portion Wc provided on arbitrary one driving wire W of the first to ninth driving wires (W11 to W33) included in the catheter unit 100 of the present embodiment will be described. In the configuration example of the present embodiment, the connecting portion Wc having substantially the same configuration as that described below is provided on each of the ninth driving wires (W11 to W33).

As illustrated in FIG. 22A, the connecting portion Wc includes the holder Wc3 serving as a first member (engaging member, holding member) connected to the drive source M, and the rod Wc4 serving as a second member (engaged member, held member) connected to the wire body Wb of the driving wire W. In the present embodiment, at least part of the holder Wc3 is a member integrally formed with the held portion Wa of the driving wire W. Therefore, the holder Wc3 is connected to the drive source M via the held portion Wa and the coupling portion 21c (FIGS. 6A to 6C). To be noted, the holder Wc3 and the held portion Wa may be separate bodies. Meanwhile, the rod Wc4 is fixed to the near end of the wire body Wb by an arbitrary fixing method such as compression bonding (swaging) or gluing.

The holder Wc3 includes a body portion (base portion) c31 integrally formed with the held portion Wa and a plate spring c32 attached to the body portion c31 with a fixing means such as a screw c321 as illustrated in FIGS. 22B and 22C. The body portion c31 has a groove shape having a C-shaped cross-section (rectangular shape opening in one direction) surrounded by a bottom portion c311 and a side wall portion c312 as viewed in the Dc direction. The plate spring c32 is attached to cover the opening portion of the body portion c31 as viewed in the Df direction, and extends in the Dc1 direction from the screw c321. Therefore, a space c31s for receiving the rod Wc4 as will be described below is defined between the bottom portion c311 and the side wall portion c312 of the body portion c31 and the plate spring c32. The body portion c31 is aligned with the held portion Wa in the Dc direction, and the held portion Wa extends in the Dc2 direction from an end portion of the body portion c31 in the Dc2 direction.

In addition, the holder Wc3 includes a protrusion portion c33 at a distal end of the plate spring c32. The protrusion portion c33 is provided to protrude from the distal end portion of the plate spring c32 in the Dc2 direction toward the space c31s between the body portion c31 and the plate spring c32. That is, the protrusion portion c33 is an example of a protrusion portion protruding in a direction intersecting with the extending direction (Dc direction) of the wire body Wb in the connecting portion Wc.

The plate spring c32 mainly deforms in the Df direction when receiving an external force. That is, the plate spring c32 functions as a deforming element capable of deforming to allow the protrusion portion c33 to be disengaged from a recess portion c43 that will be described later.

The protrusion portion c33 is disposed at a position corresponding to the recess portion c43 that will be described later on the rod Wc4 as viewed in the Dc direction. In the present embodiment, the protrusion portion c33 is provided at one position in correspondence with the recess portion c43 provided at one position. However, it suffices as long as the protrusion portion c33 is provided at a position engageable with the recess portion c43, and for example, a plurality of plate springs c32 including the protrusion portion c33 may be provided, and a plurality of recess portions c43 corresponding thereto may be provided on the rod Wc4.

As illustrated in FIGS. 22B and 22C, the rod Wc4 includes a base portion c41 having an approximate quadrangular prismatic shape extending in the Dc2 direction from the near end of the wire body Wb, and a recess portion c43 provided on a side surface (surface facing the plate spring c32) of the base portion c41 in the Df direction. The recess portion c43 is formed between protrusions p3 and p4 protruding in the Df direction from the base portion c41. The protrusions p3 and p4 are arranged in the Dc direction.

The rod Wc4 is inserted in the Dc2 direction toward the space c31s between the body portion c31 and the plate spring c32 of the holder Wc3, is pushed in until the protrusion portion c33 of the holder Wc3 fits in the recess portion c43 of the rod Wc4, and is thus attached to the holder Wc3. In the present embodiment, part of the rod Wc4 on the Dc2 direction side is an insertion portion to be inserted into the space c31s in the holder Wc3.

Next, the behavior of the connecting portion Wc in the connected state and at the time of an overload will be described with reference to FIGS. 23A to 23C. FIG. 23A is a schematic diagram illustrating the cross-section of the connecting portion Wc in the connected state. FIG. 23B is a schematic diagram illustrating the state of the connecting portion Wc in the case where an overload is applied in a direction (Dc1 direction) to press the wire body Wb. FIG. 23C is a schematic diagram illustrating the state of the connecting portion Wc in the case where an overload is applied in a direction (Dc2 direction) to pull the wire body Wb.

As illustrated in FIG. 23A, in the connected state of the connecting portion Wc, the protrusion portion c33 of the holder Wc3 is fitted in the recess portion c43 of the rod Wc4. Specifically, the protrusion portion c33 is held between the protrusions p3 and p4 in the Dc direction. Here, as illustrated in FIG. 22C, a distance in the Df direction from the bottom surface of the base portion c41 to the apices of the protrusions p3 and p4 in a state in which the rod Wc4 is not attached to the holder Wc3 is denoted by Y4. This distance Y4 is larger than a distance Y3 in the Df direction from the protrusion portion c33 of the plate spring c32 of the holder Wc3 to a bottom portion c311 of the body portion c31. Therefore, in the connected state illustrated in FIG. 23A, the protrusion portion c33 of the holder Wc3 and part of the rod Wc4 (protrusions p3 and p4) are in such a positional relationship as to interfere with each other in the Dc direction. In other words, at least part of a protrusion portion provided on one of the first member and the second member overlaps a recess portion provided on the other of the first member and the second member as viewed in the extending direction (Dc direction) of the wire body in the connected state.

According to such a configuration, in the case where the connecting portion Wc is in the connected state, relative movement of the holder Wc3 and the rod Wc4 is restricted, and thus the holder Wc3 and the rod Wc4 integrally move in the Dc1 direction and the Dc2 direction. That is, in the case where the connecting portion Wc is in the connected state, the driving force from the drive source M is transmitted to the wire body Wb through the connecting portion Wc.

In the connected state of FIG. 23A, the protrusion portion c33 of the holder Wc3 is configured to fit in the recess portions c43 of the rod Wc4 in a state in which the backlash is reduced. In the present embodiment, the protrusion portion 33 is provided at a distal end portion of the plate spring c32 having elasticity, and the protrusion portion c33 is pressed against the protrusions p3 and p4 on the two sides thereof by the elastic force of the plate spring c32 as a result of the plate spring c32 being slightly elastically deformed in the Df direction (upward in the drawing) in the connected state. That is, the connecting portion Wc according to the present embodiment connects the holder Wc3 serving as a first member and the rod Wc4 serving as a second member by a mechanism (snap fit mechanism) including a deforming element formed from an elastic member.

As described above, the responsiveness of the bending portion 12 of the catheter 11 to the driving of the drive source M can be improved by the configuration of engaging the holder Wc3 and the rod Wc4 with each other in the state in which the backlash is reduced.

### (When Overload Is Generated in Pressing Direction)

Next, a case where an excessive force in a direction (Dc1 direction) to press the wire body Wb acts on the connecting portion Wc will be described. As illustrated in FIG. 23B, when a driving force in a direction (Dc1 direction) to press the wire body Wb is transmitted from the drive source M to the holder Wc3, a compressive force Fc acts between the holder Wc3 and the rod Wc4.

In the case where this compressive force Fc does not exceed a preset threshold value (second threshold value), the compressive force Fc is received by the rod Wc4 via the contact portion between the protrusion portion c33 of the holder Wc3 and the protrusion p4 of the rod Wc4. That is, the connecting portion Wc of the present embodiment is configured to maintain the state in which the protrusion portion c33 is fitted in the recess portion c43 and transmit the driving force from the drive source M to the wire body Wb in the case where the compressive force Fc equal to or smaller than the second threshold value is applied.

However, in the case where a large compressive force Fc exceeding the preset threshold value (second threshold value) is applied, the plate spring c32 elastically deforms such that the protrusion portion c33 climbs up the protrusion p4 of the rod Wc4. As a result of this, the holder Wc3 and the rod Wc4 relatively move while the protrusion portion c33 climbs over the protrusion p4, and the protrusion portion c33 is disengaged from the recess portions c43. In the state (disconnected state) in which the protrusion portion c33 is disengaged from the recess portion c43, the rod Wc4 does not move in the Dc1 direction even when the holder Wc3 moves in the Dc1 direction. That is, the connecting portion Wc of the present embodiment is configured such that in the case where the compressive force Fc exceeding the second threshold value is applied, the protrusion portion c33 is disengaged from the recess portion c43 and the transmission of the driving force from the drive source M to the wire body Wb is cut off (FIG. 23B).

As a result of the connecting portion Wc switching from the connected state to the disconnected state at the time of overload, a possibility that the bending portion 12 of the catheter 11 is bent by an excessively strong force due to an operation abnormality of the drive source M or a member such as the coupling portion 21c is damaged when the wire body Wb receives an external force can be reduced.

To be noted, as illustrated in FIG. 23A, a clearance Ld allowing the relative movement of the holder Wc3 and the rod Wc4 is secured between the distal end position of the rod Wc4 in the Dc2 direction in the connected state and the wall surface c318 of the bottom portion of the space c31s of the holder Wc3. The length in the Dc direction of the clearance Ld is set on the basis of a position X0 of the protrusion portion c33 in the connected state such that the wall surface c318 does not come into contact with the rod Wc4 even when the holder Wc3 has further moved in the Dc1 direction by a predetermined distance after the protrusion portion c33 is completely disengaged from the recess portion c43. As a result of securing such clearance Ld, a situation in which the rod Wc4 is pressed by the wall surface c318 of the holder Wc3 and the driving force in the Dc1 direction is transmitted even though the protrusion portion c33 has been disengaged from the recess portion c43 can be prevented.

### (When Overload Is Generated in Pulling Direction)

Next, a case where an excessive force in a direction (Dc2 direction) to pull the wire body Wb acts on the connecting portion Wc will be described. As illustrated in FIG. 23C, when a driving force in a direction (Dc2 direction) to pull the wire body Wb is transmitted from the drive source M to the holder Wc3, a tensile force Ft acts between the holder Wc3 and the rod Wc4.

In the case where this tensile force Ft does not exceed a preset threshold value (first threshold value), the tensile force Ft is received by the rod Wc4 via the contact portion between the protrusion portion c33 of the holder Wc3 and the protrusion p3 of the rod Wc4. That is, the connecting portion Wc of the present embodiment is configured to maintain the state in which the protrusion portion c33 is fitted in the recess portion c43 and transmit the driving force from the drive source M to the wire body Wb in the case where the tensile force Ft equal to or smaller than the first threshold value is applied.

However, in the case where a large tensile force Ft exceeding the preset threshold value (first threshold value) is applied, the plate spring c32 elastically deforms such that the protrusion portion c33 climbs up the protrusion p3 of the rod Wc4. As a result of this, the holder Wc3 and the rod Wc4 relatively move while the protrusion portion c33 climbs over the protrusion p3, and the protrusion portion c33 is disengaged from the recess portions c43. In the state (disconnected state) in which the protrusion portion c33 is disengaged from the recess portion c43, the rod Wc4 does not move in the Dc2 direction even when the holder Wc3 moves in the Dc2 direction. That is, the connecting portion Wc of the present embodiment is configured such that in the case where the tensile force Ft exceeding the second threshold value is applied, the protrusion portion c33 is disengaged from the recess portion c43 and the transmission of the driving force from the drive source M to the wire body Wb is cut off (FIG. 23C).

As a result of the connecting portion Wc switching from the connected state to the disconnected state at the time of overload, a possibility that the bending portion 12 of the catheter 11 is bent by an excessively strong force due to an operation abnormality of the drive source M or a member such as the coupling portion 21c is damaged when the wire body Wb receives an external force can be reduced.

As described above, also according to the present embodiment, the connection between the drive source M and the wire body Wb can be cut off in response to both an overload in a direction to press the wire body Wb and an overload in a direction to pull the wire body Wb.

### [Third Embodiment]

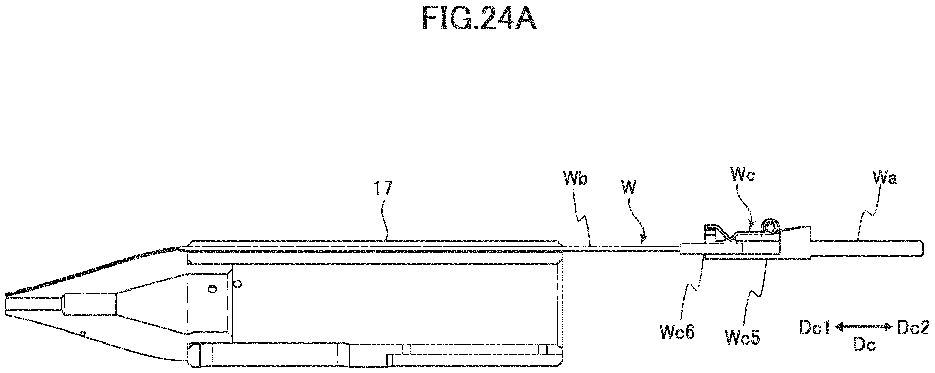

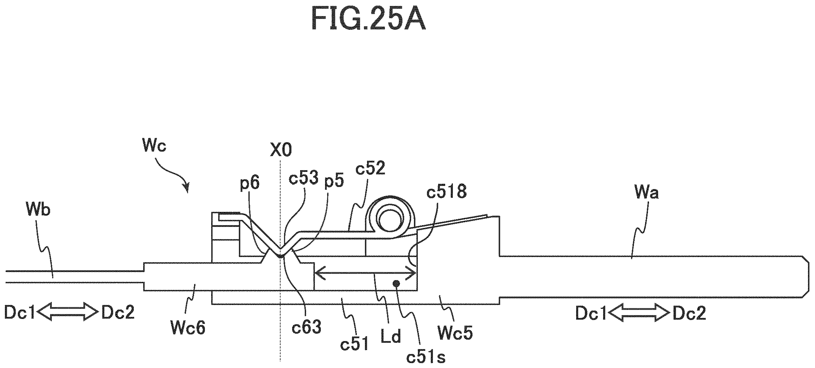

A medical apparatus according to a third embodiment will be described with reference to FIGS. 24A to 24C and 25A to 25C. In the present embodiment, the configuration of the connecting portion Wc interconnecting the drive source M and the wire body Wb is different from the first embodiment. In the description below, part different from the first embodiment will be mainly described assuming that elements denoted by the same reference signs as in the first embodiment have substantially the same configurations and effects as those described in the first embodiment.

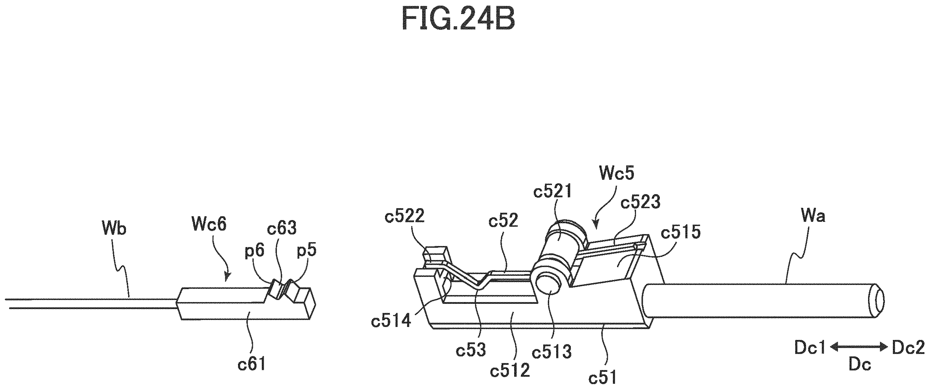

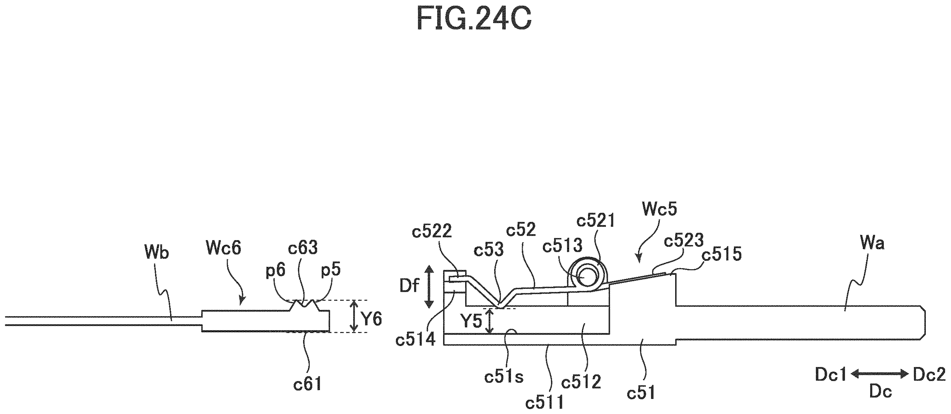

FIG. 24A is a schematic view of the driving wire W according to the present embodiment including the connecting portion Wc. FIG. 24B is a perspective view of a holder Wc5 and a rod Wc6 constituting the connecting portion Wc. FIG. 24C is a section view of the holder Wc5 and the rod Wc6 constituting the connecting portion Wc. FIG. 24A illustrates a state (connected state) in which the rod Wc6 is held by the holder Wc5, and FIGS. 24B and 24C illustrate a state (disconnected state) in which the rod Wc6 is not held by the holder Wc5.

In the description below, the connecting portion Wc provided on arbitrary one driving wire W of the first to ninth driving wires (W11 to W33) included in the catheter unit 100 of the present embodiment will be described. In the configuration example of the present embodiment, the connecting portion Wc having substantially the same configuration as that described below is provided on each of the ninth driving wires (W11 to W33).

As illustrated in FIG. 24A, the connecting portion Wc includes the holder Wc5 serving as a first member (engaging member, holding member) connected to the drive source M, and the rod Wc6 serving as a second member (engaged member, held member) connected to the wire body Wb of the driving wire W. In the present embodiment, at least part of the holder Wc5 is a member integrally formed with the held portion Wa of the driving wire W. Therefore, the holder Wc5 is connected to the drive source M via the held portion Wa and the coupling portion 21c (FIGS. 6A to 6C). To be noted, the holder Wc5 and the held portion Wa may be separate bodies. Meanwhile, the rod Wc6 is fixed to the near end of the wire body Wb by an arbitrary fixing method such as compression bonding (swaging) or gluing.

The holder Wc5 includes a body portion (base portion) c51 integrally formed with the held portion Wa and a torsion spring (torsion coil spring) c52 supported by the body portion c31 as illustrated in FIGS. 24B and 24C. The body portion c51 has a groove shape having a C-shaped cross-section (rectangular shape opening in one direction) surrounded by a bottom portion c511 and a side wall portion c512 as viewed in the Dc direction. The torsion spring c52 is attached to an opening portion of the body portion c51 as viewed in the Df direction. Therefore, a space c51s for receiving the rod Wc6 as will be described below is defined between the bottom portion c511 and the side wall portion c512 of the body portion c51 and the torsion spring c52. The body portion c51 is aligned with the held portion Wa in the Dc direction, and the held portion Wa extends in the Dc2 direction from an end portion of the body portion c51 in the Dc2 direction.

The torsion spring c52 includes a coil portion c521 supported by a shaft portion c513 attached to the body portion c51, an arm portion c522 extending in the Dc1 direction from the coil portion c521, and an arm portion c523 extending in the Dc2 direction from the coil portion c521. The arm portion c522 on the Dc1 direction side is a free end supported by a support portion 514 of the body portion c51 and capable of elastically deforming to be separated from the support portion 514 by being pressed by the rod Wc6. The arm portion c523 on the Dc2 direction side is a fixed end that is supported by a support portion c515 of the body portion c51 and that remains in contact with the support portion c515 even in the case where the other arm portion c522 is pressed by the rod Wc6.

In addition, the holder Wc5 includes a protrusion portion c53 that is a bent shape formed in the arm portion c523 on the free end side of the torsion spring c52. The protrusion portion c53 protrudes toward the space c51s between the torsion spring c52 and the body portion c51 at a position between a distal end of the arm portion c523 and the coil portion c521. That is, the protrusion portion c53 is an example of a protrusion portion protruding in a direction intersecting with the extending direction (Dc direction) of the wire body Wb in the connecting portion Wc.

The torsion spring c52 mainly deforms in the Df direction when receiving an external force. That is, the torsion spring c52 functions as a deforming element capable of deforming to allow the protrusion portion c53 to be disengaged from a recess portion c63 that will be described later.

The protrusion portion c53 is disposed at a position corresponding to the recess portion c63 that will be described later on the rod Wc6 as viewed in the Dc direction. In the present embodiment, the protrusion portion c53 is provided at one position in correspondence with the recess portion c63 provided at one position. However, it suffices as long as the protrusion portion c53 is provided at a position engageable with the recess portion c63, and for example, a plurality of torsion springs c52 including the protrusion portion c53 may be provided, and a plurality of recess portions c63 corresponding thereto may be provided on the rod Wc6.

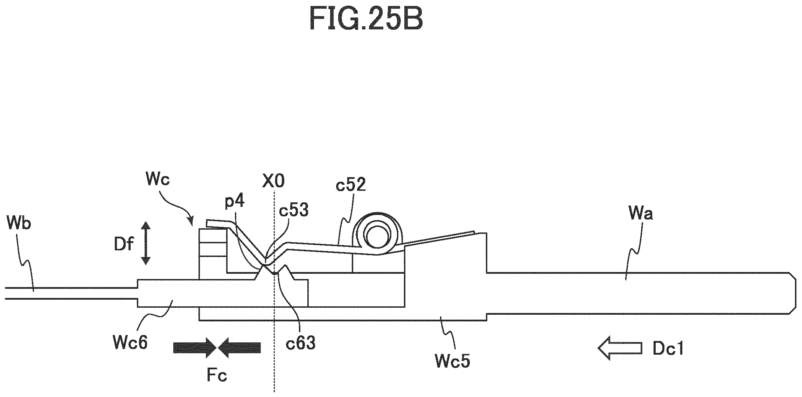

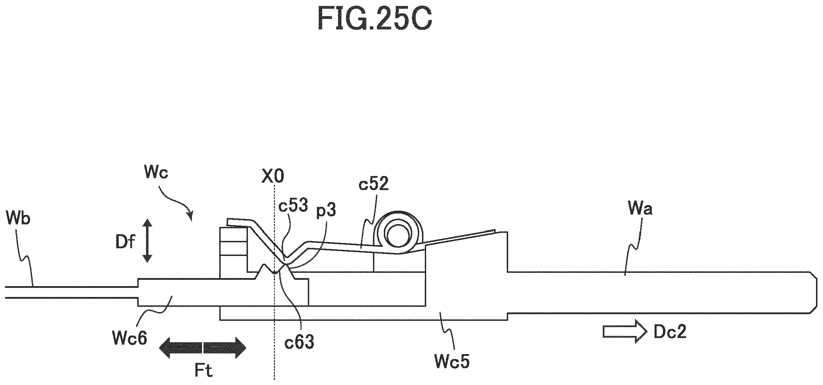

As illustrated in FIGS. 25B and 25C, the rod Wc6 includes a base portion c61 having an approximate quadrangular prismatic shape extending in the Dc2 direction from the near end of the wire body Wb, and a recess portion c63 provided on a side surface (surface facing the torsion spring c52) of the base portion c61 in the Df direction. The recess portion c63 is formed between protrusions p5 and p6 protruding in the Df direction from the base portion c61. The protrusions p5 and p6 are arranged in the Dc direction.

The rod Wc6 is inserted in the Dc2 direction toward the space c51s between the body portion c51 and the torsion spring c52 of the holder Wc5, is pushed in until the protrusion portion c53 of the holder Wc5 fits in the recess portion c63 of the rod Wc6, and is thus attached to the holder Wc5. In the present embodiment, part of the rod Wc6 on the Dc2 direction side is an insertion portion to be inserted into the space c51s in the holder Wc5.

Next, the behavior of the connecting portion Wc in the connected state and at the time of an overload will be described with reference to FIGS. 25A to 25C. FIG. 25A is a schematic diagram illustrating the cross-section of the connecting portion Wc in the connected state. FIG. 25C is a schematic diagram illustrating the state of the connecting portion Wc in the case where an overload is applied in a direction (Dc1 direction) to press the wire body Wb. FIG. 25C is a schematic diagram illustrating the state of the connecting portion Wc in the case where an overload is applied in a direction (Dc2 direction) to pull the wire body Wb.

As illustrated in FIG. 25A, in the connected state of the connecting portion Wc, the protrusion portion c53 of the holder Wc5 is fitted in the recess portion c63 of the rod Wc6. Specifically, the protrusion portion c53 is held between the protrusions p5 and p6 in the Dc direction. Here, as illustrated in FIG. 24C, a distance in the Df direction from the bottom surface of the base portion c61 to the apices of the protrusions p5 and p6 in a state in which the rod Wc6 is not attached to the holder Wc5 is denoted by Y6. This distance Y6 is larger than a distance Y5 in the Df direction from the protrusion portion c53 of the torsion spring c52 of the holder Wc5 to the bottom portion c511 of the body portion c51. Therefore, in the connected state illustrated in FIG. 25A, the protrusion portion c53 of the holder Wc5 and part of the rod Wc6 (protrusions p5 and p6) are in such a positional relationship as to interfere with each other in the Dc direction. In other words, at least part of a protrusion portion provided on one of the first member and the second member overlaps a recess portion provided on the other of the first member and the second member as viewed in the extending direction (Dc direction) of the wire body in the connected state.

According to such a configuration, in the case where the connecting portion Wc is in the connected state, relative movement of the holder Wc5 and the rod Wc6 is restricted, and thus the holder Wc5 and the rod Wc6 integrally move in the Dc1 direction and the Dc2 direction. That is, in the case where the connecting portion Wc is in the connected state, the driving force from the drive source M is transmitted to the wire body Wb through the connecting portion Wc.

In the connected state of FIG. 25A, the protrusion portion c53 of the holder Wc5 is configured to fit in the recess portions c63 of the rod Wc6 in a state in which the backlash is reduced. In the present embodiment, the protrusion portion c53 is provided on the arm portion c522 of the torsion spring c52 having elasticity, and the torsion spring c52 is slightly elastically deformed in the connected state such that the arm portion c522 is lifted up in the Df direction (upward in the drawing). Therefore, the protrusion portion c53 is pressed against the protrusions p5 and p6 on the two sides thereof by the elastic force of the torsion spring c52. That is, the connecting portion Wc according to the present embodiment connects the holder Wc5 serving as a first member and the rod Wc6 serving as a second member by a mechanism (snap fit mechanism) including a deforming element formed from an elastic member.

As described above, the responsiveness of the bending portion 12 of the catheter 11 to the driving of the drive source M can be improved by the configuration of engaging the holder Wc5 and the rod Wc6 with each other in the state in which the backlash is reduced.

### (When Overload Is Generated in Pressing Direction)

Next, a case where an excessive force in a direction (Dc1 direction) to press the wire body Wb acts on the connecting portion Wc will be described. As illustrated in FIG. 25B, when a driving force in a direction (Dc1 direction) to press the wire body Wb is transmitted from the drive source M to the holder Wc5, a compressive force Fc acts between the holder Wc5 and the rod Wc6.

In the case where this compressive force Fc does not exceed a preset threshold value (second threshold value), the compressive force Fc is received by the rod Wc6 via the contact portion between the protrusion portion c53 of the holder Wc5 and the protrusion p6 of the rod Wc6. That is, the connecting portion Wc of the present embodiment is configured to maintain the state in which the protrusion portion c53 is fitted in the recess portion c63 and transmit the driving force from the drive source M to the wire body Wb in the case where the compressive force Fc equal to or smaller than the second threshold value is applied.

However, in the case where a large compressive force Fc exceeding the preset threshold value (second threshold value) is applied, the torsion spring c52 elastically deforms such that the protrusion portion c53 climbs up the protrusion p6 of the rod Wc6. As a result of this, the holder Wc5 and the rod Wc6 relatively move while the protrusion portion c53 climbs over the protrusion p6, and the protrusion portion c53 is disengaged from the recess portions c63. In the state (disconnected state) in which the protrusion portion c53 is disengaged from the recess portion c63, the rod Wc6 does not move in the Dc1 direction even when the holder Wc5 moves in the Dc1 direction. That is, the connecting portion Wc of the present embodiment is configured such that in the case where the compressive force Fc exceeding the second threshold value is applied, the protrusion portion c53 is disengaged from the recess portion c63 and the transmission of the driving force from the drive source M to the wire body Wb is cut off (FIG. 25B).

As a result of the connecting portion Wc switching from the connected state to the disconnected state at the time of overload, a possibility that the bending portion 12 of the catheter 11 is bent by an excessively strong force due to an operation abnormality of the drive source M or a member such as the coupling portion 21c when the wire body Wb receives an external force can be reduced.

To be noted, as illustrated in FIG. 25A, a clearance Ld allowing the relative movement of the holder Wc5 and the rod Wc6 is secured between the distal end position of the rod Wc6 in the Dc2 direction in the connected state and a wall surface c518 of a bottom portion of the space c51 s of the holder Wc5. The length in the Dc direction of the clearance Ld is set on the basis of a position X0 of the protrusion portion c53 in the connected state such that the wall surface c518 does not come into contact with the rod Wc6 even when the holder Wc5 has further moved in the Dc1 direction by a predetermined distance after the protrusion portion c53 is completely disengaged from the recess portion c63. As a result of securing such clearance Ld, a situation in which the rod Wc6 is pressed by the wall surface c518 of the holder Wc5 and the driving force in the Dc1 direction is transmitted even though the protrusion portion c53 has been disengaged from the recess portion c63 can be prevented.

### (When Overload Is Generated in Pulling Direction)

Next, a case where an excessive force in a direction (Dc2 direction) to pull the wire body Wb acts on the connecting portion Wc will be described. As illustrated in FIG. 25C, when a driving force in a direction (Dc2 direction) to pull the wire body Wb is transmitted from the drive source M to the holder Wc5, a tensile force Ft acts between the holder Wc5 and the rod Wc6.

In the case where this tensile force Ft does not exceed a preset threshold value (first threshold value), the tensile force Ft is received by the rod Wc6 via the contact portion between the protrusion portion c53 of the holder Wc5 and the protrusion p5 of the rod Wc6. That is, the connecting portion Wc of the present embodiment is configured to maintain the state in which the protrusion portion c53 is fitted in the recess portion c63 and transmit the driving force from the drive source M to the wire body Wb in the case where the tensile force Ft equal to or smaller than the first threshold value is applied.

However, in the case where a large tensile force Ft exceeding the preset threshold value (first threshold value) is applied, the torsion spring c52 elastically deforms such that the protrusion portion c53 climbs up the protrusion p5 of the rod Wc6. As a result of this, the holder Wc5 and the rod Wc6 relatively move while the protrusion portion c53 climbs over the protrusion p5, and the protrusion portion c53 is disengaged from the recess portions c63. In the state (disconnected state) in which the protrusion portion c53 is disengaged from the recess portion c63, the rod Wc6 does not move in the Dc2 direction even when the holder Wc5 moves in the Dc2 direction. That is, the connecting portion Wc of the present embodiment is configured such that in the case where the tensile force Ft exceeding the second threshold value is applied, the protrusion portion c53 is disengaged from the recess portion c63 and the transmission of the driving force from the drive source M to the wire body Wb is cut off (FIG. 25C).

As a result of the connecting portion Wc switching from the connected state to the disconnected state at the time of overload, a possibility that the bending portion 12 of the catheter 11 is bent by an excessively strong force due to an operation abnormality of the drive source M or a member such as the coupling portion 21c is damaged when the wire body Wb receives an external force can be reduced.

As described above, also according to the present embodiment, the connection between the drive source M and the wire body Wb can be cut off in response to both an overload in a direction to press the wire body Wb and an overload in a direction to pull the wire body Wb.

### [Fourth Embodiment]

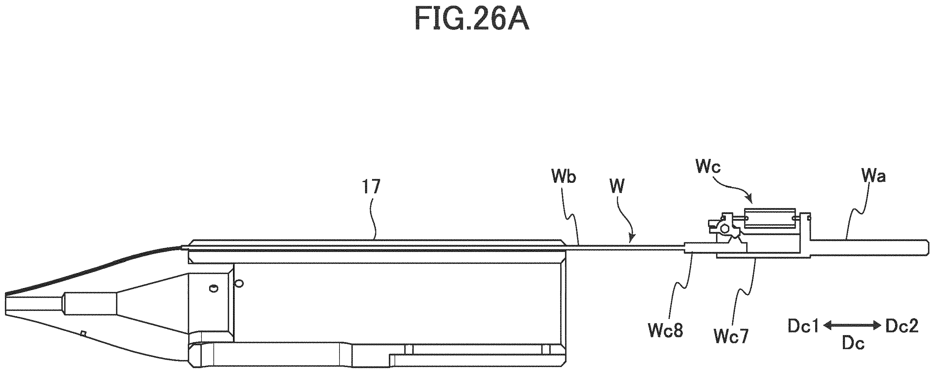

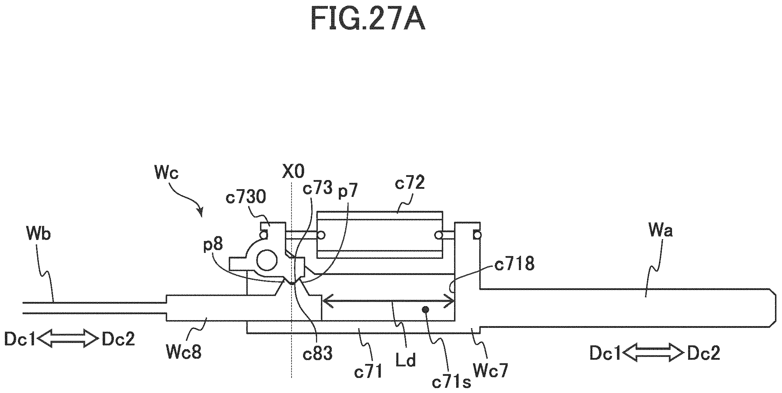

A medical apparatus according to a fourth embodiment will be described with reference to FIGS. 26A to 26C and 27A to 27C. In the present embodiment, the configuration of the connecting portion Wc interconnecting the drive source M and the wire body Wb is different from the first embodiment. In the description below, part different from the first embodiment will be mainly described assuming that elements denoted by the same reference signs as in the first embodiment have substantially the same configurations and effects as those described in the first embodiment.

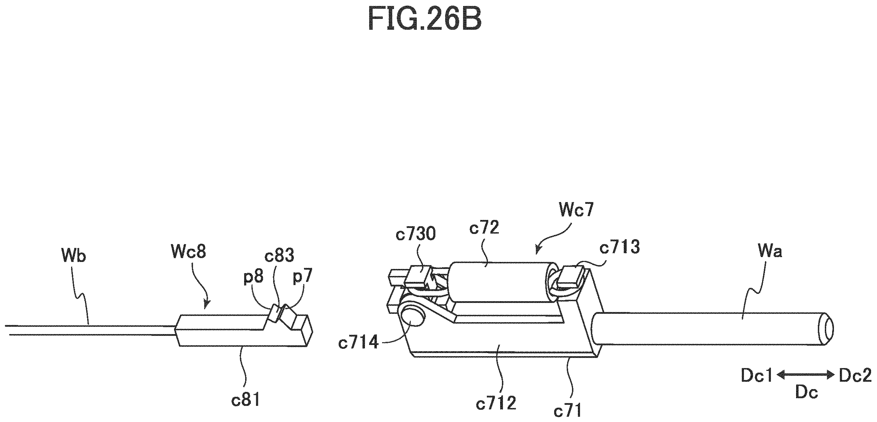

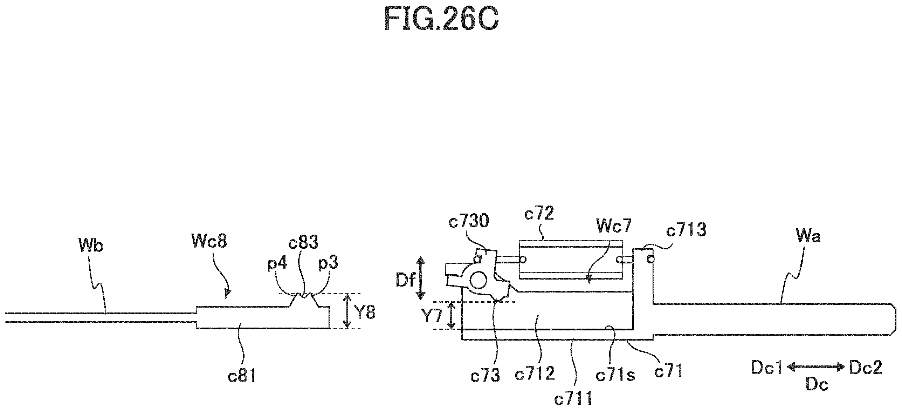

FIG. 26A is a schematic view of the driving wire W according to the present embodiment including the connecting portion Wc. FIG. 26B is a perspective view of a holder Wc7 and a rod Wc8 constituting the connecting portion Wc. FIG. 26C is a section view of the holder Wc7 and the rod Wc8 constituting the connecting portion Wc. FIG. 26A illustrates a state (connected state) in which the rod Wc8 is held by the holder Wc7, and FIGS. 26B and 26C illustrate a state (disconnected state) in which the rod Wc8 is not held by the holder Wc7.

In the description below, the connecting portion Wc provided on arbitrary one driving wire W of the first to ninth driving wires (W11 to W33) included in the catheter unit 100 of the present embodiment will be described. In the configuration example of the present embodiment, the connecting portion Wc having substantially the same configuration as that described below is provided on each of the ninth driving wires (W11 to W33).

As illustrated in FIG. 26A, the connecting portion Wc includes the holder Wc7 serving as a first member (engaging member, holding member) connected to the drive source M, and the rod Wc8 serving as a second member (engaged member, held member) connected to the wire body Wb of the driving wire W. In the present embodiment, at least part of the holder Wc7 is a member integrally formed with the held portion Wa of the driving wire W. Therefore, the holder Wc7 is connected to the drive source M via the held portion Wa and the coupling portion 21c (FIGS. 6A to 6C). To be noted, the holder Wc7 and the held portion Wa may be separate bodies. Meanwhile, the rod Wc8 is fixed to the near end of the wire body Wb by an arbitrary fixing method such as compression bonding (swaging) or gluing.

The holder Wc7 includes a body portion (base portion) c71 integrally formed with the held portion Wa, a torsion coil spring c72 supported by the body portion c31, and a rotary member c730 as illustrated in FIGS. 26B and 26C. The body portion c71 has a groove shape having a C-shaped cross-section (rectangular shape opening in one direction) surrounded by a bottom portion c711 and a side wall portion c712 as viewed in the Dc direction. The torsion coil spring c72 and the rotary member c730 are attached to an opening portion of the body portion c71 as viewed in the Df direction. Therefore, a space c71s for receiving the rod Wc8 as will be described below is defined between the bottom portion c711 and the side wall portion c712 of the body portion c71 and the torsion coil spring c72 and the rotary member c730. The body portion c71 is aligned with the held portion Wa in the Dc direction, and the held portion Wa extends in the Dc2 direction from an end portion of the body portion c71 in the Dc2 direction.

The torsion coil spring c72 is disposed such that the Dc direction is the axial direction (extending direction) thereof, an end portion thereof in the Dc2 direction is supported by a support portion c713 of the body portion c71, and an end portion thereof in the Dc1 direction is attached to the rotary member c730. The rotary member c730 is capable of rotating about a support shaft c714 provided on the body portion c71. The rotary member c730 is positioned by being urged in the clockwise direction in the drawing by the elastic force of the torsion coil spring c72 being latched by a latch portion c715 provided on the body portion c71.

In addition, the holder Wc7 includes a protrusion portion c73 formed on the rotary member c730. The protrusion portion c73 protrudes toward the bottom portion c711 side of the body portion c71 more than the support shaft c714 in a state in which the rotary member c730 is latched by the latch portion c715. That is, the protrusion portion c73 is an example of a protrusion portion protruding in a direction intersecting with the extending direction (Dc direction) of the wire body Wb in the connecting portion Wc.

In the case where the protrusion portion c73 of the rotary member c730 is pressed by the rod Wc8 and the rotary member c730 is rotated in the counterclockwise direction such that the protrusion portion c73 retracts upward in the drawing, the torsion coil spring c72 deforms in accordance with the rotation of the rotary member c730. That is, the torsion coil spring c72 functions as a deforming element capable of deforming to allow the protrusion portion c73 to be disengaged from a recess portion c83 that will be described later.

The protrusion portion c73 is disposed at a position corresponding to the recess portion c83 on the rod Wc8 as viewed in the Dc direction. In the present embodiment, the protrusion portion c73 is provided at one position in correspondence with the recess portion c83 provided at one position. To be noted, it suffices as long as the protrusion portion c73 is provided at a position engageable with the recess portion c83.

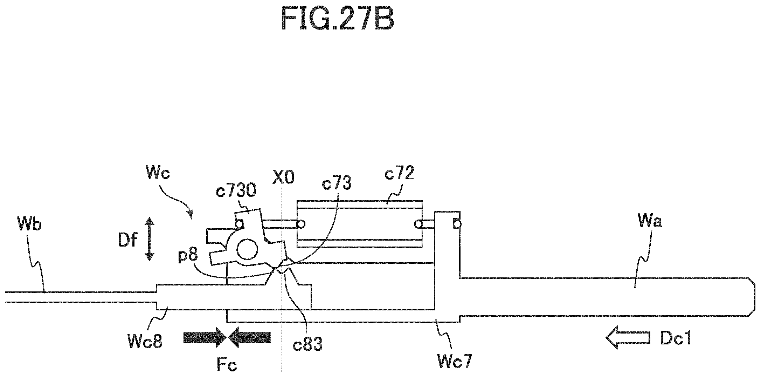

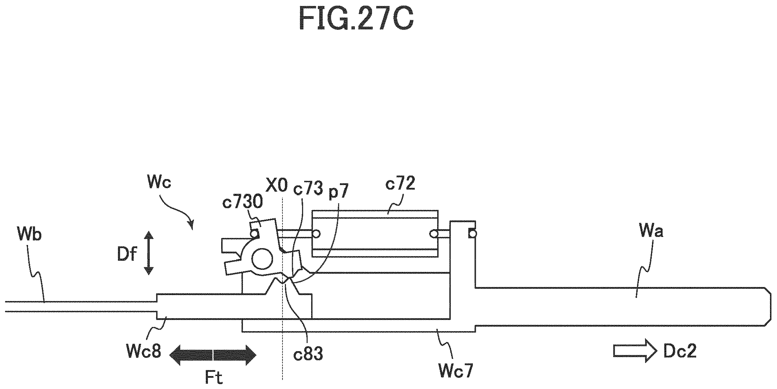

As illustrated in FIGS. 27B and 27C, the rod Wc8 includes a base portion c81 having an approximate quadrangular prismatic shape extending in the Dc2 direction from the near end of the wire body Wb, and a recess portion c83 provided on a side surface (surface facing the rotary member c730) of the base portion c81 in the Df direction. The recess portion c83 is formed between protrusions p7 and p8 protruding in the Df direction from the base portion c81. The protrusions p7 and p8 are arranged in the Dc direction.

The rod Wc8 is inserted in the Dc2 direction toward the space c71s between the body portion c71 of the holder Wc7 and the rotary member c730 and the torsion coil spring c72. Then, the rod Wc8 is attached to the holder Wc7 by being pushed in until the protrusion portion c73 of the holder Wc7 fits in the recess portion c83 of the rod Wc8. In the present embodiment, part of the rod Wc8 on the Dc2 direction side is an insertion portion to be inserted into the space c71s in the holder Wc7.

Next, the behavior of the connecting portion Wc in the connected state and at the time of an overload will be described with reference to FIGS. 27A to 27C. FIG. 27A is a schematic diagram illustrating the cross-section of the connecting portion Wc in the connected state. FIG. 27C is a schematic diagram illustrating the state of the connecting portion Wc in the case where an overload is applied in a direction (Dc1 direction) to press the wire body Wb. FIG. 27C is a schematic diagram illustrating the state of the connecting portion Wc in the case where an overload is applied in a direction (Dc2 direction) to pull the wire body Wb.

As illustrated in FIG. 27A, in the connected state of the connecting portion Wc, the protrusion portion c73 of the holder Wc7 is fitted in the recess portion c83 of the rod Wc8. Specifically, the protrusion portion c73 is held between the protrusions p7 and p8 in the Dc direction. Here, as illustrated in FIG. 26C, a distance in the Df direction from the bottom surface of the base portion c81 to the apices of the protrusions p7 and p8 in a state in which the rod Wc8 is not attached to the holder Wc7 is denoted by Y8. This distance Y8 is larger than a distance Y7 in the Df direction from the protrusion portion c73 of the holder Wc7 to the bottom portion c711 of the body portion c71. Therefore, in the connected state illustrated in FIG. 27A, the protrusion portion c73 of the holder Wc7 and part of the rod Wc8 (protrusions p7 and p8) are in such a positional relationship as to interfere with each other in the Dc direction. In other words, at least part of a protrusion portion provided on one of the first member and the second member overlaps a recess portion provided on the other of the first member and the second member as viewed in the extending direction (Dc direction) of the wire body in the connected state.

According to such a configuration, in the case where the connecting portion Wc is in the connected state, relative movement of the holder Wc7 and the rod Wc8 is restricted, and thus the holder Wc7 and the rod Wc8 integrally move in the Dc1 direction and the Dc2 direction. That is, in the case where the connecting portion Wc is in the connected state, the driving force from the drive source M is transmitted to the wire body Wb through the connecting portion Wc.

In the connected state of FIG. 27A, the protrusion portion c73 of the holder Wc7 is configured to fit in the recess portion c83 of the rod Wc8 in a state in which the backlash is reduced. In the present embodiment, the protrusion portion c73 is connected to the torsion coil spring c72 having elasticity, and the torsion coil spring c72 is slightly elastically deformed in the connected state such that the protrusion portion c73 is lifted up in the Df direction (upward in the drawing). Therefore, the protrusion portion c73 is pressed against the protrusions p7 and p8 on the two sides thereof by the elastic force of the torsion coil spring c72. That is, the connecting portion Wc according to the present embodiment connects the holder Wc7 serving as a first member and the rod Wc8 serving as a second member by a mechanism (snap fit mechanism) including a deforming element formed from an elastic member.

As described above, the responsiveness of the bending portion 12 of the catheter 11 to the driving of the drive source M can be improved by the configuration of engaging the holder Wc7 and the rod Wc8 with each other in the state in which the backlash is reduced.

### (When Overload Is Generated in Pressing Direction)

Next, a case where an excessive force in a direction (Dc1 direction) to press the wire body Wb acts on the connecting portion Wc will be described. As illustrated in FIG. 27B, when a driving force in a direction (Dc1 direction) to press the wire body Wb is transmitted from the drive source M to the holder Wc7, a compressive force Fc acts between the holder Wc7 and the rod Wc8.

In the case where this compressive force Fc does not exceed a preset threshold value (second threshold value), the compressive force Fc is received by the rod Wc8 via the contact portion between the protrusion portion c73 of the holder Wc7 and the protrusion p8 of the rod Wc8. That is, the connecting portion Wc of the present embodiment is configured to maintain the state in which the protrusion portion c73 is fitted in the recess portion c83 and transmit the driving force from the drive source M to the wire body Wb in the case where the compressive force Fc equal to or smaller than the second threshold value is applied.

However, in the case where a large compressive force Fc exceeding the preset threshold value (second threshold value) is applied, the rotary member c730 rotates such that the protrusion portion c73 climbs up the protrusion p8 of the rod Wc8, and thus the torsion coil spring c72 elastically deforms. As a result of this, the holder Wc7 and the rod Wc8 relatively move while the protrusion portion c73 climbs over the protrusion p8, and the protrusion portion c73 is disengaged from the recess portions c83. In the state (disconnected state) in which the protrusion portion c73 is disengaged from the recess portion c83, the rod Wc8 does not move in the Dc1 direction even when the holder Wc7 moves in the Dc1 direction. That is, the connecting portion Wc of the present embodiment is configured such that in the case where the compressive force Fc exceeding the second threshold value is applied, the protrusion portion c73 is disengaged from the recess portion c83 and the transmission of the driving force from the drive source M to the wire body Wb is cut off (FIG. 27B).

As a result of the connecting portion Wc switching from the connected state to the disconnected state at the time of overload, a possibility that the bending portion 12 of the catheter 11 is bent by an excessively strong force due to an operation abnormality of the drive source M or a member such as the coupling portion 21c is damaged when the wire body Wb receives an external force can be reduced.

To be noted, as illustrated in FIG. 27A, a clearance Ld allowing the relative movement of the holder Wc7 and the rod Wc8 is secured between the distal end position of the rod Wc8 in the Dc2 direction in the connected state and the wall surface c718 positioned at the bottom portion of the space c71s of the holder Wc7. The length in the Dc direction of the clearance Ld is set on the basis of a position X0 of the protrusion portion c73 in the connected state such that the wall surface c718 does not come into contact with the rod Wc8 even when the holder Wc7 has further moved in the Dc1 direction by a predetermined distance after the protrusion portion c73 is completely disengaged from the recess portion c83. As a result of securing such clearance Ld, a situation in which the rod Wc8 is pressed by the wall surface c718 of the holder Wc7 and the driving force in the Dc1 direction is transmitted even though the protrusion portion c73 has been disengaged from the recess portion c83 can be prevented.

### (When Overload Is Generated in Pulling Direction)

Next, a case where an excessive force in a direction (Dc2 direction) to pull the wire body Wb acts on the connecting portion Wc will be described. As illustrated in FIG. 27C, when a driving force in a direction (Dc2 direction) to pull the wire body Wb is transmitted from the drive source M to the holder Wc7, a tensile force Ft acts between the holder Wc7 and the rod Wc8.

In the case where this tensile force Ft does not exceed a preset threshold value (first threshold value), the tensile force Ft is received by the rod Wc8 via the contact portion between the protrusion portion c73 of the holder Wc7 and the protrusion p7 of the rod Wc8. That is, the connecting portion Wc of the present embodiment is configured to maintain the state in which the protrusion portion c73 is fitted in the recess portion c83 and transmit the driving force from the drive source M to the wire body Wb in the case where the tensile force Ft equal to or smaller than the first threshold value is applied.

However, in the case where a large tensile force Ft exceeding the preset threshold value (first threshold value) is applied, the rotary member c730 rotates such that the protrusion portion c73 climbs up the protrusion p7 of the rod Wc8, and thus the torsion coil spring c72 elastically deforms. As a result of this, the holder Wc7 and the rod Wc8 relatively move while the protrusion portion c73 climbs over the protrusion p7, and the protrusion portion c73 is disengaged from the recess portions c83. In the state (disconnected state) in which the protrusion portion c73 is disengaged from the recess portion c83, the rod Wc8 does not move in the Dc2 direction even when the holder Wc7 moves in the Dc2 direction. That is, the connecting portion Wc of the present embodiment is configured such that in the case where the tensile force Ft exceeding the second threshold value is applied, the protrusion portion c73 is disengaged from the recess portion c83 and the transmission of the driving force from the drive source M to the wire body Wb is cut off (FIG. 27C).

As a result of the connecting portion Wc switching from the connected state to the disconnected state at the time of overload, a possibility that the bending portion 12 of the catheter 11 is bent by an excessively strong force due to an operation abnormality of the drive source M or a member such as the coupling portion 21c is damaged when the wire body Wb receives an external force can be reduced.

As described above, also according to the present embodiment, the connection between the drive source M and the wire body Wb can be cut off in response to both an overload in a direction to press the wire body Wb and an overload in a direction to pull the wire body Wb.

### (Other Embodiments)

In each embodiment described above, description has been given assuming that each element of the connecting portion Wc is disposed in the catheter unit 100 that is a replaceable unit, but the connecting portion Wc may be disposed in the base unit 200. For example, a configuration in which the tractor support shaft 21cs (FIG. 6A) provided at the coupling portion 21c is divided into the two members of a holder and a rod described in each embodiment can be employed.

In addition, in each embodiment described above, description has been given assuming that regarding the threshold value of the load by which the connection of the connecting portion Wc is cut off, the first threshold value in the case where a tensile force acts between the first member and the second member and the second threshold value in the case where a compressive force acts between the first member and the second member are equal. However, the first threshold value and the second threshold value may be set to different values. For example, the first threshold value and the second threshold value are set in accordance with the range of an expected load in the case where, for example, the load expected in a normal use condition varies depending on the direction in which the wire body Wb is driven.

In addition, in each embodiment described above, the values of the first threshold value and the second threshold value in the connecting portion are the same for each pair of the plurality of drive sources (M11 to M33) and the plurality of wire bodies (Wb11 to Wb33). However, the first threshold value and the second threshold value in the connecting portion may be set to different values for each pair of the drive source and the wire body. For example, in the case where the expected load differs between a wire body connected to a guide ring that is more on the distal end side and a wire body connected to a guide ring that is more on the proximal end side in the bending portion 12 of the catheter 11, the first threshold value and the second threshold value are set in accordance with the range of the expected load.

In addition, in each embodiment described above, the drive source and the wire body are connected via a connecting portion for each pair of the plurality of drive sources (M11 to M33) and the plurality of wire bodies (Wb11 to Wb33). However, a configuration in which the connecting portion is provided in only some of the pairs of the drive sources and the wire bodies may be employed.

In addition, in each embodiment described above, description has been given by taking the bendable catheter 11 as an example of a target obj ect to be operated. However, this is not limiting, and examples of the target object to be operated may include articulated robots. Examples of the articulated robots include medical robot arms including surgical tools (such as forceps and scalpels) at a tip end thereof. When bending ajoint of this robot by using a wire and the like, the breakaway mechanism described in each embodiment described above may be applied.

### INDUSTRIAL APPLICABILITY

The present invention is not limited to the embodiments described above, and can be modified and deformed in various manners without departing from the concept and scope of the present invention. Therefore, to publicize the scope of the present invention, the following claims are attached.

### REFERENCE SIGNS LIST

12: bending portion
M: drive source
Wb: linear body (wire body)
Wc: connecting portion
Wc1, Wc3, Wc5, Wc7: first member
Wc2, Wc4, Wc6, Wc8: second member
c13, c33, c53, c73: protrusion portion
c23, c43, c63, c83: recess portion
c18: first protrusion portion
c19: second protrusion portion
c28: third protrusion portion
c29: fourth protrusion portion

## Claims

1. A medical apparatus comprising:
a drive source;
a bending portion capable of bending;
a linear body configured to bend the bending portion by a driving force of the drive source; and
a connecting portion which includes a first member connected to the drive source, a second member connected to the linear body, a protrusion portion provided in one of the first member and the second member and protruding in a direction intersecting with an extending direction of the linear body, and a recess portion provided in another of the first member and the second member, and in which the first member and the second member are connected in a state in which the protrusion portion is fitted in the recess portion,
wherein the connecting portion is configured such that
in a case where a tensile force equal to or smaller than a first threshold value or a compressive force equal to or smaller than a second threshold value acts between the first member and the second member, the state in which the protrusion portion is fitted in the recess portion is maintained to transmit the driving force from the drive source to the linear body, and
in a case where a tensile force exceeding the first threshold value or a compressive force exceeding the second threshold value acts between the first member and the second member, the protrusion portion is disengaged from the recess portion to cut off transmission of the driving force from the drive source to the linear body.

2. The medical apparatus according to claim 1,
wherein the connecting portion includes an elastic element capable of elastic deformation, and is configured such that in the case where the tensile force exceeding the first threshold value or the compressive force exceeding the second threshold value acts between the first member and the second member, the protrusion portion is allowed to be disengaged from the recess portion.

3. The medical apparatus according to claim 1,
wherein the connecting portion is configured such that in the case where the tensile force exceeding the first threshold value or the compressive force exceeding the second threshold value acts between the first member and the second member, the protrusion portion is allowed to be disengaged from the recess portion by plastic deformation of the protrusion portion or the recess portion.

4. The medical apparatus according to any one of claims 1 to 3,
wherein a space into which the second member is insertable from one side in the extending direction of the linear body is provided in the first member, and
wherein the protrusion portion is fitted in the recess portion in a state in which the second member is inserted in the space of the first member.

5. The medical apparatus according to claim 4,
wherein the first member includes a tubular portion extending in the extending direction of the linear body and defining the space,
wherein the second member includes an insertion portion to be inserted into the space in the tubular portion,
wherein the protrusion portion is provided on one of an inner surface of the tubular portion and an outer surface of the insertion portion, and
wherein the recess portion is provided on another of the inner surface of the tubular portion and the outer surface of the insertion portion.

6. The medical apparatus according to claim 4,
wherein the first member includes a body portion having a groove shape extending in the extending direction of the linear body and opening in a direction intersecting with the extending direction, and a plate spring disposed to cover the groove shape, and the space is defined between the body portion and the plate spring,
wherein the second member includes an insertion portion to be inserted into the space,
wherein the protrusion portion is provided on one of a surface of the plate spring facing the space and a surface of the insertion portion facing the plate spring, and
wherein the recess portion is provided on another of the surface of the plate spring facing the space and the surface of the insertion portion facing the plate spring.

7. The medical apparatus according to claim 4,
wherein the first member includes a body portion having a groove shape extending in the extending direction of the linear body and opening in a direction intersecting with the extending direction, and a torsion spring disposed at an opening portion of the groove shape, and the space is defined between the body portion and the torsion spring,
wherein the second member includes an insertion portion to be inserted into the space,
wherein the protrusion portion is provided on one of a surface of the torsion spring facing the space and a surface of the insertion portion facing the torsion spring, and
wherein the recess portion is provided on another of the surface of the torsion spring facing the space and the surface of the insertion portion facing the torsion spring.

8. The medical apparatus according to claim 4,
wherein the first member includes a body portion having a groove shape extending in the extending direction of the linear body and opening in a direction intersecting with the extending direction, a rotary member that is rotatable and disposed at an opening portion of the groove shape, and a torsion coil spring configured to urge the rotary member, and the space is defined between the body portion and the rotary member,
wherein the second member includes an insertion portion to be inserted into the space,
wherein the protrusion portion is provided on one of a surface of the rotary member facing the space and a surface of the insertion portion facing the rotary member, and
wherein the recess portion is provided on another of the surface of the rotary member facing the space and the surface of the insertion portion facing the rotary member.

9. The medical apparatus according to any one of claims 1 to 8,
wherein in the state in which the protrusion portion is fitted in the recess portion, no clearance in the extending direction of the linear body is provided between the protrusion portion and the recess portion, and relative movement of the first member and the second member is restricted.

10. A medical apparatus comprising:
a drive source;
a bending portion capable of bending;
a linear body configured to bend the bending portion by a driving force of the drive source; and
a connecting portion which includes a first member connected to the drive source, a second member connected to the linear body, a first protrusion portion and a second protrusion portion provided in the first member and protruding in a direction intersecting with an extending direction of the linear body, and a third protrusion portion and a fourth protrusion portion provided in the second member, and in which the first member and the second member are connected in a state in which the first protrusion portion is engaged with the third protrusion portion and the second protrusion portion is engaged with the fourth protrusion portion,
wherein the connecting portion is configured such that
in a case where a tensile force equal to or smaller than a first threshold value acts between the first member and the second member, the state in which the first protrusion portion is engaged with the third protrusion portion is maintained to transmit the driving force from the drive source to the linear body,
in a case where a tensile force exceeding the first threshold value acts between the first member and the second member, the first protrusion portion is disengaged from the third protrusion portion to cut off transmission of the driving force from the drive source to the linear body,
in a case where a compressive force equal to or smaller than a second threshold value acts between the first member and the second member, the state in which the second protrusion portion is engaged with the fourth protrusion portion is maintained to transmit the driving force from the drive source to the linear body, and
in a case where a compressive force exceeding the second threshold value acts between the first member and the second member, the second protrusion portion is disengaged from the fourth protrusion portion to cut off transmission of the driving force from the drive source to the linear body.

11. The medical apparatus according to claim 10,
wherein the connecting portion includes an elastic element capable of elastic deformation, and is configured such that:
in the case where the tensile force exceeding the first threshold value acts between the first member and the second member, the first protrusion portion is allowed to be disengaged from the third protrusion portion by the elastic deformation of the elastic element; and
in the case where the compressive force exceeding the second threshold value acts between the first member and the second member, the second protrusion portion is allowed to be disengaged from the fourth protrusion portion by the elastic deformation of the elastic element.

12. The medical apparatus according to claim 10,
wherein the connecting portion is configured such that:
in the case where the tensile force exceeding the first threshold value acts between the first member and the second member, the first protrusion portion is allowed to be disengaged from the third protrusion portion by plastic deformation of the first protrusion portion or the third protrusion portion; and
in the case where the compressive force exceeding the second threshold value acts between the first member and the second member, the second protrusion portion is allowed to be disengaged from the fourth protrusion portion by plastic deformation of the second protrusion portion or the fourth protrusion portion.

13. The medical apparatus according to any one of claims 10 to 12,
wherein a space into which the second member is insertable from one side in the extending direction of the linear body is provided in the first member, and
wherein, in a state in which the second member is inserted in the space of the first member, the first protrusion portion is engaged with the third protrusion portion and the second protrusion portion is engaged with the fourth protrusion portion.

14. The medical apparatus according to claim 13,
wherein the first member includes a tubular portion extending in the extending direction of the linear body and defining the space,
wherein the second member includes an insertion portion to be inserted into the space in the tubular portion,
wherein the first protrusion portion and the second protrusion portion are provided on an inner surface of the tubular portion, and
wherein the third protrusion portion and the fourth protrusion portion are provided on an outer surface of the insertion portion.

15. The medical apparatus according to claim 13,
wherein the first member includes a body portion having a groove shape extending in the extending direction of the linear body and opening in a direction intersecting with the extending direction, and a plate spring disposed to cover the groove shape, and the space is defined between the body portion and the plate spring,
wherein the second member includes an insertion portion to be inserted into the space,
wherein the first protrusion portion and the second protrusion portion are provided on a surface of the plate spring facing the space, and
wherein the third protrusion portion and the fourth protrusion portion are provided on a surface of the insertion portion facing the plate spring.

16. The medical apparatus according to claim 13,
wherein the first member includes a body portion having a groove shape extending in the extending direction of the linear body and opening in a direction intersecting with the extending direction, and a torsion spring disposed at an opening portion of the groove shape, and the space is defined between the body portion and the torsion spring,
wherein the second member includes an insertion portion to be inserted into the space,
wherein the first protrusion portion and the second protrusion portion are provided on a surface of the torsion spring facing the space, and
wherein the third protrusion portion and the fourth protrusion portion are provided on a surface of the insertion portion facing the torsion spring.

17. The medical apparatus according to claim 13,
wherein the first member includes a body portion having a groove shape extending in the extending direction of the linear body and opening in a direction intersecting with the extending direction, a rotary member that is rotatable and disposed at an opening portion of the groove shape, and a torsion coil spring configured to urge the rotary member, and the space is defined between the body portion and the rotary member,
wherein the second member includes an insertion portion to be inserted into the space,
wherein the first protrusion portion and the second protrusion portion are provided on a surface of the rotary member facing the space, and
wherein the third protrusion portion and the fourth protrusion portion are provided on a surface of the insertion portion facing the rotary member.

18. The medical apparatus according to any one of claims 10 to 17,
wherein in the state in which the first protrusion portion is engaged with the third protrusion portion and the second protrusion portion is engaged with the fourth protrusion portion, no clearance in the extending direction of the linear body is provided between the first protrusion portion and the third protrusion portion and between the second protrusion portion and the fourth protrusion portion, and relative movement of the first member and the second member is restricted.

19. The medical apparatus according to any one of claims 1 to 18, further comprising:
a plurality of drive sources including the drive source;
a plurality of linear bodies including the linear body and configured to bend the bending portion by being driven respectively by the plurality of drive sources; and
a plurality of connecting portions including the connecting portion and provided in correspondence with respective pairs of a drive source and a linear body each constituted by one drive source among the plurality of drive sources and one linear body among the plurality of linear bodies driven by that drive source,
wherein the plurality of connecting portions each have a same configuration as the connecting portion.
